# EUROPEAN PATENT SPECIFICATION

(11) **EP 3 778 612 B1**
(45) Date of publication and mention of the grant of the patent: **12.02.2025**
(21) Application number: 19777124.9
(22) Date of filing: 25.03.2019
(51) Int. Cl.: C07F 9/38, A61K 31/662, A61P 35/00, C07F 9/6509, C07F 9/655, A61P 35/02, C07F 9/58, A61P 35/04, C07F 9/6533, A61K 45/06

(54) **PHOSPHATE DERIVATIVES AND USE THEREOF**
PHOSPHATDERIVATE UND VERWENDUNG DAVON
DÉRIVÉS DE PHOSPHATE ET UTILISATION ASSOCIÉE

(30) Priority: 30.03.2018 CN 201810275072
(43) Date of publication of application: 17.02.2021
(73) Proprietor: Shanghai Shengyue Pharmaceutical Technology Co., Ltd., Shanghai 201800 (CN)
(72) Inventor: Zhu, Qing, Gongan County Hubei 434311 (CN)
(74) Representative: Sun, Yiming
(86) International application number: PCT/CN2019/079492
(87) International publication number: WO 2019/184866

(56) References cited:
- WO-A2-2006/055525
- CN-A- 102 093 416
- CN-A- 104 755 425
- US-A- 2 917 528
- US-A- 2 964 549
- HOLMES C P ET AL: "Discovery and structure-activity relationships of novel sulfonamides as potent PTP1B inhibitors", BIOORGANIC & MEDICINAL CHEMISTRY LETTERS, ELSEVIER, AMSTERDAM NL, vol. 15, no. 19, 1 October 2005 (2005-10-01), pages 4336 - 4341, XP027801445, ISSN: 0960-894X, [retrieved on 20051001]
- RAWLS KATHERINE A ET AL: "Fragment-based discovery of selective inhibitors of theMycobacterium tuberculosisprotein tyrosine phosphatase PtpA", BIOORGANIC & MEDICINAL CHEMISTRY LETTERS, vol. 19, no. 24, 2007, pages 6851 - 6854, XP029120865, ISSN: 0960-894X, DOI: 10.1016/J.BMCL.2009.10.090
- ELDO J ET AL: "Design, synthesis, and bioactivity of novel inhibitors of E. coli aspartate transcarbamoylase", BIORGANIC & MEDICINAL CHEMISTRY LETTERS, ELSEVIER, AMSTERDAM , NL, vol. 17, no. 7, 1 April 2007 (2007-04-01), pages 2086 - 2090, XP026265858, ISSN: 0960-894X, [retrieved on 20070312], DOI: 10.1016/J.BMCL.2006.12.050

## Description

### FIELD OF THE INVENTION

The present invention relates to the technical field of medicines, and in particular to phosphate derivatives and uses thereof in inhibiting tumor proliferation and metastasis.

### BACKGROUND OF THE INVENTION

The micro-environment of solid tumor includes three characters, low extracellular pH, hypoxia, high absorptivity of glucose. These three are closely relevant, and mutually synergistic, to promote tumor occurrence, proliferation, invasion, and metastasis. Glycometabolism were processed via two pathways, mitochondria oxidative phosphorylation and glycolysis. The normal cell metabolite through aerobic circulation, mitochondria oxidative phosphorylation, while tumor cell via glycolysis (anaerobic and aerobic glycolysis). Malignant tumor takes in glycose extremely high, up to 12 times comparing to normal cell, and positron emission tomography PET scan utilized this principle, applicating 2-¹⁸F-FDG ([18-F]-fluorodeoxyglucose)) to image the tumor (Ann Surg 2005;241 (2):286-94; Schwartz DL, *et al*.,). The overquick growth of cancer cell caused the anaerobic condition, which chased the cancer cell to shut down the pathway of mitochondria oxidative phosphorylation, and open up the anaerobic glycolysis. The glycometabolism to the pyruvic acid was not able to proceed via TCA of mitochondria oxidative phosphorylation, instead of lactic dehydrogenase (LDH), and the lactic was produced and eliminated out of cell. The anaerobic environment automatically promoted the glycolysis, and the final production of glycolysis was lactate. This kind of abnormal glycolysis of cancel cell directly created the acidic tumor microenvironment. The acidic environment accelerated tumor proliferation dramatically, and promoted the invasion of cancel cell to normal cells, meanwhile increased cancel cell capability of adaptation and modulation to this wicked environment. On the other hand, the anaerobic acidic microenvironment was tightly but negatively associated to the drug resistance.

The ambitus microenvironment of malignant tumor had a pH (pHe) of 6.5-6.9, the core could reach 6.2, comparably, the normal cell pH 7.3-7.5. The saliva of normal person was of pH 6.5-7.4, but cancer patient lower to pH 4.5- 5.7.

The acidic microenvironment of malignant tumor was generated mainly by below aspects. Warburg effect caused the aggregation of lactic acid, the anoxia drove the carbonic anhydrase and proton transporter. Both aspects co-worked and promoted the extracellular pH value decreased. Tumor cell took up a large amount of glucose, highly-efficiently turned the glucose to big volume of lactic acid, which was transported to the extracellular microenvironment by mono carboxylic acid transporter MCT. The intracellular generated CO₂ was released to the extracellular via diffusion, due to the anoxia, hypoxia-inducible factor 1 (HIF-1) induced the cancer cell to highly express CA-IX, and promote CO₂ with H₂O to generate form carbonic acid. Therefore, the acidic microenvironment of malignant tumor was generated mainly by glycolysis and hypoxia (Progress in Modern Biomedicine 2014, Vol.14 NO.19, 3775-3777).

There are two types of lactic acid: L-lactic acid and D-lactic acid. The lactic acid produced in cancer cells is mostly D-lactic acid. Cancer is caused by high concentrations of D-lactic acid and low concentrations of methylglyoxal in cells. It is believed that under normal circumstances, intestinal mucosa has a barrier effect on D-lactic acid, and a large amount of D-lactic acid produced is excreted through feces, so there is no cancer in general. Only in the case of intestinal mucosa damage, intestinal surgery and enteritis, the permeability of intestinal mucosa will increase, and D-lactic acid will enter the blood. D-lactic acid entering the blood is the first step of cancer. Therefore, more than 90% of cancer patients previously suffered from long-term gastrointestinal discomfort, chronic constipation and chronic enteritis. D-lactic acid entering into the blood may not necessarily lead to cancer, because the cell membrane also has a barrier effect on D-lactic acid. If there is inflammation, abscesses, ulcers or surgery or trauma in the cell at this time, the barrier effect of the cell membrane will disappear, and D-lactic acid will enter the cell, which is the second step to get cancer. When a certain amount of D-lactic acid enters the cell, it activates insulin, which in turn activates glyoxalase, which converts methylglyoxal into D-lactic acid, and cancer forms. That's the cancer trilogy. This creates a vicious cycle, chemically called autocatalysis, which no one can control. Automatic catalysis has the property that as long as there is a supply of raw materials and glucose is continuously ingested, its reaction rate will get faster and faster and will never stop automatically. The result: an imbalance between high concentrations of D-lactic acid and low concentrations of methylglyoxal is the root cause of cancer.

Tumor cells themselves adapt to acidic microenvironment, mainly through autophagy, upregulation of the transport system and other mechanisms. Due to the continuous stimulation of various carcinogens and growth factors, the dynamics of hydrogen ions in tumor cells was seriously disturbed, and the dynamic pH balance of the cells was abnormal. In order to avoid the toxicity of acidic microenvironment, the tumor cells then discharged hydrogen ions outward, and finally produced extracellular acidic environment and intracellular alkaline environment. Tumor cells realize this mechanism by upregulation of hydrogen ion-related transporters in the plasma membrane, such as Na/H exchanger protein (NHE), Na/K ATPase, vacuolar H-ATpase (V -- ATPase), H/C1 transporter and monocarboxylate transporter (MCT), etc. There are other mechanisms for tumor cells to adapt to acidic microenvironment, such as upregulation of VEGF and other genes to promote tumor angiogenesis; release of cathepsin B and other proteolytic enzymes, resulting in extracellular matrix degradation, leading to immune dysfunction, avoidance of host immune response, and immunotherapy; it makes tumor cells insensitive to radiotherapy, makes tumor cells develop drug resistance, and escapes the damage of chemotherapy drugs.

Acidic microenvironment is a powerful weapon for tumor cells to achieve their invasions and metastases. Tumor cells themselves generate growth signals, induce apoptosis, unlimited proliferation, invasion, metastasis and immune escape, and generate new blood vessels, which can be realized by inducing acidic environment. The whole field of tumor research from pathogenesis to treatment is closely related to the acidic microenvironment of tumors.

Acidic tumor microenvironment affects the function of T cells. Hypoxia-induced acidic tumor microenvironment is a powerful obstacle to the function of T cells. Severe hypoxia and lactic acid strongly inhibit the activation, proliferation and cytotoxicity of T cells. When the extracellular pH was 6.7, il-2-induced T cell proliferation stopped, and the extracellular pH of hypoxic tumor cells could be as low as 5.8-6.5. At this time, lymphocytes were actually apoptotic, while tumor cells could tolerate and continue to grow. In vitro experiments, acidic pH reduced the secretion of IFN- and TNF- by T cells, suggesting that the acidic microenvironment could broadly block the production of pro-inflammatory cytokines. In addition, specific acid receptor families can convert changes in extracellular acidity into intracellular signals. Acidic microenvironment can promote the expression of G-protein, T-cell inhibitory receptor and T-cell death-related gene -8, which mediates the translation and expression of C-MYC in lymphocytes.

Acidic microenvironment affects tumor aggression: the acidic microenvironment outside tumor cells can stimulate cell proliferation, activate transcription factors, enhance the expression of target genes, and promote tumor formation. Tumor cells can use lactic acid to maintain their own acidic microenvironment for metastasis. Induced extracellular matrix remodeling increases tumor invadability and metastasis, delays cell metabolism of carcinogens, and inhibits cell repair of DNA damage caused by carcinogens.

The effects of acidic microenvironment on tumor cells themselves are: changing the biological activity of tumor cells; due to the vigorous growth of tumor cells, tumor tissues are prone to accumulate abnormal acid metabolites due to insufficient relative blood flow perfusion, or promote the formation of tumor neovascularization by up-regulating the expression of VEGF and other genes, which also provides a suitable environment for tumor metastasis. Lead to abnormal immune function, including other components of the immune system, such as DC, MDSC, or macrophages. Cells evade host immune response and immunotherapy; for example, lactic acidosis is a strong negative prognostic indicator of sepsis; it makes tumor cells insensitive to radiotherapy and makes tumor cells develop drug resistance to avoid damage caused by chemotherapy drugs.

The damage caused by acid microenvironment products of tumor to normal tissues exposed to cancer is mainly caused by necrosis and apoptosis of normal peripheral cells. Extracellular matrix degradation is caused by the release of cathepsin B and other proteolytic enzymes.

Many chemotherapeutic agents are hampered by pH gradients, interstitial high pressure and hypoxic pressure caused by differences in intracellular and intracellular pH. The weak acid environment (pH= 6-7) is the optimal environment for mAb. The extracellular acidic environment in solid tumor tissues may reduce the therapeutic effect of mAb because the microenvironment is too acidic, which leads to the degradation of mAb and ultimately reduces its activity.

Based on the important role of acidic microenvironment in tumor, the relationship between tumor microenvironment and anticancer has been studied extensively. Magnetic resonance spectrum analysis showed that oral administration of NaHCOS or intrasplenal injection of NaHCOS could selectively increase the extracellular pH of the tumor and reduce the formation of lymph node and liver metastasis. Proton pump inhibitors (PPI) are the first-line drugs for the treatment of peptic ulcer, mainly including pantoprazole, lansoprazole, omeprazole, etc. They inhibit the activity of H + /K + -ATPase to act as an acid inhibitor. In recent years, the antitumor effect of PPI has been gradually recognized and become a hot spot in antitumor therapy. In vitro and in vivo experiments showed that PPI sensitized tumor cell lines were more sensitive to cisplatin, 5-fluorouracil, and vincine, and omeprazole could induce cisplatin sensitivity to C30 in human solid tumors inoculated in nude mice. This mechanism of PPI may be mainly due to the drug inhibition of the V-ATPase activity, enhanced the pH value in the extracellular and lysosome organelles, increased the drug concentration in cells, and reversed the high expression of the multi drug resistance MDR genes, which are sensitive to tumor drugs. The ability of PPI to inhibit tumor cells was directly related to the acidity level of the medium. PPI can induce the selective cytotoxicity of B cell tumors by activating a large number of reactive oxygen species (ROS) and destroying the lysosomal membrane structure, thus causing apoptosis of caspase-dependent cells. PPI can also improve the sensitivity of chemotherapy drugs, inhibit the growth of human transplanted tumors in animal models, and PPI does not cause systemic whole body reactions. Down-regulation of NHE1 can also significantly inhibit the invasion and metastasis of glioma cells. All these suggest that targeted intervention of NHE1 expression can regulate the pH of tumor cells and change the biological activity of tumor cells, so as to interfere the growth and metastasis of tumors.

Tumor metastasis is the root cause of failure in clinical treatment of malignant tumors. More than 80% of cancer deaths result from metastases. The accumulation of lactic acid in the tumor not only reflects the malignancy of the tumor, but also is closely related to its distant metastasis. Tumor metastasis refers to the invasion of tumor cells from the primary site into lymphatic vessels, blood vessels, body cavity, migration to other places and continue to grow, the formation of the same type of tumor as the primary tumor, this process is called metastasis. Benign tumors do not metastasize, but only malignant tumors metastasize. The common ways of metastasis are as follows: lymphatic metastasis: malignant tumors in epithelial tissues metastasize through lymphatic channels; blood passage metastasis: all kinds of malignant tumors can occur, especially meat cancer, kidney cancer, liver cancer, thyroid follicular cancer and choriocarcinoma. Implantation metastases: carcinomas of the abdominal organs. Lactic acid enters endothelial cells through MCT1 and leads to degradation and phosphorylation of I κ B α , which in turn stimulates the autocrine nuclear transcription factor κ B/ interleukin-8 pathway leading to cell migration and vascular formation. It was pointed out in the study that transplanted tumor models in human colorectal cancer and breast cancer mice showed that lactic acid released by tumor cells could be transported through MCT4, further stimulating the angiogenesis and tumor growth of interleukin-8-dependent tumors. To achieve the metastasis of the tumor. Lactic acid interferes with the body's immune response to cancer cells and helps them spread. Research has shown that lactic acid is present and involved in every step of cancer development.

Sun Xueying, professor of Harbin Medical University, pointed out that immunotherapy represented by blocking monoclonal antibodies and antigen receptor T cell immunotherapy has made a major breakthrough and has been applied in the clinical treatment of a variety of malignant tumors. However, tumor-specific microenvironment affects the effectiveness of immunotherapy. To further understand the influence of tumor hypoxic and acidic microenvironment on immunotherapy, and to study the mechanism of cancer cells escaping immune surveillance and attack, will help to explore new ideas and methods, and improve the efficacy of tumor immunotherapy (World Chinese Journal of Digestion, July 28, 2017; 25 (21): 1934-1944).

Hu Xun, a professor at Zhejiang Medical University, proposed starving cancer cells to death in 2017. Tumors contain large amounts of lactic acid, which dissociates into lactate anions and hydrogen ions, which act as "helpers" to the cancer cell and allow it to "consume" how much food it consumes. If either of these factors is removed, the cancer cells will quickly die if glucose is starved or deficient. They found that by removing hydrogen ions from tumors with a base such as sodium bicarbonate (baking soda), the synergy between lactate and hydrogen ions was destroyed, quickly and effectively killing tumor cells that were starved or starved of glucose. "Conventional arterial cannulated chemoembolization (cTACE) cuts off the tumor's 'food channel' and then removes hydrogen ions from the tumor with sodium bicarbonate, which is equivalent to not only not feeding the tumor, but also sending it to the gym for rapid consumption and rapid 'starvation'." Happily, clinical studies showed that cTACE was effective in 18 of the 37 patients treated. TILA-TACE was used to treat 40 patients, 40 of whom were effective.

Robert Gillies of H. LEE MOFFITT Cancer Research Center in Florida conducted a large number of studies on the effects of baking soda, imidazole and its derivatives, Tris, lysine and other buffering agents with buffering capability on the acidic microenvironment of tumors, and drew a clear conclusion that the buffering solution could effectively inhibit the growth and metastasis of tumors in mice. Sodium bicarbonate has also been used in clinical trials at the University of Arizona.

Eldo et al., BIOORGANIC&MEDICINAL CHEMISTRY LETTERS, vol.17, no.7, 1 April 2007, 2086-2090 discloses a series of inhibitors of the aspartate transcarbamoylase, an enzyme involved in pyrimidine nucleotide biosynthesis which has been synthesized. These inhibitors are analogues of a highly potent inhibitor of this enzyme, N-phosphonacetyl-L-aspartate (PALA). Analogues have been synthesized with modifications at the α- and β-carboxylates as well as at the aspartate moiety. The ability of these compounds to inhibit the enzyme was evaluated. These studies, with functional group modified PALA derivatives, showed that amide groups can be a useful substitute of the carboxylate in order to reduce the charge on the molecule, and indicate that the relative position of the functional group in the β-position is more critical than the nature of the functional group. Some of the molecules synthesized here are potent inhibitors of the enzyme.

At present, a large number of studies have shown that increasing the pH value of tumor microenvironment can effectively inhibit tumor growth and metastasis. Therefore, the acidic microenvironment of solid tumors is a potential therapeutic target for cancer, especially metastatic tumors.

Therefore, the field of biomedical technology is in urgent need of modulators that can effectively adjust the acidic microenvironment of tumors, especially new small-molecule compounds with higher pH value.

### SUMMARY OF THE INVENTION

The technical problem to be solved for the invention is to provide a small molecule which can effectively increase the pH value of the tumor microenvironment. The present invention provides a novel type of phosphate derivatives that have a pH value over 8 with monodentate or multidentate base function groups, and it can modulate the acidity of the tumor microenvironment, effectively inhibit cancer proliferation and metastasis, to get better therapeutic effect on cancers in clinic.

In order to solve the above technical problem, the present invention is implemented which is defined in the appended claims.

In one aspect, the present invention provides a pharmaceutical composition, which includes a therapeutically effective amount of the compound shown in formula (II), or the tautomer, mesomer, racemate, enantiomer, and diastereoisomer thereof, or the mixture form thereof, or the pharmaceutically acceptable salt thereof, and further includes one or more pharmaceutically acceptable carriers, diluents, excipients.

The above acceptable carrier is nontoxic and can be used for auxiliary application without adverse effect on the therapeutic effect of the compound. Such carrier can be any commonly available solid excipient, liquid excipient, semi-solid excipient or gas excipient in aerosol composition for those skilled in the art. Solid drug excipients include starch, cellulose, talc, glucose, lactose, sucrose, gelatin, malt, rice, flour, chalk, silica gel, magnesium stearate, sodium stearate, glyceryl stearyl ester, sodium chloride, anhydrous skim milk, etc. Liquid and semi-solid excipients can be selected from glycerin, propylene glycol, water, ethanol and various oils, including the oil originated from petroleum, animal and plant or synthetic oil, such as peanut oil, soybean oil, mineral oil, sesame oil, etc. Preferable liquid carriers, especially those used for injectable solutions, include, water, saline, glucose aqueous solution and glycol. In addition, other adjuvants such as flavoring agent, sweetening agent, etc. can also be added in the composition.

The compound of the present invention can be administrated in the therapeutically effective dose, it can be administrated either orally or systemically (such as transcutaneously, nasal inhalation or suppository) or parenterally (such as intramuscularly, intravenously or subcutaneously). Oral administration is preferred, and it can be adjusted according to the severity of disease.

The actual application amount (i.e., active ingredient) for the compound of the invention depends on multiple factors, such as the severity of disease to be treated, the age and relative health level of the treated subject, the efficacy of the used compound, way and form of administration, and other factors.

Various dosage forms of the medicinal composition of the invention can be prepared in accordance with the conventional methods in the field of pharmacy. For example, the compound can be mixed with one or more carriers, and then it was prepared into the desired dosage form, such as tablets, pills, capsules, semi solid, powder, slow release formulation, solution, suspension, compounding solvent, aerosol, etc.

In another aspect, the present invention further provides the compound shown in formula (II), or the tautomer, mesomer, racemate, enantiomer, and diastereoisomer thereof, or a mixture form thereof, or the pharmaceutically acceptable salt thereof, or the above-mentioned pharmaceutical composition for use in preparing drugs for preventing and/or treating the diseases caused or mediated by acidic microenvironment. The diseases include a variety of cancers and a variety of cancer metastasis.

Tumor microenvironment is a target with obvious effect for anti-tumor drugs and inhibiting cancer metastasis, whereas the compound of the invention has significant activity in inhibiting cancer proliferation and metastasis, experiments have confirmed that these compounds have inhibitory effect on the proliferation of various cancer cells, and thus the compound of the invention is applicable for treating various cancers and cancer metastasis. Especially, it has better therapeutic effects on liver cancer, kidney cancer, prostate cancer, lung cancer, gastric cancer, ovarian cancer, colon cancer, pancreatic cancer, etc., and its metastasis. The treatment of the cancer metastasis caused by orthotopic tumor is very obvious and therapeutically effective.

The phosphate derivatives of the present invention, playing the role of tumor acidic microenvironment modulator, combining with clinical drugs, can effectively inhibit cancer proliferation and metastasis, and are therapeutically effective to a variety of cancers, especially liver cancer, kidney cancer, prostate cancer, lung cancer, gastric cancer, ovarian cancer, colon cancer, pancreatic cancer, etc., and its metastasis, and very minor side effect can be observed in all treatment, has a very broad application prospect.

### BRIEF DESCRIPTION OF THE DRAWINGS

The present invention will be further explained in details by combining with the drawings and embodiments.
FIG.1 is the fitted for the inhabitation on the proliferation of orthotopic xenograft HCC78 Hep3B2.1-7-Luc in Example 8 compound of the invention via method of Example 25 of the present invention;
FIG.2 is the Drug-Time graph of BALB\c nude mice ip injection of Example 8 compound of the present invention via method of Example 25 of the present invention; and
FIG.3 is the histograph of drug concentration distributed in organs and tissue after 24 hours ip injection of Example 8 compound of the invention via method of Example 25 of the present invention.

### DETAILED DESCRIPTION OF THE INVENTION

### Example 1 Synthesis of Compound 1 sodium (((((1,3-dihydroxy-2-(hydroxymethyl) propan-2-yl)azanediyl)bis(methylene))bis(4,1-phenylene))bis(methylene))bis(phosphonate) (not claimed)

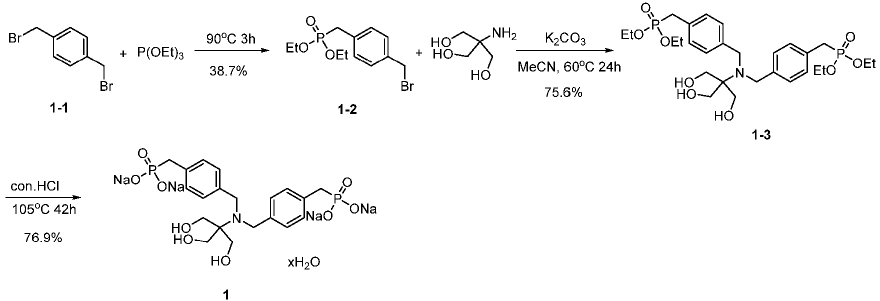

Step (1): To a 250 mL flask was added 1,4-bis(bromomethyl)-benzene 53.0 g (0.201 mol, triethyl phosphite 33.4 g (0.201 mol). The flask was heat up in an oil bath. The mixture was stirred for 3 hrs under 90°C. After removal of heating system, to the cool mixture was added 50 mL dichloromethane. The resulted mixture was kept stirring for 10 minutes, and was added 100 mL petroleum ether, and was stirred overnight. Then the suspension was filtered, the residue was washed with 100 mL solvent mixture of CH₂Cl₂ and petroleum ether (v/v, 1:10). The filtrate was collected and combined, concentrated to give a thick liquid which was purified by column chromatography to provide compound 1-2 diethyl (4-(bromomethyl)benzyl)phosphonate with 25.0g, 38.7% of yield; C1₂H₁₈BrO₃P, MS (ES+) m/z: 321.0 (M+H)⁺.

Step (2): To a 500 mL flask was added, compound 1-2 diethyl (4-(bromomethyl)benzyl) phosphonate with 25.0 g (77.9 mmol), Tris 4.29 g (35.4 mmol), anhydrous K₂CO₃ 12.2g (88.5 mmol) , 150 mL acetonitrile, and 150 mL acetonitrile. The mixture was stirred over 24 hrs under 60°C. After removal of heating system, to the cool mixture was added 50 mL dichloromethane. The resulted mixture was kept stirring for 10 minutes, and was added 100 mL CH₂Cl₂ twice, and was stirred overnight. Then the suspension was filtered, the residue was washed with 100 mL twice. The filtrate was collected and combined, concentrated to give a thick liquid which was purified by column chromatography to provide compound 1-3 tetraethyl (((((1,3-dihydroxy-2-(hydroxymethyl)propan-2-yl)azanediyl)bis(methylene))bis(4,1-phenylene))bis(methylene))bis(phosphonate) with 16.1 g, 75.6% yield; C₂₈H₄₅NO₉P₂, MS (ES+) m/z: 602.2 (M+H)⁺.

Step (3): To a 500 mL flask was added, compound 1-3 tetraethyl (((((1,3-dihydroxy-2-(hydroxymethyl)propan-2-yl)azanediyl)bis(methylene))bis(4,1-phenylene))bis(methylene)) bis(phosphonate) with 16.1 g (26.8 mmol), 150 mL aqueous concentrate HCl. The reaction mixture was heated to 105°C for 42 hrs, and keep stirring. After removal of heating system, to the cool mixture was concentrated to resulted a pale solid, which was purified by resin column. The resulted solid was dissolved in water, and was added 2N NaOH. After concentration the solid was recrystallized by ethanol to give a while solid, compound 1 sodium (((((1,3-dihydroxy-2-(hydroxymethyl)propan-2-yl)azanediyl)bis(methylene)) bis(4,1-phenylene))bis(methylene))bis(phosphonate), 16.0g with 76.9% yield; 1H NMR (500 MHz, D2O) δ 7.15 (s, 4H), 3.90 (s, 2H), 3.61 (s, 3H), 2.72 (d, J = 19.8 Hz, 2H); C₂₀H₂₅NNa₄O₉P₂•xH₂O, MS (ES+) m/z: 490.2 (M+H)⁺.

### Example 2 Synthesis of compound 2 sodium ((5-((bis(2-hydroxyethyl)amino)methyl)-1,3-phenylene)bis(ethane-2,1-diyl))bis(phosphonate)

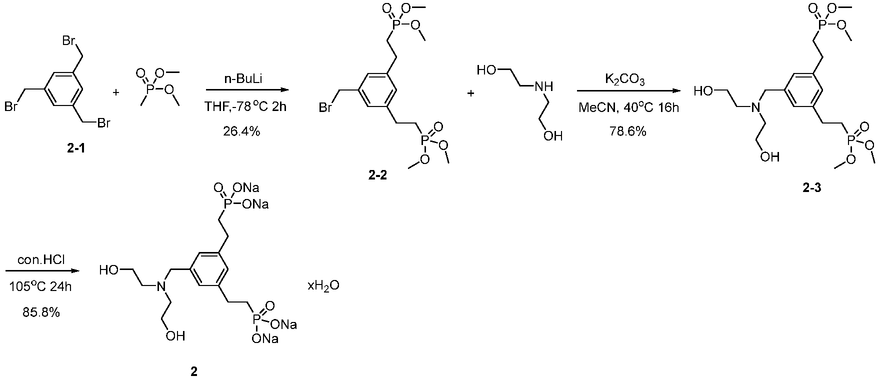

Step (1): To a 2000 mL three neck flask, was added 400 mL of anhydrate THF, dimethyl-methylphosphate 33.4 g (0.201 mol) 24.8 g (0.200 mol). The flask was cooled down to - 78°C via a dry ice-acetone bath, and was dropwise added 88 mL Butyl lithium in hexane (0.22 mol, 2.5 M) in 50 minutes. After keep stirring for another hour at -78°C, 1,3,5-tris(bromomethyl)benzene 35.7 g (0.100 mol) in 200 mL of THF was added in 30 minutes. The mixture was kept stirring for 1 hr. LCMS showed the major product is desired. The reaction mixture was quenched by monopotassium phosphate (1 M, 100 mL), and was warmed up to room temperature. The mixture of liquid was separated by filtration funnel. The aqueous phase was washed by 50 mL of solvent mixture chloroform and isopropylol (3: 1) with four times. The organic phase was combined and added Na₂SO₄, filtered and concentrated. The residue was purified to give compound 2-2 tetramethyl ((5-(bromomethyl)-1,3-phenylene)bis(ethane-2,1-diyl))bis(phosphonate) 11.7 g, with 26.4% yield, C₁₅H₂₅BrO₆P₂, MS (ES+) m/z: 443.0 (M+H)⁺.

Step (2): To a 50 mL flask, was added compound 2-2 tetramethyl ((5-(bromomethyl)-1,3-phenylene)bis(ethane-2,1-diyl))bis(phosphonate) 290 mg (0.653 mmol), diethanol amine 137 mg (1.31 mmol), and K₂CO₃ 90 mg (0.653 mmol),and 10 mL of acetonitrile. The mixture was stirring for 16hrs at 40 °C. After cooled down, the mixture was filtrated. The filter residue was washed with 10 mL dichloromethane twice. The combined liquid was concentrated to give a viscous liquid, which was purified with flash column chromatography to give compound 2-3 tetramethyl ((5-((bis(2-hydroxyethyl)amino)methyl)-1,3-phenylene)bis(ethane-2,1-diyl))bis(phosphonate) 240 mg with 78.6% yield; C₁₉H₃₅NO₈P₂, MS (ES+) m/z: 468.2 (M+H)⁺.

Step (3): To a 50 mL flask, was added compound 2-3 tetramethyl ((5-((bis(2-hydroxyethyl)amino)methyl)-1,3-phenylene)bis(ethane-2,1-diyl))bis(phosphonate) 240 mg (0.513 mmol), and 5 mL concentrated HCl. The mixture was heat up to reflux at 105 °C over 24 hrs under an oil bath. The mixture was cooled down and concentrated to give a solid. The solid was purified by resin column. The eluent concentrated and dried by lyophylizer. The solid was dissolved in water, and was added 2 N NaOH solution, concentration. And the solid was crystalized with ethanol to give compound 2 sodium ((5-((bis(2-hydroxyethyl)amino)methyl)-1, 3-phenylene)bis(ethane-2,1-diyl))bis(phosphonate) 250 mg with 85.8% yield; 1H NMR (500 MHz, D₂O) δ 7.08 (s, 1H), 7.01 (s, 2H), 3.66 (s, 2H), 3.59 (t, J = 6.2 Hz, 4H), 2.70-2.53 (m, 8H), 1.60-1.43 (m, 4H); C₁₅H₂₃NNa₄O₈P₂•xH₂O, MS (ES+) m/z: 412.1 (M+H)⁺.

### Example 3: Synthesis of Compound 3 sodium (((1,3-dihydroxy-2-(hydroxymethyl)propan-2-yl)azanediyl)bis(butane-4,1-diyl))bis(phosphonate)

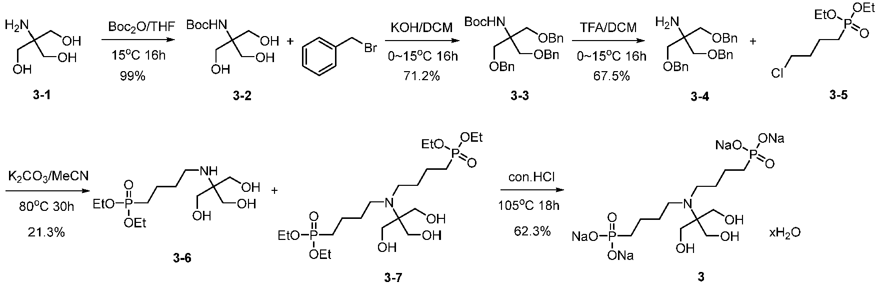

Step (1): To a 500 mg flask, was added tris amine 12.1 g (0.100 mol), 100 mL THF, and added dropwise Boc₂O 21.8 g (0.100 mol) in 80 mL THF at r.t within 30 min. The mixture was kept stirring for 16 hrs, and concentrated to give compound 3-2 tert-butyl (1,3-dihydroxy-2-(hydroxymethyl)propan-2-yl)carbamate 22.1 g with 99% yield as a viscous liquid, which was directly used for next step; C₉H₁₉NO₅, MS (ES+) m/z: 244.0 (M+Na)⁺.

Step (2): To a 1000 mL three-neck flask, was added compound 3-2 tert-butyl (1,3-dihydroxy-2-(hydroxymethyl)propan-2-yl)carbamate 22.1 g (0.100 mol), and 300 mL dichloromethane. The mixture was cooled to 0°C with ice-water bath. And powder of KOH 18.6g (0.32mol), and benzyl bromide 53.0g (0.31mol) was added. The mixture was kept stirring at 0-15°C for 16 hrs. After filtration, the organic phase was washed with water 3 × 200 mL, and dried over Na2SO4, and was concentration. The resulted solid was purified by silico gel column chromatography to give a viscous liquid Compound 3-3 tert-butyl (1,3-bis(benzyloxy)-2-((benzyloxy)methyl)propan-2-yl)carbamate 35.0g with 71.2% yield; C₃₀H₃₇NO₅, MS (ES+) m/z: 514.3 (M+Na)⁺.

Step (3): To a 500 mL three-neck flask, was added compound 3-3 tert-butyl (1,3-bis(benzyloxy)-2-((benzyloxy)methyl)propan-2-yl)carbamate 22.1g (0.100mol), 26.0g (52.9mmol), 120 mL dichloromethane. The mixture was cooled to 0°C with ice-water bath. And 40 mL trifluoroacetic acid was added. The mixture was kept stirring at 0-15°C for 16 hrs. The mixture was concentrated to give a residue. The residue was washed with petroleum ether, and was added 200 mL water, and added 5% aq. Sodium bicarbonate to make pH 9. The mixture then was extracted with dichloromethane 2x200 mL, then washed with 200 mL water, 200 mL brine, and was dried over Na2SO4.The solution was concentrated to give compound 3-4 1,3-bis(benzyloxy)-2-((benzyloxy)methyl)propan-2-amine 14.0g with 67.5% yield as a viscous liquid; C₃₀H₃₇NO₅, MS (ES+) m/z: 392.2 (M+H)⁺. Step (4): To a 100mg flask, was added compound 3-4 1,3-bis(benzyloxy)-2-((benzyloxy)methyl)propan-2-amine 782mg (2.00mmol), 3-5 915mg (4.00mmol) , K₂CO₃ 552mg (4.0mmol), and 20 mL acetonitrile. The mixture was kept stirring for 30 hrs at 80 degrees. The mixture was filtrated and concentrated to yield crude product, which was further purified by silico gel flash chromatography to give desired Compound 3-7 tetraethyl (((1,3-dihydroxy-2-(hydroxymethyl)propan-2-yl)azanediyl)bis(butane-4,1-diyl))bis (phosphonate) 608mg with 39.2% yield; C₄₁H₆₃NO₉P, MS (ES+) m/z: 776.4 (M+H)⁺; and compound 3-6 249mg with 21.3% yield; C₃₀H₃₇NO₅, MS (ES+) m/z: 584.3 (M+H)⁺.

Step (5): To a 100 mL flask, was added compound 3-7 tetraethyl (((1,3-dihydroxy-2-(hydroxymethyl)propan-2-yl)azanediyl)bis(butane-4,1-diyl))bis(phosphonate) 608mg (0.784 mmol), and 10 mL concentrated HCl. The mixture was heat up to reflux at 105 °C over 18 hrs under an oil bath. The mixture was cooled down and concentrated to give a solid. The solid was purified by resin column. The eluent concentrated and dried by lyophylizer. The solid was dissolved in water, and was added 2N NaOH solution, concentration. And the solid was crystalized with ethanol to give compound 3 sodium (((1,3-dihydroxy-2-(hydroxymethyl)propan-2-yl)azanediyl)bis(butane-4,1-diyl))bis (phosphonate) 279mg with 62.3% yield as a while solid; C₁₂H₂₅NNa₄O₉P₂•xH₂O, MS (ES+) m/z: 394.1 (M+H)⁺.250mg with 85.8% yield; 1H NMR (500 MHz, D2O) δ 7.08 (s, 1H), 7.01 (s, 2H), 3.66 (s, 2H), 3.59 (t, J = 6.2 Hz, 4H), 2.70-2.53 (m, 8H), 1.60-1.43 (m, 4H); C₁₅H₂₃NNa₄O₈P₂•xH₂O, MS (ES+) m/z: 412.1 (M+H)⁺

### Example 4: Synthesis of Compound 4 sodium (((((2-hydroxyethyl)azanediyl)bis (methylene))bis(4,1-phenylene))bis(ethane-2,1-diyl))bis(phosphonate)

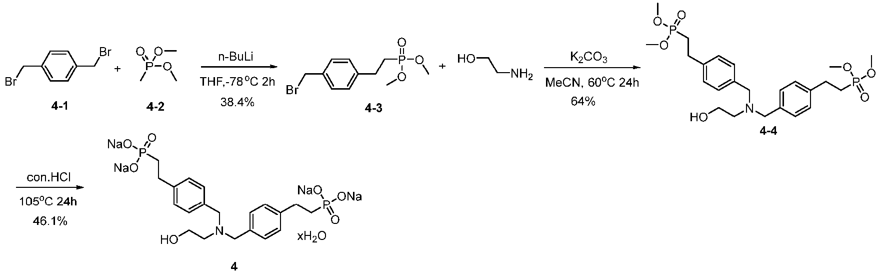

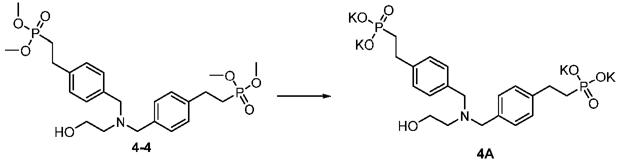

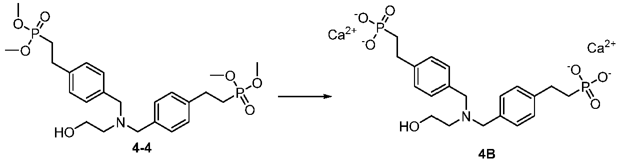

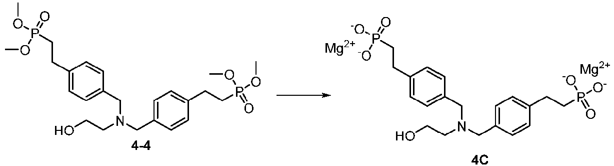

Step (1): To a 1000 mL three-neck flask, was added 400 mL of anhydrate THF, dimethyl-methylphosphate (12.4 g, 100 mmol). The flask was cooled down to -78°C via a dry ice-acetone bath, and was dropwise added 40 mL butyl lithium in hexane (100 mmol, 2.5M) in 30 minutes. After keep stirring for another hour at -78°C, 1, 4-di (bromomethyl) benzene (26.4 g, 100 mmol) in 200 mL of THF was added in 30 minutes. The mixture was kept stirring for 1hr. LCMS showed the major product is desired. The reaction mixture was quenched by monopotassium phosphate (1M, 100 mL), and was warmed up to room temperature. The mixture of liquid was separated by filtration funnel. The aqueous phase was washed by 50 mL of solvent mixture chloroform and isopropylol (3: 1) with four times. The organic phase was combined and added Na₂SO₄, filtered and concentrated. The residue was purified to give compound **4-3** dimethyl (4-(bromomethyl)phenethyl) phosphonate 11.8 g, with 38.4% yield; C₁₁H₁₆BrO₃P, MS (ES+) m/z: 307.0 (M+H)⁺. Step (2): To a 50 mL flask was added, compound **4-3** dimethyl (4-(bromomethyl)phenethyl)phosphonate with 675 mg (2.2 mmol), ethanol amine 61mg (1.0 mmol), K₂CO₃ (304mg, 2.2 mmol) and 10 mL acetonitrile. The mixture was stirred over 24 hrs under 60°C. After removal of heating system, to the cool mixture was added 50 mL dichloromethane. The resulted mixture was kept stirring for 10 minutes, and was added 10 mL CH2Cl2 twice, and was stirred overnight. Then the suspension was filtered, the residue was washed with 10 mL twice. The filtrate was collected and combined, concentrated to give a thick liquid which was purified by column chromatography to provide compound **4-4** tetramethyl (((((2-hydroxyethyl)azanediyl)bis(methylene))bis(4,1-phenylene))bis(ethane-2,1-diyl))bis(phosphonate) with 330 mg with 64.0% yield as a colorless liquid: C₂₄H₃7NO₇P₂, MS (ES+) m/z: 514.2 (M+H)⁺.

Step (3): To a 50 mL flask was added, compound **4-4** tetramethyl (((((2-hydroxyethyl)azanediyl)bis(methylene))bis(4,1-phenylene))bis(ethane-2,1-diyl))bis(phosphonate) with 32 8mg (0.638 mmol), 10 mL aqueous concentrate HCl. The reaction mixture was heated to 105°C for 24 hrs, and keep stirring. After removal of heating system, to the cool mixture was concentrated to resulted a pale solid, which was purified by resin column. The resulted solid was dissolved in water, and was added 2N NaOH. After concentration the solid was recrystallized by ethanol to give a while solid, compound **4** sodium (((((2-hydroxyethyl)azanediyl)bis(methylene))bis(4,1-phenylene))bis(ethane-2,1-diyl))bis(phosphonate), 200 mg, 46.1%; C₂₀H₂₅NNa₄O₇P₂•xH₂O, MS (ES+) m/z: 458.1 (M+H)⁺.

Step (4): To a 50 mL flask was added, compound **4-4** tetramethyl (((((2-hydroxyethyl) azanediyl)bis(methylene))bis(4,1-phenylene))bis(ethane-2,1-diyl))bis(phosphonate) with 328 mg (0.638 mmol), 10 mL aqueous concentrate HCl. The reaction mixture was heated to 105°C for 24 hrs, and keep stirring. After removal of heating system, to the cool mixture was concentrated to resulted a pale solid, which was purified by resin column. The resulted solid was dissolved in water, and was added 2N KOH. After concentration the solid was recrystallized by ethanol to give a while solid, compound **4A** potassium (((((2-hydroxyethyl)azanediyl)bis(methylene))bis(4,1-phenylene))bis(ethane-2,1-diyl))bis (phosphonate), 160 mg.

Step (5): To a 50 mL flask was added, compound **4-4** tetramethyl (((((2-hydroxyethyl) azanediyl)bis(methylene))bis(4,1-phenylene))bis(ethane-2,1-diyl))bis(phosphonate) with 328 mg (0.638 mmol), 10 mL aqueous concentrate HCl. The reaction mixture was heated to 105°C for 24 hrs, and keep stirring. After removal of heating system, to the cool mixture was concentrated to resulted a pale solid, which was purified by resin column. The resulted solid was dissolved in water, and was added 2 equivalent of Ca(OH)₂. After concentration the solid was recrystallized by ethanol to give a while solid, compound **4B** calcium (((((2-hydroxyethyl)azanediyl)bis(methylene))bis(4,1-phenylene))bis(ethane-2,1-diyl))bis(phosphonate), 100 mg.

Step (6): To a 50 mL flask was added, compound **4-4** tetramethyl (((((2-hydroxyethyl) azanediyl)bis(methylene))bis(4,1-phenylene))bis(ethane-2,1-diyl))bis(phosphonate) with 328 mg (0.638 mmol), 10 mL aqueous concentrate HCl. The reaction mixture was heated to 105°C for 24 hrs, and keep stirring. After removal of heating system, to the cool mixture was concentrated to resulted a pale solid, which was purified by resin column. The resulted solid was dissolved in water, and was added 2 equivalent of Mg(OH)2. After concentration the solid was recrystallized by ethanol to give a while solid, compound **4C** magnesium (((((2-hydroxyethyl)azanediyl)bis(methylene))bis(4,1-phenylene))bis(ethane-2,1-diyl))bis(phosphonate), 80 mg.

### Example 5: Synthesis of Compound 5 sodium (((((2-hydroxyethyl)azanediyl) bis(methylene))bis(benzene-5,1,3-triyl))tetrakis(ethane-2,1-diyl))tetrakis(phosphonate)

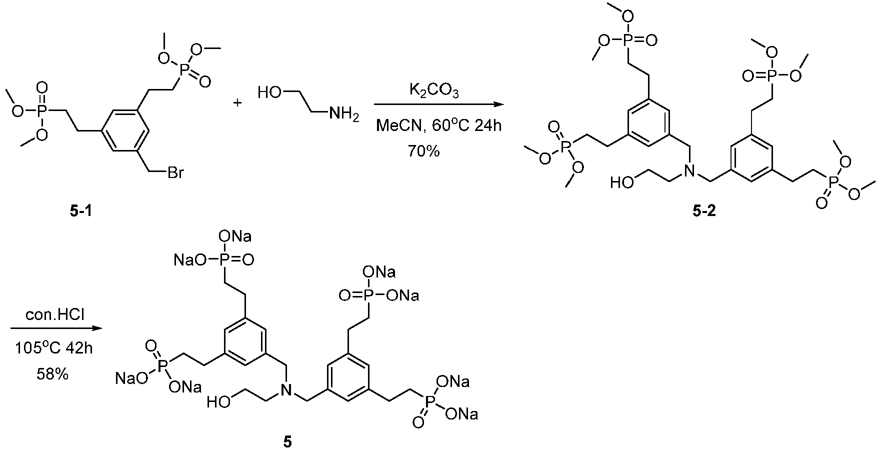

Step (1): To a 50 mL flask was added, compound **5-1** tetramethyl ((5-(bromomethyl)-1,3-phenylene)bis(ethane-2,1-diyl))bis(phosphonate)with 972mg (2.2 mmol), ethanol amine 61mg (1.0 mmol), K₂CO₃ (304mg, 2.2 mmol),and 20 mL acetonitrile. The mixture was stiied over 24 hrs under 60°C. After removal of heating system, to the cool mixture was added 50 mL dichloromethane. The resulted mixture was kept stirring for 10 minutes, and was added 20 mL CH₂Cl₂ twice, and was stirred overnight. Then the suspension was filtered, the residue was washed with 20 mL twice. The filtrate was collected and combined, concentrated to give a thick liquid which was purified by column chromatography to provide compound **5-2** octamethyl (((((2-hydroxyethyl)azanediyl)bis(methylene))bis (benzene-5,1,3-triyl))tetrakis(ethane-2,1-diyl))tetrakis(phosphonate) 550 mg with 70% yield, as a colorless liquid: C₃₂H₅₅NO₁₃P₄, MS (ES+) m/z: 786.3 (M+H)⁺.

Step (2): To a 50 mL flask was added, compound **5-2** octamethyl (((((2-hydroxyethyl) azanediyl)bis(methylene))bis(benzene-5,1,3-triyl))tetrakis(ethane-2,1-diyl))tetrakis (phosphonate) 550mg (0.7mmol), 10 mL aqueous concentrate HCl. The reaction mixture was heated to 105°C for 42 hrs, and was kept stirring. After the removal of heating system, the cool mixture was concentrated to result a pale solid, which was purified by resin column. The resulted solid was dissolved in water, and was added 2N NaOH. After concentration the solid was recrystallized by ethanol to give a while solid, compound **5** sodium (((((2-hydroxyethyl)azanediyl)bis(methylene))bis(benzene-5,1,3-triyl))tetrakis (ethane-2,1-diyl))tetrakis(phosphonate), 390mg, 58% yield: C₂₄H₃₁NNa₈O₁₃P₄•xH₂O, MS (ES+) m/z: 674.1 (M+H)⁺.

### Example 6: Synthesis of Compound 6 sodium (((((1,3-dihydroxy-2-(hydroxymethyl) propan-2-yl)azanediyl)bis(methylene))bis(benzene-5,1,3-triyl))tetrakis(methylene)) tetrakis(phosphonate)

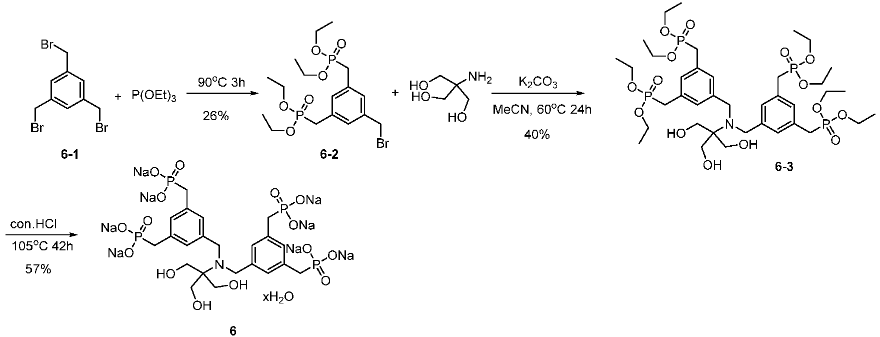

Step (1): To a 50 mL flask, was added 1,3,5-tris(bromomethyl)-benzene 7.14g (20.0 mmol), triethyl phosphite 6.68g (40.2 mmol). The flask was heated up in an oil bath. The mixture was stirred for 3 hrs under 90°C. After removal of heating system, to the cool mixture was added 30 mL dichloromethane. The resulted mixture was kept stirring for 10 minutes, and was added 30 mL petroleum ether, and was stirred overnight. Then the suspension was filtered, the residue was washed with 30 mL solvent mixture of CH₂Cl₂ and petroleum ether (v/v, 1:5). The filtrate was collected and combined, concentrated to give a thick liquid which was purified by column chromatography to provide compound **6-2** tetraethyl ((5-(bromomethyl)-1,3-phenylene)bis(methylene))bis(phosphonate) 2.45g,26% yield: C₁₇H₂₉BrO₆P₂, MS (ES+) m/z: 493.0 (M+Na)⁺.

Step (2): To a 50 mL flask was added, compound **6-2** tetraethyl ((5-(bromomethyl)-1,3-phenylene)bis(methylene))bis(phosphonate) (1040 mg, 2.2 mmol), tris amine (121 mg, 1.0 mmol), K₂CO₃ (304 mg, 2.2 mmol),and 15 mL acetonitrile. The mixture was stirred over 24 hrs under 60°C. After removal of heating system, to the cool mixture was added 50 mL dichloromethane. The resulted mixture was kept stirring for 10 minutes, and was added 20 mL CH₂Cl₂ twice, and was stirred overnight. Then the suspension was filtered, the residue was washed with 20 mL twice. The filtrate was collected and combined, concentrated to give a thick liquid which was purified by column chromatography to provide compound **6-3** octaethyl (((((1,3-dihydroxy-2-(hydroxymethyl)propan-2-yl)azanediyl)bis(methylene))bis(benzene-5,1,3-triyl))tetrakis(methylene))tetrakis (phosphonate), 360 mg with 40% yield, as a colorless liquid: C₃₈H₆₇NO₁₅P₄, MS (ES+) m/z: 902.4 (M+H)⁺.

Step (3): To a 50 mL flask was added, compound **6-3** octaethyl (((((1,3-dihydroxy-2-(hydroxymethyl)propan-2-yl)azanediyl)bis(methylene))bis(benzene-5,1,3-triyl))tetrakis(methylene))tetrakis(phosphonate) with (360 mg, 0.4 mmol), 10 mL aqueous concentrate HCl. The reaction mixture was heated to 105°C for 42 hrs, and was kept stirring. After the removal of heating system, the cool mixture was concentrated to result a pale solid, which was purified by resin column. The resulted solid was dissolved in water, and was added 2N NaOH. After concentration the solid was recrystallized by ethanol to give a while solid, compound **6** sodium (((((1,3-dihydroxy-2-(hydroxymethyl)propan-2-yl)azanediyl)bis(methylene))bis(benzene-5,1,3-triyl))tetrakis(methylene))tetrakis (phosphonate), 210mg, with 57% yield: C₂₂H₂₇NNa₈O₁₅P₄•xH₂O, MS (ES+) m/z: 678.1 (M+H)⁺.

### Example 7: Synthesis of Compound 7 sodium (((((1,3-dihydroxy-2-(hydroxymethyl) propan-2-yl)azanediyl)bis(methylene))bis(benzene-5,1,3-triyl))tetrakis(ethane-2,1-diyl))tetrakis(phosphonate)

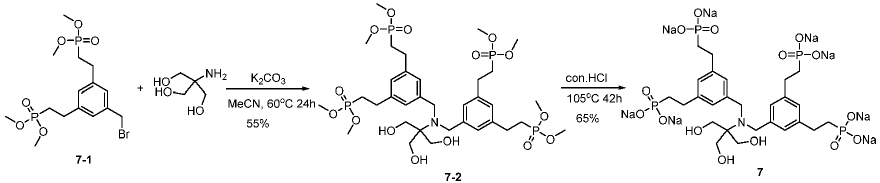

Step (1): To a 50 mL flask was added, compound **7-1** tetramethyl ((5-(bromomethyl)-1,3-phenylene)bis(ethane-2,1-diyl))bis(phosphonate) (972 mg, 2.2 mmol), tris amine (121 mg, 1.0 mmol), K₂CO₃ (304 mg, 2.2 mmol),and 15 mL acetonitrile. The mixture was stirred over 24 hrs under 60°C. After removal of heating system, to the cool mixture was added 50 mL dichloromethane. The resulted mixture was kept stirring for 10 minutes, and was added 20 mL CH₂Cl₂ twice, and was stirred overnight. Then the suspension was filtered, the residue was washed with 20 mL twice. The filtrate was collected and combined, concentrated to give a thick liquid which was purified by column chromatography to provide compound **7-2** octamethyl (((((1,3-dihydroxy-2-(hydroxymethyl)propan-2-yl)azanediyl)bis(methylene))bis(benzene-5,1,3-triyl))tetrakis(ethane-2,1-diyl))tetrakis(phosphonate) 465 mg with 55% yield, as a colorless liquid: C₃₄H₅₉NO₁₅P₄, MS (ES+) m/z: 846.3 (M+H)⁺.

Step (2): To a 50 mL flask was added, compound **7-2** octamethyl (((((1,3-dihydroxy-2-(hydroxymethyl)propan-2-yl)azanediyl)bis(methylene))bis(benzene-5,1,3-triyl))tetrakis(ethane-2,1-diyl))tetrakis(phosphonate) (465 mg, 0.55 mmol), 10 mL aqueous concentrate HCl. The reaction mixture was heated to 105°C for 42 hrs, and was kept stirring. After the removal of heating system, the cool mixture was concentrated to result a pale solid, which was purified by resin column. The resulted solid was dissolved in water, and was added 2N NaOH. After concentration the solid was recrystallized by ethanol to give a while solid, compound **7** sodium (((((1,3-dihydroxy-2-(hydroxymethyl)propan-2-yl)azanediyl)bis(methylene))bis(benzene-5,1,3-triyl))tetrakis(ethane-2,1-diyl))tetrakis(phosphonate), 360mg, with 65% yield: C₂₆H₃₅NNa₈O₁₅P₄•xH₂O, MS (ES+) m/z: 606.2 (M+H)⁺.

### Example 8: Synthesis of Compound 8 sodium ((5-(((1,3-dihydroxy-2-(hydroxymethyl) propan-2-yl)amino)methyl)-1,3-phenylene)bis(ethane-2,1-diyl))bis(phosphonate)

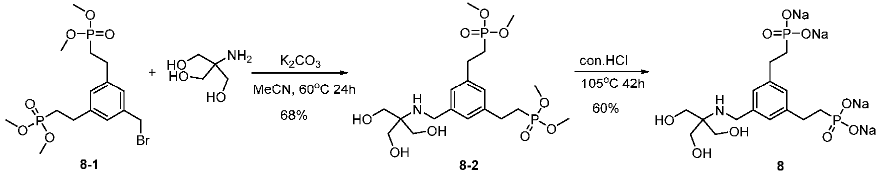

Step (1): To a 50 mL flask was added, compound **8-1** tetramethyl ((5-(bromomethyl)-1,3-phenylene)bis(ethane-2,1-diyl))bis(phosphonate) (663 mg, 1.5 mmol), tris amine (363 mg, 3.0 mmol), K₂CO₃ (207 mg, 1.5 mmol),and 10 mL acetonitrile. The mixture was stirred over 16 hrs under 60°C. After removal of heating system, to the cool mixture was added 50 mL dichloromethane. The resulted mixture was kept stirring for 10 minutes, and was added 20 mL CH₂Cl₂ twice, and was stirred overnight. Then the suspension was filtered, the residue was washed with 20 mL twice. The filtrate was collected and combined, concentrated to give a thick liquid which was purified by column chromatography to provide compound **8-2** tetramethyl ((5-(((1,3-dihydroxy-2-(hydroxymethyl)propan-2-yl)amino)methyl)-1,3-phenylene)bis(ethane-2,1-diyl))bis(phosphonate) 465 mg with 55% yield, as a colorless liquid: C₃₄H₅₉NO₁₅P₄, MS (ES+) m/z: 846.3 (M+H)⁺.

Step (2): To a 50 mL flask was added, compound **8-2** tetramethyl ((5-(((1,3-dihydroxy-2-(hydroxymethyl)propan-2-yl)amino)methyl)-1,3-phenylene)bis(ethane-2,1-diyl))bis (phosphonate) (493 mg, 1.02 mmol), 10 mL aqueous concentrate HCl. The reaction mixture was heated to 105°C for 24 hrs, and was kept stirring. After the removal of heating system, the cool mixture was concentrated to result a pale solid, which was purified by resin column. The resulted solid was dissolved in water, and was added 2N NaOH. After concentration the solid was recrystallized by ethanol to give a while solid, compound **8** sodium ((5-(((1,3-dihydroxy-2-(hydroxymethyl)propan-2-yl)amino)methyl)-1,3-phenylene) bis(ethane-2,1-diyl))bis(phosphonate), 349 mg, with 60% yield: C₁₅H₂₃NNa₄O₉P₂•xH₂O, MS (ES+) m/z: 428.1 (M+H)⁺.

### Example 9: Synthesis of Compound 9 sodium ((5-(((1,3-dihydroxy-2-(hydroxymethyl) propan-2-yl)(methyl)amino)methyl)-1,3-phenylene)bis(ethane-2,1-diyl))bis(phosphonate)

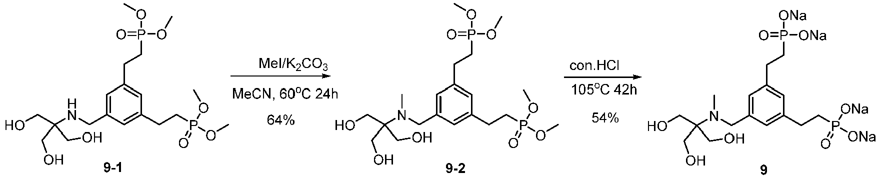

Step (1): To a 10 mL microwave tube was added, compound 9-1 tetramethyl ((5-(((1,3-dihydroxy-2-(hydroxymethyl)propan-2-yl)amino)methyl)-1,3-phenylene)bis(ethane-2,1-diyl))bis(phosphonate) (450 mg, 0.93 mmol), Methyl iodide (284 mg, 2.0 mmol), K₂CO₃ (276 mg, 2.0 mmol), and 4 mL acetonitrile. The mixture was warmed to 60 degrees, and was kept stirring for 2 hrs. The reaction mixture was cooled to room temperature, filtrated. The filter residue was washed with dichloromethane 2 × 10 mL. The combined filtrate was concentrated to give a viscous liquid, which was purified by column chromatography to give compound **9-2** tetramethyl ((5-(((1,3-dihydroxy-2-(hydroxymethyl)propan-2-yl)(methyl)amino)methyl)-1,3-phenylene)bis(ethane-2,1-diyl))bis(phosphonate) 300 mg with 64% yield, as a colorless liquid: C₂₀H₃₇NO₉P₂, MS (ES+) m/z: 498.2 (M+H)⁺.

Step (2): To a 50 mL flask was added, compound **9-2** tetramethyl ((5-(((1,3-dihydroxy-2-(hydroxymethyl)propan-2-yl)(methyl)amino)methyl)-1,3-phenylene)bis(ethane-2,1 - diyl))bis(phosphonate) (300 mg, 0.6 mmol), 10 mL aqueous concentrate HCl. The reaction mixture was heated to 105°C for 24 hrs, and was kept stirring. After the removal of heating system, the cool mixture was concentrated to result a pale solid, which was purified by resin column. The resulted solid was dissolved in water, and was added 2N NaOH. After concentration the solid was recrystallized by ethanol to give a while solid, compound **9** sodium ((5-(((1,3-dihydroxy-2-(hydroxymethyl)propan-2-yl)(methyl)amino)methyl)-1,3-phenylene)bis(ethane-2,1-diyl))bis(phosphonate), 170 mg, with 54% yield: C₁₆H₂₅NNa₄O₉P₂•xH₂O, MS (ES+) m/z: 442.1 (M+H)⁺.

### Example 10: Synthesis of Compound 10 sodium (((((1-butoxy-3-hydroxy-2-(hydroxymethyl)propan-2-yl)azanediyl)bis(methylene))bis(benzene-5,1,3-triyl))tetrakis(ethane-2,1-diyl))tetrakis(phosphonate)

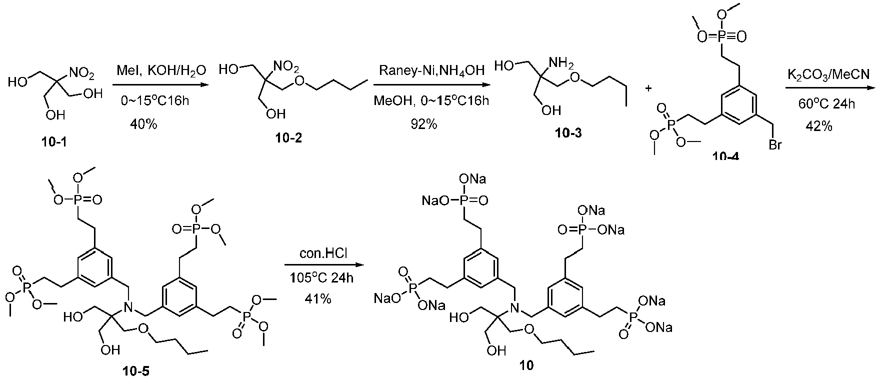

Step (1): To a 250 mL flask, was added **10-1** 2-(hydroxymethyl)-2-nitropropane-1,3-diol (15.1 g, 0.100 mol). The mixture was cooled down to zero degree, and was added dropwise aq. 50% KOH (50 mL), and kept stirring for 30 min. Bromo-butane (6.85 g, 50 mmol) was added to the mixture at 0 °C. The reaction mixture was kept stirring for 16 hrs at 0-15 °C. The cool reaction mixture was then poured into 200 mL sat. NH4CI. The resulted mixture was extracted with dichloromethane and isopropylol (v/v, 10:1) three times. The organic phases were combined and concentrated to result a thick liquid, which was purified by column chromatography to give compound **10-2** 2-(butoxymethyl)-2-nitropropane-1,3-diol 4.14 g with 40% yield as a pale yellow liquid: C₈H₁₇NO₅, MS (ES+) m/z: 230.1 (M+Na)⁺.

Step (2): Compound **10-2** 2-(butoxymethyl)-2-nitropropane-1,3-diol (4.14 g, 40 mmol) was dissolved in 80 mL methanol. And Raney nickel (800 mg), 4 mL concentrated ammonia was added to the solution. The mixture was furnished with hydrogen column at 101325 Pa (1 atm), and was stirred for 16 hrs at 30 °C. Then, the mixture was filtrated. The residue was washed with methanol (2 × 30 mL). The organic solution was combined and concentrated to result the desired compound **10-3** 2-amino-2-(butoxymethyl)propane-1,3-diol 3.3 g, with 92% yield as a while solid: C₈H₁₉NO₃, MS (ES+) m/z: 178.1 (M+H)⁺.

Step (3) : synthesis of 2, 2', 2"-(5,5'-(1-butoxy-3-hydroxy-2-(hydroxymethyl) propane-2-n-azane diyl) dimethyl (methylene) dimethyl (phenane-5, 3, 1-triyl) tetraphosphonic acid octyl ester. Weighing 2, 2'- (5 - bromine (meth) - 1, 3 - phenylene) double (2, 1 - two base ethane) four methyl phosphonic acid 972 mg (2.2 mmol), 2 - amino - 2 - methyl - 1, 3 - butyl oxygen propylene glycol 178 mg (1.0 mmol), anhydrous potassium carbonate 304 mg (2.2 mmol), 10 ml of acetonitrile in the volume of 50 ml of stand-up round bottom flask, 60 °C in the oil bath heating magnetic stirring for 24 hours. The reaction liquid was cooled, filtered, filtrate was collected, and the filtrate residue was washed twice with 10mL dichloromethane. Merge filtrate, spin steaming for viscous liquid, then after column chromatography purification by 2, 2 ', 2 ', 2 '- (5, 5' - (1 - butoxy - 3 - hydroxy - 2 - (hydroxymethyl) propane - 2 - n alkanes 2) 2 (methylene) 2 (alkyl benzene - 5,3,1 - sanki)) 4-2, 1-2 (ethane) 4 methyl phosphonic acid, eight colourless liquid 380 mg, production rate is 42%; C38H67NO15P4, MS (ES+) m/ Z: 902.3 (m +H) +.

Step (4): Refer to step (2) of Example 8, to obtain Compound **10** sodium (((((1-butoxy-3-hydroxy-2-(hydroxymethyl)propan-2-yl)azanediyl)bis(methylene))bis(benzene-5,1,3-triyl))tetrakis(ethane-2,1-diyl))tetrakis(phosphonate), 180 mg, 41% yield, as a while solid: C₃₀H₄₃NNa₈O₁₅P₄•xH₂O, MS (ES+) m/z: 790.2 (M+H)⁺.

### Example 11: Synthesis of Compound 11 sodium ((5-(((1-butoxy-3-hydroxy-2-(hydroxymethyl)propan-2-yl)amino)methyl)-1,3-phenylene)bis(ethane-2,1-diyl))bis(phosphonate)

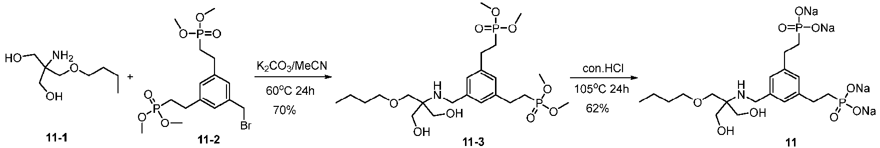

Step (1): Refer to step (1) of Example 8, to obtain compound **11-3** tetramethyl ((5-(((1-butoxy-3-hydroxy-2-(hydroxymethyl)propan-2-yl)amino)methyl)-1,3-phenylene)bis (ethane-2,1-diyl))bis(phosphonate), 567 mg,70% yield, as a colorless liquid: C₂₃H₄₃NO₉P₂, MS (ES+) m/z: 540.2 (M+H)⁺.

Step (2): Refer to step (2) of Example 8, to obtain Compound **11** sodium ((5-(((1-butoxy-3-hydroxy-2-(hydroxymethyl)propan-2-yl)amino)methyl)-1,3-phenylene)bis(ethane-2,1-diyl))bis(phosphonate), 160 mg, 62% yield, as a while solid: C₁₉H₃₁NNa₄O₉P₂ •xH₂O, MS (ES+) m/z: 484.2 (M+H)⁺.

### Example 12: Synthesis of Compound 12 sodium (((((1,3-dihydroxy-2-(hydroxymethyl) propan-2-yl)azanediyl)bis(methylene))bis(pyridine-6,3-diyl))bis(methylene))bis (phosphonate)

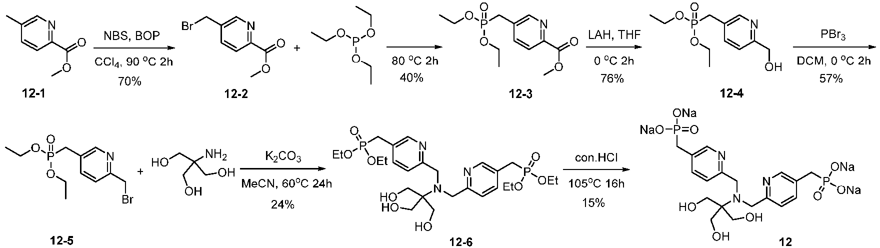

Step (1): Synthesis of 5- (bromomethyl) pyridine formate. Weigh 5- (methyl) pyridinium formate 7.56g (50.0mmol), N- bromosuccinimide 9.79g (55mmol), 100ml carbon tetrachloride in a single-mouth round bottom flask with a volume of 500ml, heat magnetic stirring at 90°C for 10 minutes in an oil bath, and add 650mg (2.5mmol) of dibenzoyl peroxide. The reaction fluid continued to reflux for 2 hours. After cooling, add 100mL dichloromethane, 100ml water wash, wash twice with 5% sodium bicarbonate, 100ml water wash, dry, filter, spin dry, and get the crude product. Purified by silica gel column chromatography, the viscous liquid of 5- (bromomethyl) pyridine formate was 8.05g with a yield of 70%. C₈H₈BrNO₂, MS (ES+) m/ Z: 252 (M+Na) +.

Step (2): To a 100 mL flask, was added, compound **12-2** methyl 5-(bromomethyl)picolinate (8.05 g, 35 mmol), triethylphosphite (8.35 g, 50 mmol). The mixture was heated to 90 degree, and stirred for 3 hrs, and then cooled to r.t, and was concentrated to result a thick liquid which was purified by flash column chromatography to give compound **12-3** methyl 5-((diethoxyphosphoryl)methyl)picolinate, 4.02 g, with 40% yield as a viscous liquid: C₁₂H₁₈NO₅P, MS (ES+) m/z: 288.1 (M+H)⁺.

Step (3): Compound **12-3** methyl 5-((diethoxyphosphoryl)methyl)picolinate (3.75 g, 13.1 mmol) was dissolved in 50 mL THF in a 250 mL three-neck flask. The solution was cooled to 0°C, and was added dropwise 1 M LiAlH₄ (15.6 mL, 15.6 mmol). And the reaction was stirred over 2 hrs at 0 °C. Na₂SO₄•10H₂O was added to the cold solution to quench the reaction. And the resulted mixture was filtrate. The residue was washed with FTE (2 × 50 mL). The organic solution was combined and dried over Na2SO4, and filtrated, and concentrated. The produced residue was purified by flash chromatography to give compound **12-4** diethyl ((6-(hydroxymethyl)pyridin-3-yl)methyl)phosphonate, 2.59 g, with 76% yield as a sticky liquid; C₁₁H₁₈NO₄P, MS (ES+) m/z: 260.1 (M+H)⁺.

Step (4): To a 250 mL flask, was added, compound **12-4** diethyl ((6-(hydroxymethyl)pyridin-3-yl)methyl)phosphonate (2.25 g, 8.7 mmol), and 50 mL DCM. The solution was cooled to 0°C, and, and was added PBr3 (4.72g,17.4 mmol), and kept stirring for 3 hrs. The solution was poured into 30 g ice, and was neutralized by 5% NaHCO3. The mixture was extracted by DCM (3 × 60 mL). The organic phases were combined and dried over Na2SO4, filtered, and concentrated in low temperature to give a stick liquid which was washed with DCM and petroleum ether to give compound **12-5** diethyl ((6-(bromomethyl)pyridin-3-yl)methyl)phosphonate, 1.6 g, with 57% yield: C₁₁H₁₇BrNO₃P, MS (ES+) m/z: 322.0 (M+H)⁺.

Step (5): Refer to step (1) of Example 8, to obtain **12-6** tetraethyl (((((1,3-dihydroxy-2-(hydroxymethyl)propan-2-yl)azanediyl)bis(methylene))bis(pyridine-6,3-diyl))bis(methylene))bis(phosphonate), 320 mg, 24% yield, as a colorless liquid: C₂₈H₄₃N₃O₉P₂, MS (ES+) m/z: 604.2 (M+H)⁺.

Step (6): Refer to step (2) of Example 8, to obtain Compound **12** sodium (((((1,3-dihydroxy-2-(hydroxymethyl)propan-2-yl)azanediyl)bis(methylene))bis(pyridine-6,3-diyl))bis(methylene))bis(phosphonate), 50 mg, 15% yield, as a while solid: C₁₈H₂₃N₃Na₄O₉P₂•xH₂O, MS (ES+) m/z: 492.1 (M+H)⁺.

### Example 13: Synthesis of Compound 13 sodium (((((1,3-dihydroxy-2-(hydroxymethyl)propan-2-yl)azanediyl)bis(methylene))bis(4,1-phenylene))bis(propane-1,2-diyl))bis(phosphonate)

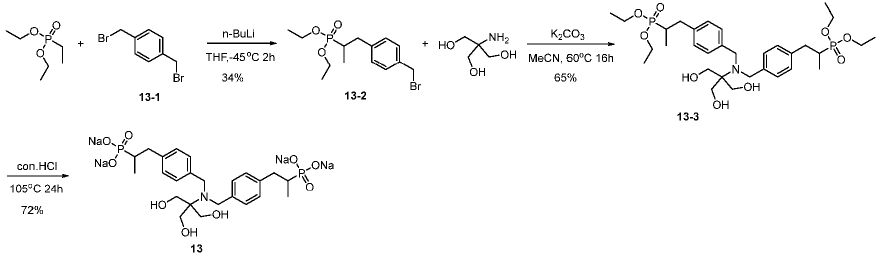

Step (1): To a 250 mL three neck flask, was added 40 mL of anhydrate THF, diethyl ethylphosphate (1.66 g, 10 mmol). The flask was cooled down to -78°C via a dry ice-acetone bath, and was dropwise added 4 mL butyl lithium in hexane (10 mmol, 2.5M) in 30 minutes. After keep stirring for another hour at -78°C, 1, 4-di (bromomethyl) benzene (2.64 g, 10 mol) in 10 mL of THF was added in 30 minutes. The mixture was kept stirring for 1hr. LCMS showed the major product is desired. The reaction mixture was quenched by monopotassium phosphate (1 M, 40 mL), and was warmed up to room temperature. The mixture of liquid was separated by filtration funnel. The aqueous phase was washed by 50 mL of solvent mixture chloroform and isopropylol (3: 1) with four times. The organic phase was combined and added Na2SO4, filtered and concentrated. The residue was purified by flash chromatography to give compound 13-2 diethyl (1-(4-(bromomethyl)phenyl)propan-2-yl)phosphonate **12 g,** 34% yield as a yellow oil: C₁₄H₂₂BrO₃P, MS (ES+) m/z: 349.0 (M+H)⁺..

Step (2): Refer to step (1) of Example 8, to obtain **13-3** tetraethyl (((((1,3-dihydroxy-2-(hydroxymethyl)propan-2-yl)azanediyl)bis(methylene))bis(4,1-phenylene))bis(propane-1,2-diyl))bis(phosphonate), 672 mg, 65% yield, as a colorless liquid: C₃₂H₅₃NO₉P₂, MS (ES+) m/z: 658.3 (M+H)⁺.

Step (3): Refer to step (2) of Example 8, to obtain Compound **13** sodium (((((1,3-dihydroxy-2-(hydroxymethyl)propan-2-yl)azanediyl)bis(methylene))bis(4,1-phenylene))bis(propane-1,2-diyl))bis(phosphonate), 514 mg, 72% yield, as a while solid: C₂₄H₃₃NNa₄O₉P₂•xH₂O, MS (ES+) m/z: 546.2 (M+H)⁺.

### Example 14: Synthesis of Compound 14 sodium (((((1,3-dihydroxy-2-methylpropan-2-yl)azanediyl)bis(methylene))bis(4,1-phenylene))bis(ethane-2,1-diyl))bis(phosphonate)

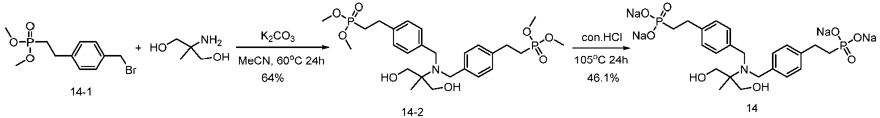

Step (1): Refer to step (1) of Example 8, to obtain **14-2** tetramethyl (((((1,3-dihydroxy-2-methylpropan-2-yl)azanediyl)bis(methylene))bis(4,1-phenylene))bis(ethane-2,1-diyl))bis(phosphonate), 380 mg, 68% yield, as a colorless liquid: C₂₆H₄₁NO₈P₂, MS (ES+) m/z: 558.2 (M+H)⁺.

Step (2): Refer to step (2) of Example 8, to obtain Compound **14** sodium (((((1,3-dihydroxy-2-methylpropan-2-yl)azanediyl)bis(methylene))bis(4,1-phenylene))bis(ethane-2,1-diyl))bis(phosphonate), 337 mg, 74% yield, as a while solid: C₂₂H₂₉NNa₄O₈P₂•xH₂O, MS (ES+) m/z: 502.2 (M+H)⁺.

### Example 15: Synthesis of Compound 15 sodium (2-(((1,3-dihydroxy-2-(hydroxymethyl) propan-2-yl)amino)methyl)propane-1,3-diyl)bis(phosphonate)

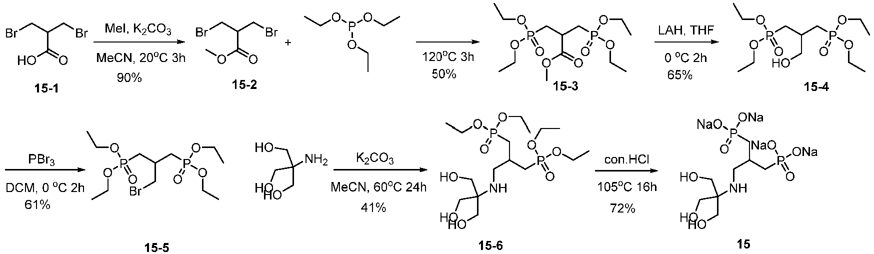

Step (1): To a 250 mL round flask, was added **15-1** 3-bromo-2-(bromomethyl)propanoic acid (12.3 g, 50 mmol), K₂CO₃ (6.9 g, 50 mmol), and then added dropwise Methane iodide (10.65 g, 75 mmol) at 20 degree. The mixture was stirred for 3 hrs, and filtrated, concentrated. The filter residue was washed with DCM (2 × 50 mL). The combined organic solution was dried over NaSO4 to give the desired crude product **15-2** methyl 3-bromo-2-(bromomethyl)propanoate, 11.7 g, 90% yield: C₅H₈Br₂O₂, MS (ES+) m/z: 281.0 (M+Na)⁺, which was directly used to next step.

Step (2): To a 100 mL flask, was added, compound **15-2** methyl 3-bromo-2-(bromomethyl) propanoate (11.0 g, 42.5 mmol), triethylphosphite (21.2 g, 127 mmol). The mixture was heated to 120 degree, and stirred for 3 hrs, and then cooled to r.t, and was concentrated to result a thick liquid which was purified by flash column chromatography to give compound **15-3** methyl 3-(diethoxyphosphoryl)-2-((diethoxyphosphoryl)methyl) propanoate, 7.96 g, with 50% yield as a viscous liquid: C₁₃H₂₈O₈P₂, MS (ES+) m/z: 375.1 (M+H)⁺.

Step (3): Compound **15-3** methyl 3-(diethoxyphosphoryl)-2-((diethoxyphosphoryl)methyl) propanoate (7.5 g, 20 mmol) was dissolved in 100 mL THF in a 500 mL three-neck flask. The solution was cooled to 0°C, and was added dropwise 1 M LiAIH4 (15.6 mL, 15.6 mmol). And the reaction was stirred over 2 hrs at 0 °C. Na₂SO₄•10H₂O was added to the cold solution to quench the reaction. And the resulted mixture was filtrate. The residue was washed with THF (2 × 100 mL). The organic solution was combined and dried over Na2SO4, and filtrated, and concentrated. The produced residue was purified by flash chromatography to give compound **15-4** tetraethyl (2-(hydroxymethyl)propane-1,3-diyl)bis(phosphonate), 4.5 g, with 65% yield as a sticky liquid: C₁₂H₂₈O₇P₂, MS (ES+) m/z: 347.1 (M+H)⁺.

Step (4): To a 250 mL flask, was added, compound **15-4** tetraethyl (2-(hydroxymethyl) propane-1,3-diyl)bis(phosphonate) (4.3 g, 12.4 mmol), and 0 mL DCM. The solution was cooled to 0°C, and was added PBr3 (5.04 g,18.6 mmol), and kept stirring for 3 hrs. The solution was poured into 50 g ice-water, and was neutralized by 5% NaHCO3. The mixture was extracted by DCM (3 × 80 mL). The organic phases were combined and dried over Na2SO4, filtered, and concentrated in low temperature to give a stick liquid which was washed with DCM and petroleum ether to give compound **15-5** tetraethyl (2-(bromomethyl)propane-1,3-diyl)bis(phosphonate), 3.1 g, with 61% yield: C₁₂H₂₇BrO₆P₂, MS (ES+) m/z: 409.0 (M+H)⁺.

Step (5): Refer to step (1) of Example 8, to obtain **15-6** tetraethyl (2-(((1,3-dihydroxy-2-(hydroxymethyl)propan-2-yl)amino)methyl)propane-1,3-diyl)bis(phosphonate), 276 mg, 41% yield, as a colorless liquid: C₁₆H₃₇NO₉P₂, MS (ES+) m/z: 450.2 (M+H)⁺.

Step (6): Refer to step (2) of Example 8, to obtain Compound **15** sodium (2-(((1,3-dihydroxy-2-(hydroxymethyl)propan-2-yl)amino)methyl)propane-1,3-diyl)bis(phosphonate), 220 mg, 72% yield, as a while solid: C₈H₁₇NNa₄O₉P₂ •xH₂O, MS (ES+) m/z: 338.1 (M+H)⁺.

### Example 16: Synthesis of Compound 16 sodium (2-(((1,3-dihydroxy-2-(hydroxymethyl) propan-2-yl)amino)methyl)-2-(phosphonatomethyl)propane-1,3-diyl)bis(phosphonate) (not claimed)

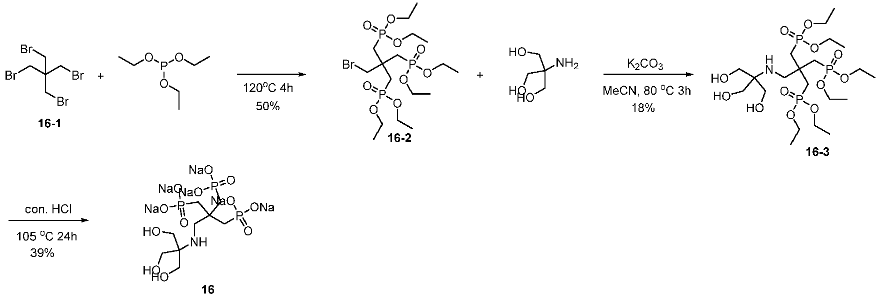

Step (1): To a 50 mL round flask, was added 1,3-dibromo-2,2-bis(bromomethyl)-propane (7.76 g, 20.0 mmol), triethyl phosphite (13.3 g, 40.2 mmol). The flask was heated up in an oil bath. The mixture was stirred for 3 hrs under 120°C. After removal of heating system, to the cool mixture was added 30 mL dichloromethane. The resulted mixture was kept stirring for 10 minutes, and was added 30 mL petroleum ether, and was stirred overnight. Then the suspension was filtered, the residue was washed with 30 mL solvent mixture of CH₂Cl₂ and petroleum ether (v/v, 1:5). The filtrate was collected and combined, concentrated to give a thick liquid which was purified by column chromatography to provide compound **16-2** tetraethyl (2-(bromomethyl)-2-((diethoxyphosphoryl)methyl) propane-1,3-diyl)bis(phosphonate) 5.6 g,50% yield: C₁₇H₃₈BrO₉P₃, MS (ES+) m/z: 559.1 (M+H)⁺.

Step (2): Refer to step (1) of Example 8, to obtain **16-3** tetraethyl (2-((diethoxyphosphoryl)methyl)-2-(((1,3-dihydroxy-2-(hydroxymethyl)propan-2-yl)amino) methyl)propane-1,3-diyl)bis(phosphonate), 520 mg, 18% yield, as a colorless liquid: C₂₁H₄₈NO₁₂P₃, MS (ES+) m/z: 600.2 (M+H)⁺.

Step (3): Refer to step (2) of Example 8, to obtain Compound **16** sodium (2-(((1,3-dihydroxy-2-(hydroxymethyl)propan-2-yl)amino)methyl)-2-(phosphonatomethyl)propane-1,3-diyl)bis(phosphonate), 210 mg, 39% yield, as a while solid: C₉H₁₈NNa₆O₁₂P₃•xH₂O, MS (ES+) m/z: 432.1 (M+H)⁺.

### Example 17: Synthesis of Compound 17 sodium ((5-(2-(2-((1,3-dihydroxy-2-(hydroxymethyl)propan-2-yl)amino)ethoxy)ethoxy)-1,3-phenylene)bis(ethane-2,1-diyl))bis(phosphonate)

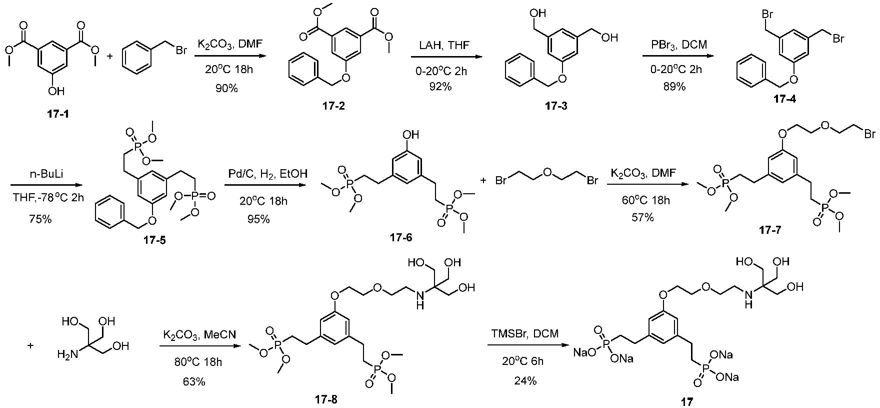

Step (1): To a 500 mL three-neck round flask, was added 17-1 (21.02g,100 mmol), 100 mL anhydrous DMF, and K₂CO₃ (20.7 g, 150 mmol), and benzyl bromide (25.7g, 150 mmol). The mixture was stirred at room temperature overnight, and was poured into 500 mL of ice water, and was extracted by EtOAc (2 × 200 mL). The combined organic solution was washed with 200 mL water, 200 mL brine, and was dried over Na2SO4. The suspension was filtrated. The liquid phase was concentrated to give crude product which was purified by flash chromatography to give 17-2 dimethyl 5-(benzyloxy)isophthalate, 27 g, 90% yield: C₁₇H₁₆O₅, MS (ES+) m/z: 323.1 (M+Na)⁺.

Step (2): To a 1000 mL three-neck round flask, was added, **17-2** dimethyl 5-(benzyloxy)isophthalate (27 g, 90 mmol), and 200 mL anhydrous THF. The mixture was cooled to 0°C, and was added dropwise 1 M LiALH4 (198 mL, 198 mmol). The suspension was stirred at 0-20 °C for 2 hrs, and was cooled to 0°C. To the mixture, 50g of aqueous Na2SO4 was carefully added in batches. The resulted mixture was filtrated. The filter residue was washed with 200 mL DCM. The combined organic solution was dried over Na2SO4, filtrated, concentrated to give the crude product **17-3** (5-(benzyloxy)-1,3-phenylene)dimethanol, 20.2 g, with 92% yield as a viscous liquid: C₁₅H₁₆O₃, MS (ES+) m/z: 267.1 (M+Na)⁺.

Step (3): To a 1000 mL three-neck round flask, was added **17-3** (5-(benzyloxy)-1,3-phenylene)dimethanol (20.2 g, 82.7 mmol) , 200 mL anhydrous DCM. The mixture was cooled to 0 degree, and was added slowly PBr3 (67.2 g, 248 mmol). The reaction mixture was stirred at 0-20 °C for 2 hrs, and was poured into 500g of ice water carefully, and was extracted with DCM (2 × 200 mL). the combined organic solution was washed with 200 brine, and dried over Na2SO4, filtrated, concentrated to give **17-4** 1-(benzyloxy)-3,5-bis(bromomethyl)benzene,27.5 g, with 89% yield as a pale yellow solid: C₁₅H₁₄Br₂O, MS (ES+) m/z: 391.0 (M+Na)⁺.

Step (4): To a 1000 mL three-neck flask, was added 400 mL of anhydrate THF, dimethyl-methylphosphate (23.06 g, 186 mmol). The flask was cooled down to -78°C via a dry ice-acetone bath, and was dropwise added 74.4 mL butyl lithium in hexane (186 mmol, 2.5M) in 30 minutes. After keep stirring for another hour at -78°C, **17-4** 1-(benzyloxy)-3,5-bis(bromomethyl)benzene (27.5 g, 74.3 mmol) in 100 mL of THF was added in 30 minutes. The mixture was kept stirring for 1hr. LCMS showed the major product is desired. The reaction mixture was quenched by monopotassium phosphate (1M, 100 mL), and was warmed up to room temperature. The mixture of liquid was separated by filtration funnel. The aqueous phase was washed by 50 mL of solvent mixture chloroform and isopropylol (3: 1) with four times. The organic phase was combined and added Na2SO4, filtered and concentrated. The residue was purified to give compound **17-5** tetramethyl ((5-(benzyloxy)-1,3-phenylene)bis(ethane-2,1-diyl))bis(phosphonate), 25.4 g, with 75% yield: C₂₁H₃₀O₇P₂, MS (ES+) m/z: 479.2 (M+Na)⁺.

Step (5): the mixture of **17-5** tetramethyl ((5-(benzyloxy)-1,3-phenylene)bis(ethane-2,1-diyl))bis(phosphonate) (25.4g, 55.7 mmol) in 250 mL ethanol, was added 1g of 10% pd/C, and degas, in a 1000 mL three-neck flask. Hydrogen balloon at 101325 Pa (1 atm) was installed. And the mixture was stirred over 18 hrs at r.t. The mixtures was filtered. And the mother liquid was concentrated to give desired **17-6** tetramethyl ((5-hydroxy-1,3-phenylene)bis(ethane-2,1-diyl))bis(phosphonate), 19.4 g, with 95% yield as a pale yellow solid: C₁₄H₂₄O₇P₂, MS (ES+) m/z: 389.1 (M+Na)⁺.

Step (6): To a 100 mL round flask, was added **17-6** tetramethyl ((5-hydroxy-1,3-phenylene)bis(ethane-2,1-diyl))bis(phosphonate) (1.83 g, 5.0 mmol), 1-bromo-2- (2-ethoxyl) ethane (2.32 g, 10.0 mmol), K₂CO₃ (1.38 g, 10.0 mmol), and 20 mL anhydrous DMF. The mixture was stirred for 18 hrs at 60 °C. Then the reaction mixture was poured into 120 mL of ice water, and was extracted with EtOAc (2 × 120 mL), The combined organic solvent was combined, and dried over Na2SO4, and filtrated. The solution was concentrated. And the residue was purified by flash chromatography to give **17-7** tetramethyl((5-(2-(2-bromoethoxy)ethoxy)-1,3-phenylene)bis(ethane-2,1-diyl))bis (phosphonate), 1.47 g, with 57% yield, as a viscous liquid: C₁₈H₃₁BrO₈P₂, MS (ES+) m/z: 517.1 (M+H)⁺.

Step (7): Refer to step (1) of Example 8, to obtain **17-8** tetramethyl ((5-(2-(2-((1,3-dihydroxy-2-(hydroxymethyl)propan-2-yl)amino)ethoxy)ethoxy)-1,3-phenylene)bis (ethane-2,1-diyl))bis(phosphonate), 914 mg, 63% yield, as a colorless liquid: C₂₂H₄₁NO₁₁P₂, MS (ES+) m/z: 558.2 (M+H)⁺.

Step (8): Refer to step (2) of Example 8, to obtain Compound **17** sodium ((5-(2-(2-((1,3-dihydroxy-2-(hydroxymethyl)propan-2-yl)amino)ethoxy)ethoxy)-1,3-phenylene)bis (ethane-2,1-diyl))bis(phosphonate), 236 mg, 24% yield, as a while solid: C₁₈H₂₉NNa₄O₁₁P₂•xH₂O, MS (ES+) m/z: 502.2 (M+H)⁺.

### Example 18: Synthesis of Compound 18 sodium ((5-(2-amino-3-hydroxy-2-(hydroxymethyl)propoxy)-1,3-phenylene)bis(ethane-2,1-diyl))bis(phosphonate)

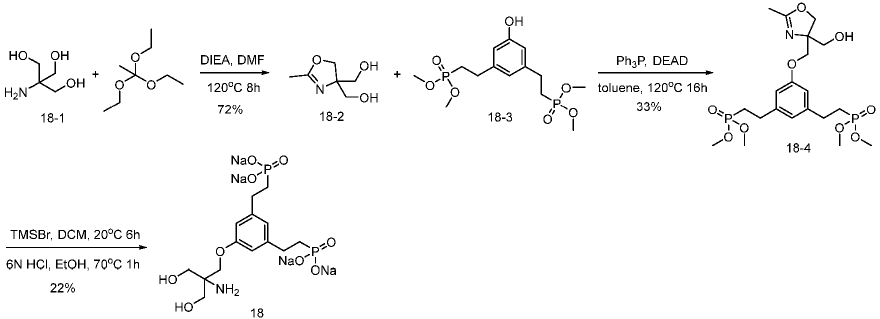

Step (1): To a 250 mL round flask, was added, tris amine (5.0 g, 41.3 mmol), 50 mL DMF,9.1 mL triethyl orthoacetate, and 7.2 mL DIPEA. The mixture was stirred 8 hrs at 120 °C. After the removal of solvent and addition of 100 mL petroleum ether, the solid was precipitated out. The solid was collected as product compound **18-2** tetramethyl ((5-hydroxy-1,3-phenylene)bis(ethane-2,1-diyl))bis(phosphonate), 4.31g, with 72% yield: C₆H₁₁NO₃, MS (ES+) m/z: 146.1 (M+H)⁺.

Step (2): To a 250 mL tree-neck round flask, was added, triphenyl phosphine (576 mg, 2.2 mmol), and 40 mL anhydrous toluene. The solution was cooled to 0 °C, and was slowly added DEAD (383 mg, 2.2 mmol). After stirring 30 min at 0 °C, compound **18-3** tetramethyl ((5-hydroxy-1,3-phenylene)bis(ethane-2,1-diyl))bis(phosphonate) (732 mg, 2.0 mmol) and 18-2 (436 mg, 3.0 mmol) was added. The resulted reaction mixture was refluxed for 16hrs, and concentrated. The residue was purified by flash chromatography to give Compound **18-4** tetramethyl ((5-((4-(hydroxymethyl)-2-methyl-4,5-dihydrooxazol-4-yl)methoxy)-1,3-phenylene)bis(ethane-2,1-diyl))bis(phosphonate), 325 mg, with 33% yield as a viscous liquid: C₂₀H₃₃NO₉P₂, MS (ES+) m/z: 494.2 (M+H)⁺.

Step (3): Compound **18-4** tetramethyl ((5-((4-(hydroxymethyl)-2-methyl-4,5-dihydrooxazol-4-yl)methoxy)-1,3-phenylene)bis(ethane-2,1-diyl))bis(phosphonate) (325 mg, 0.67 mmol) was dissolved in 10 mL DCM in a 50 mL round flask. The mixture was cooled to 0 °C, at N2 atmosphere, was dropwise added 3 mL TMS-Br. The reaction was gradually increased to 20 °C within 1 hr, and kept stirring for 6 hrs. The reaction solution was concentrated to result a viscous liquid. 2 mL anhydrous ethanol was added to the residue, and followed with 2 mL of 6 N HCl. And the mixture was heated to 70 °C, and was kept stirring for another 1 hr. The mixture was cooled down, and was concentrated to give a viscous liquid. To it was added 10 mL DCM and 5 mL methanol, and kept stirring for 30 min at 20 °C. The mixture was concentrated to dry again. And residue was purified by resin column chromatography to give compound **18** sodium ((5-(2-amino-3-hydroxy-2-(hydroxymethyl)propoxy)-1,3-phenylene)bis(ethane-2,1-diyl))bis(phosphonate) , 82 mg, with 22% yield: C₁₄H₂₁NNa₄O₉P₂•xH₂O, MS (ES+) m/z: 414.1 (M+H)⁺.

### Example 19: Synthesis of Compound 19 sodium ((5-(4-(((1,3-dihydroxy-2-(hydroxymethyl) propan-2-yl)amino)methyl)phenoxy)-1,3-phenylene)bis(ethane-2,1-diyl))bis(phosphonate)

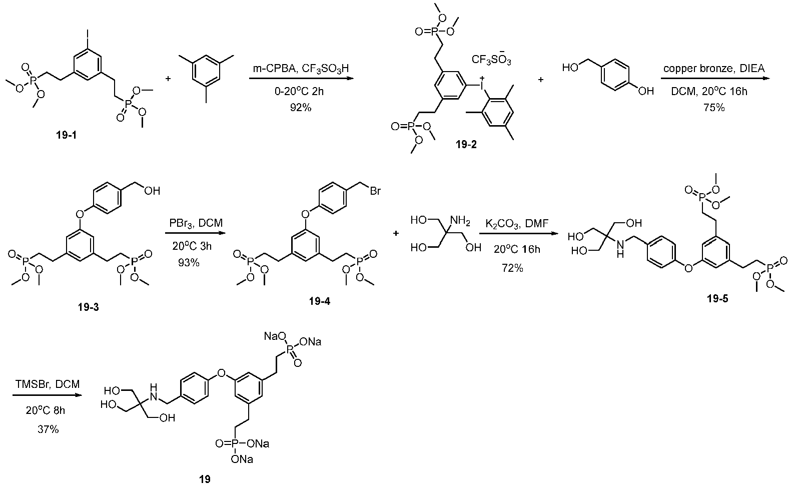

Step (1): To a 50 mL dry round flask was added, m-CPBA (759 mg, 4.4 mmol), and 40 mL anhydrous DCM. To the mixture, 19-1 tetramethyl ((5-iodo-1,3-phenylene)bis(ethane-2,1-diyl))bis(phosphonate) (1.90 g, 4.0 mmol), and mesitylene (5.29g, 4.4 mmol) were added. The mixture was cooled to 0 °C, and was slowly dropwise added trifloromethansulfonic acid (1.02 g, 6.8 mmol), and stirred for 2 hours at 20 °C. DCM was concentrated in lower temperature. And 100 mL ethyl ether was added to the residue, and the suspension was filtrated. The solid was collected as product **19-2** (3,5-bis(2-(dimethoxyphosphoryl)ethyl)phenyl)(mesityl)iodonium, 2.74 g, with 92% yield: C₂₄H₃₄F₃IO₉P₂S, MS (ES+) m/z: 595.1 (M+)⁺.

Step (2): To a dry 100 mL round flask, was added **19-2** (3,5-bis(2-(dimethoxyphosphoryl) ethyl)phenyl)(mesityl)iodonium (1.30 g, 1.75 mmol), 20 mL anhydrous DCM. To the mixture, a 10 mL solution of 4-hydroxymethyl-phenol (282 mg, 2.27 mmol, and DIPEA (677 mg, 5.25 mmol) in DCM was dropwise added at 20 °C. The reaction mixture was stirred for 16 hours. And after removal of DCM by rotation evaporation, the residue was purified by flash column chromatography to give **19-3** tetramethyl ((5-(4-(hydroxymethyl)phenoxy)-1,3-phenylene)bis(ethane-2,1-diyl))bis(phosphonate), 620 mg, with 75% yield: C₂₁H₃₀O₈P₂, MS (ES+) m/z: 473.1 (M+H)⁺.

Step (3): To a 50 mL three-neck round flask, was added, **19-3** tetramethyl ((5-(4-(hydroxymethyl)phenoxy)-1,3-phenylene)bis(ethane-2,1-diyl))bis(phosphonate) (620 mg, 1.31 mmol), and 10 mL anhydrous dichloromethane. Under ice-water bath, the PBr3 (710 mg, 2.62 mmol) was added at 0 °C. The mixture was stirred for 3 hrs at 0-20 °C, and then was poured into 50 g ice-water. The mixture was extracted with DCM (3 × 50 mL). The combined organic phase was washed with 100 mL brine. The organic layer was dried over Na2SO4, and filtrated, and concentrated. The residue was **19-4** tetramethyl ((5-(4-(bromomethyl)phenoxy)-1,3-phenylene)bis(ethane-2,1-diyl))bis(phosphonate), 652 mg, 93% yield: C₂₁H₂₉BrO₇P₂, MS (ES+) m/z: 535.1 (M+H)⁺.

Step (4): Refer to step (1) of Example 8, to obtain **19-5** tetramethyl ((5-(4-(((1,3-dihydroxy-2-(hydroxymethyl)propan-2-yl)amino)methyl)phenoxy)-1,3-phenylene)bis(ethane-2,1-diyl))bis(phosphonate), 506 mg, 72% yield, as a colorless liquid: C₂₅H₃₉NO₁₀P₂, MS (ES+) m/z: 576.2 (M+H)⁺.

Step (5): Refer to step (2) of Example 8, to obtain Compound **19** sodium ((5-(4-(((1,3-dihydroxy-2-(hydroxymethyl)propan-2-yl)amino)methyl)phenoxy)-1,3-phenylene)bis(ethane-2,1-diyl))bis(phosphonate), 215 mg, 37% yield, as a while solid: C₂₁H₂₇NNa₄O₁₀P₂•xH₂O, MS (ES+) m/z: 520.1 (M+H)⁺.

### Example 20: Synthesis of Compound 20 sodium ((4'-(2-amino-3-hydroxy-2-(hydroxymethyl)propoxy)-[1,1'-biphenyl]-3,5-diyl)bis(ethane-2,1-diyl))bis(phosphonate)

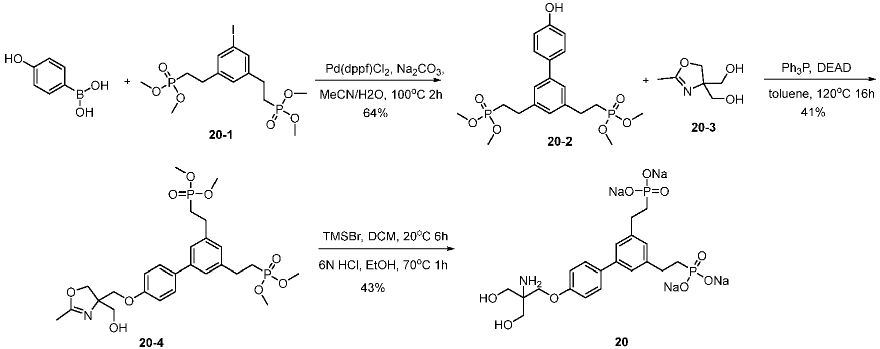

Step (1): To a 50 mL round flask, was added, **20-1** tetramethyl ((5-iodo-1,3-phenylene)bis(ethane-2,1-diyl))bis(phosphonate) (954 mg, 2.0 mmol), Pd(dppf)Cl2 (146 mg, 0.2 mmol), Na2CO3 (424 mg, 4.0 mmol), and 10 mL acetonitrile and 1 mL water. The mixtures was degas with argon, and was stirred for 5 min at r.t, and then was added DIPEA (413 mg, 3.2 mmol), and 4-hydroxy-phenylboronic acid (404 mg, 3.2 mmol). The mixture was stirred for 8 hrs at 100 °C. After the removal of solvent, the residue was purified by chromatography to give **20-2** tetramethyl ((4'-hydroxy-[1,1'-biphenyl]-3,5-diyl)bis(ethane-2,1-diyl))bis(phosphonate), 567 mg, with 64% yield: C₂₀H₂₈O₇P₂S, MS (ES+) m/z: 443.1 (M+H)⁺.

Step (2): To a 250 mL tree-neck round flask, was added, triphenyl phosphine (369 mg, 1.41 mmol), and 30 mL anhydrous toluene. The solution was cooled to 0 °C, and was slowly added DEAD (245 mg, 1.41 mmol). After stirring 30 min at 0 °C, **20-2** tetramethyl ((4'-hydroxy-[1,1'-biphenyl]-3,5-diyl)bis(ethane-2,1-diyl))bis(phosphonate) (567 mg, 1.28 mmol) and **20-3** (2-methyl-4,5-dihydrooxazole-4,4-diyl)dimethanol (279 mg, 1.92 mmol) was added. The resulted reaction mixture was refluxed for 16hrs, and concentrated. The residue was purified by flash chromatography to give **20-4** tetramethyl ((4'-((4-(hydroxymethyl)-2-methyl-4,5-dihydrooxazol-4-yl)methoxy)-[1,1'-biphenyl]-3,5-diyl)bis(ethane-2,1-diyl))bis(phosphonate), 299 mg, with 41% yield as a viscous liquid: C₂₆H₃₇NO₉P₂, MS (ES+) m/z: 570.2 (M+H)⁺.

Step (3): **20-4** tetramethyl ((4'-((4-(hydroxymethyl)-2-methyl-4,5-dihydrooxazol-4-yl)methoxy)-[1,1'-biphenyl]-3,5-diyl)bis(ethane-2,1-diyl))bis(phosphonate) (299 mg, 0.525 mmol) was dissolved in 10 mL DCM in a 50 mL round flask. The mixture was cooled to 0 °C, at N2 atmosphere, was dropwise added 3 mL TMS-Br. The reaction was gradually increased to 20 °C within 1 hr, and kept stirring for 6 hrs. The reaction solution was concentrated to result a viscous liquid.2 mL anhydrous ethanol was added to the residue, and followed with 2 mL of 6 N HCl. And the mixture was heated to 70 °C, and was kept stirring for another 1 hr. The mixture was cooled down, and was concentrated to give a viscous liquid. To it was added 10 mL DCM and 5 mL methanol, and kept stirring for 30 min at 20 °C. The mixture was concentrated to dry again. And residue was purified by resin column chromatography to give compound **20** sodium ((4'-(2-amino-3-hydroxy-2-(hydroxymethyl)propoxy)-[1,1'-biphenyl]-3,5-diyl)bis(ethane-2,1-diyl))bis(phosphonate), 142 mg, with 43% yield: C₂₀H₂₅NNa₄O₉P₂ •xH₂O, MS (ES+) m/z: 490.1 (M+H)⁺.

### Example 21: Synthesis of Compound 21 sodium ((5-(2,6-bis(2-(diethoxyphosphoryl)ethyl) -4-(((1,3-dihydroxy-2-(hydroxymethyl)propan-2-yl)amino)methyl)phenoxy)-1,3-phenylene)bis(ethane-2,1-diyl))bis(phosphonate)

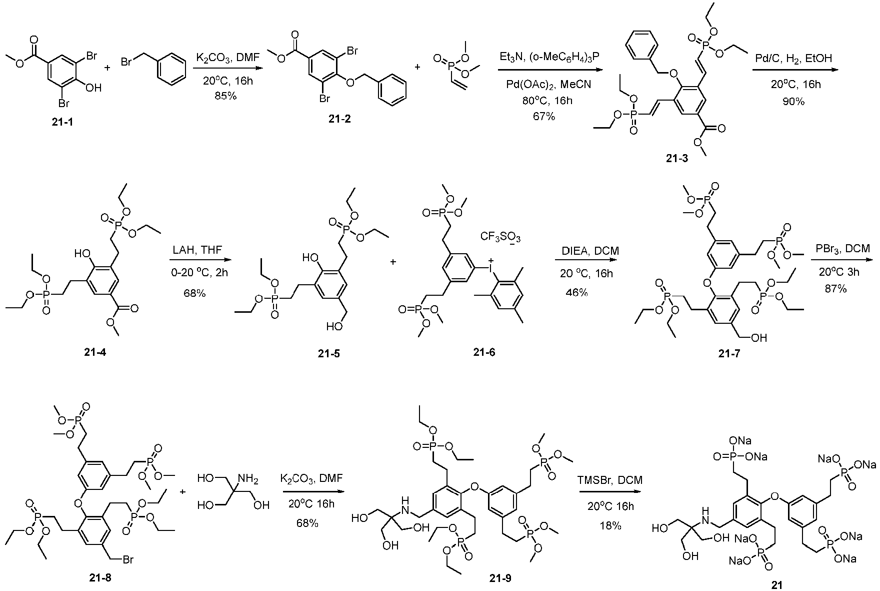

Step (1): To a 100 mL round flask, was added, **21-1** methyl 3,5-dibromo-4-hydroxybenzoate (6.2 g, 20 mmol), K₂CO₃ (4.14 g,30 mmol), 30 mL anhydrous DMF. The reaction mixture was stirred for 16 hrs at 20 °C, and then was poured into 200 mL ice-water. The mixture was extracted with EtOAc (2 × 100 mL). The combined organic phases were washed with 200 mL water, 200 mL brine, and was dried over Na2SO4, and was filtered. After the removal of solvent by rotation evaporation, the residue was purified by flash column chromatography to give **21-2** methyl 4-(benzyloxy)-3,5-dibromobenzoate, 6.78 g, with 85% yield: C15H12Br2O3, MS (ES+) m/z: 421.0 (M+Na)⁺.

Step (2): To a 250 mL round flask, was added, **21-2** methyl 4-(benzyloxy)-3,5-dibromobenzoate (6.5 g, 16.3 mmol), diethyl vinyl-phosphate (8.03 g, 48.9 mmol), TEA (6.59 g, 65.2 mmol), tris-(o-methyl phenyl)-phosphine (496 mg, 1.63 mmol), Pd (OAc)2 (183 mg, 0.82 mmol), and 100 mL acetonitrile. The mixture was stirred for 16 hrs under N2 atmosphere. The mixture was filtrated, and concentrated to produce a residue which was purified by flash column chromatography to give **21-3** methyl 4-(benzyloxy)-3,5-bis((E)-2-(diethoxyphosphoryl)vinyl)benzoate, 6.18 g, with 67% yield as a viscous liquid: C₂₇H₃₆O₉P₂, MS (ES+) m/z: 566.2 (M+H)⁺.

Step (3): (3): Compound **21-3** methyl 4-(benzyloxy)-3,5-bis((E)-2-(diethoxyphosphoryl) vinyl)benzoate (3.0 g, 5.3 mmol) was dissolved in 30 mL ethanol, and was hydrogenated with 10% Pd/C for 16 hrs. An off-red solid was obtained as **21-4** methyl 3,5-bis(2-(diethoxyphosphoryl)ethyl)-4-hydroxybenzoate, 2.29 g, with 90% yield: C₂₀H₃₄O₉P₂, MS (ES+) m/z: 481.2 (M+H)⁺.

Step (4): Compound **21-4** methyl 3,5-bis(2-(diethoxyphosphoryl)ethyl)-4-hydroxybenzoate (2.7 g, 4.37 mmol) was dissolved in 30 mL THF in a 100 mL three-neck flask. The solution was cooled to 0°C, and was added dropwise 1 M LiAIH4 (8.7 mL, 8.7 mmol). And the reaction was stirred over 2 hrs at 0 °C. 5 g Na₂SO₄•10H₂O was added to the cold solution to quench the reaction. And the resulted mixture was filtrate. The residue was washed with FTE (2 × 100 mL). The organic solution was combined and dried over Na2SO4, and filtrated, and concentrated. The produced residue was purified by flash chromatography to give compound **21-5** tetraethyl ((2-hydroxy-5-(hydroxymethyl)-1,3-phenylene)bis(ethane-2,1-diyl))bis(phosphonate), 1.34 g, with 68% yield as a pale red solid: C₁₉H₃₄O₈P₂, MS (ES+) m/z: 453.2 (M+H)⁺.

Step (5): To a dry 100 mL three-neck round flask, was added **21-6** (3,5-bis(2-(dimethoxyphosphoryl)ethyl)phenyl)(mesityl)iodonium (1.64 g, 2.21 mmol), 20 mL anhydrous DCM. To the mixture, a 10 mL solution of **21-5** tetraethyl ((2-hydroxy-5-(hydroxymethyl)-1,3-phenylene)bis(ethane-2,1-diyl))bis(phosphonate) (1.20 g, 2.65 mmol), and DIPEA (685 mg, 5.3 mmol) in DCM was dropwise added at 20 °C. The reaction mixture was stirred for 16 hours. And after removal of DCM by rotation evaporation, the residue was purified by flash column chromatography to give **21-7** tetramethyl ((5-(2,6-bis(2-(diethoxyphosphoryl)ethyl)-4-(hydroxymethyl)phenoxy)-1,3-phenylene)bis(ethane-2,1-diyl))bis(phosphonate), 815 mg, with 46% yield: C₃₃H₅₆O₁₄P₄, MS (ES+) m/z: 801.3 (M+H)⁺.

Step (6): To a 50 mL three-neck round flask, was added, **21-7** tetramethyl ((5-(2,6-bis(2-(diethoxyphosphoryl)ethyl)-4-(hydroxymethyl)phenoxy)-1,3-phenylene)bis(ethane-2,1-diyl))bis(phosphonate) (815 mg, 1.02 mmol), and 10 mL anhydrous dichloromethane. Under ice-water bath, the PBr3 (551 mg, 2.03 mmol) was added at 0 °C. The mixture was stirred for 3 hrs at 0-20 °C, and then was poured into 50 g ice-water. The mixture was extracted with DCM (3 × 50 mL). The combined organic phase was washed with 100 mL brine. The organic layer was dried over Na₂SO₄, and filtrated, and concentrated. The viscous residue was identified as **21-8** tetramethyl ((5-(4-(bromomethyl)-2,6-bis(2-(diethoxyphosphoryl)ethyl)phenoxy)-1,3-phenylene)bis(ethane-2,1 - diyl))bis(phosphonate), 764 mg, 87% yield: C₃₃H₅₅BrO₁₃P₄, MS (ES+) m/z: 863.2 (M+H)⁺.

Step (7): Refer to step (1) of Example 8, to obtain **21-9** tetramethyl ((5-(2,6-bis(2-(diethoxyphosphoryl)ethyl)-4-(((1,3-dihydroxy-2-(hydroxymethyl)propan-2-yl)amino)methyl)phenoxy)-1,3-phenylene)bis(ethane-2,1-diyl))bis(phosphonate), 543 mg, 68% yield, as a colorless liquid: C₃₇H₆₅NO₁₆P₄, MS (ES+) m/z: 904.3 (M+H)⁺.

Step (8): Refer to step (2) of Example 8, to obtain Compound **21** sodium ((5-(2,6-bis(2-(diethoxyphosphoryl)ethyl)-4-(((1,3-dihydroxy-2-(hydroxymethyl)propan-2-yl)amino)methyl)phenoxy)-1,3-phenylene)bis(ethane-2,1-diyl))bis(phosphonate), 97 mg, 18% yield, as a while solid: C₂₅H₃₃NNa₈O₁₆P₄•xH₂O, MS (ES+) m/z: 736.1 (M+H)⁺.

### Example 22: Synthesis of Compound 22 sodium ((5-(3-(((1,3-dihydroxy-2-(hydroxymethyl) propan-2-yl)amino)methyl)-5-(2-phosphonatoethyl)benzyl)-1,3-phenylene)bis(ethane-2,1-diyl))bis(phosphonate)

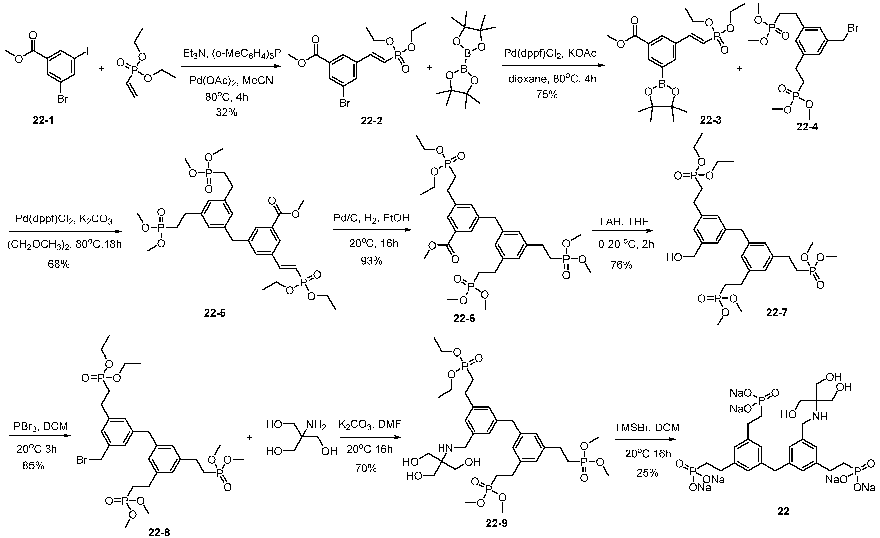

Step (1): To a 250 mL round flask, was added, 22-1 (6.82 g, 20.0 mmol), diethyl vinyl-phosphate (3.28 g, 20.0 mmol), TEA (4.04 g, 40 mmol), tris-(o-methyl phenyl)-phosphine (608 mg, 2.0 mmol), Pd (OAc)2 (223 mg, 1.0 mmol), and 100 mL acetonitrile. The mixture was stirred for 16 hrs under N2 atmosphere. The mixture was filtrated, and concentrated to produce a residue which was purified by flash column chromatography to give **22-2** methyl (E)-3-bromo-5-(2-(diethoxyphosphoryl)vinyl)benzoate, 2.41 g, with 32% yield as a viscous liquid: C₁₄H₁₈BrO₅P, MS (ES+) m/z: 399.0 (M+Na)⁺.

Step (2): To a 100 mL round flask, was added **22-2** methyl (E)-3-bromo-5-(2-(diethoxyphosphoryl)vinyl)benzoate (2.2 g, 5.83 mmol), 4,4,4',4',5,5,5',5'-octamethyl-2,2'-bi(1,3,2-dioxaborolane) (2.22 g, 8.75 mmol), KOAc (1.72 g, 17.5 mmol), Pd(dppf)Cl2 (213 mg, 0.29 mmol), and 40 mL 1,4-dioxane. The mixture was stirred for 6 hrs at 85 °C under nitrogen, and then was filtrated, concentrated to give a residue. The residue was purified by flash column chromatography to give **22-3** methyl (E)-3-(2-(diethoxyphosphoryl)vinyl)-5-(4,4,5,5-tetramethyl-1,3,2-dioxaborolan-2-yl)benzoate, 1.86 g, with 75% yield: C₂₀H₃₀BO₇P, MS (ES+) m/z: 447.2 (M+Na)⁺.

Step (3): To a 100 mL round flask, was added **22-3** methyl (E)-3-(2-(diethoxyphosphoryl)vinyl)-5-(4,4,5,5-tetramethyl-1,3,2-dioxaborolan-2-yl)benzoate (1.7 g, 4.01 mmol), **22-4** tetramethyl ((5-(bromomethyl)-1,3-phenylene)bis(ethane-2,1-diyl))bis(phosphonate) (1.95 g, 4.4 mmpl), K₂CO₃ (1.11 g, 8.02 mmol), Pd(dppf)Cl2 (293 mg, 0.40 mmol), and 30 mL glycol dimethyl ether. The reaction mixture was stirred for 18 hrs at 80 °C under nitrogen atmosphere. And the mixture was filtrated, and concentrated. The resulted residue was purified by flash column chromatography to give **22-5** methyl (E)-3-(3,5-bis(2-(dimethoxyphosphoryl)ethyl)benzyl)-5-(2-(diethoxyphosphoryl)vinyl)benzoate, 1,8 g, with 68% yield: C₂₉H₄₃O₁₁P₃, MS (ES+) m/z: 683.2 (M+Na)⁺.

Step (4): To a 100 mL round flask, was added **22-5** methyl (E)-3-(3,5-bis(2-(dimethoxyphosphoryl)ethyl)benzyl)-5-(2-(diethoxyphosphoryl)vinyl)benzoate (1.8 g, 2.72 mmol), 180 mg 10% Pd/C, and 30 mL ethanol. The reaction mixture was stirred for 16 hrs at 20 °C under nitrogen atmosphere. And the mixture was filtrated, and concentrated. The resulted residue was desired compound **22-6** methyl 3-(3,5-bis(2-(dimethoxyphosphoryl)ethyl)benzyl)-5-(2-(diethoxyphosphoryl)ethyl)benzoate, 1.67 g, with 93% yield: C₂₉H₄₃O₁₁P₃, MS (ES+) m/z: 685.2 (M+H)⁺.

Step (5): Compound **22-6** methyl 3-(3,5-bis(2-(dimethoxyphosphoryl)ethyl)benzyl)-5-(2-(diethoxyphosphoryl)ethyl)benzoate (1.67 g, 2.53 mmol) was dissolved in 30 mL THF in a 100 mL three-neck flask. The solution was cooled to 0°C, and was added dropwise 1 M LiAIH4 (5.1 mL, 5.1 mmol). And the reaction was stirred over 2 hrs at 0 °C. 5 g of Na₂SO₄•10H₂O was added to the cold solution to quench the reaction. And the resulted mixture was filtrate. The residue was washed with DCM (2 × 50 mL). The organic solution was combined and dried over Na2SO4, and filtrated, and concentrated. The produced residue was purified by flash chromatography to give compound **22-7** tetramethyl ((5-(3-(2-(diethoxyphosphoryl)ethyl)-5-(hydroxymethyl)benzyl)-1,3-phenylene)bis(ethane-2,1-diyl))bis(phosphonate), 1.22 g, with 76% yield as a sticky liquid: C₂₈H₄₅O₁₀P₃, MS (ES+) m/z: 635.2 (M+H)⁺.

Step (6): To a 50 mL three-neck round flask, was added, **22-7** tetramethyl ((5-(3-(2-(diethoxyphosphoryl)ethyl)-5-(hydroxymethyl)benzyl)-1,3-phenylene)bis(ethane-2,1-diyl))bis(phosphonate) (1.22 g, 1.92 mmol), and 10 mL anhydrous dichloromethane. Under ice-water bath, the PBr3 (1.04 g, 3.84 mmol) was added at 0 °C. The mixture was stirred for 3 hrs at 0-20 °C, and then was poured into 50 g ice-water. The mixture was extracted with DCM (3 × 50 mL). The combined organic phase was washed with 100 mL brine. The organic layer was dried over Na2SO4, and filtrated, and concentrated. The viscous residue was identified as **22-8** tetramethyl ((5-(3-(bromomethyl)-5-(2-(diethoxyphosphoryl)ethyl)benzyl)-1,3-phenylene)bis(ethane-2,1 -diyl))bis(phosphonate), 1.14 g, 85% yield: C₂₈H₄₄BrO₉P₃, MS (ES+) m/z: 697.2 (M+H)⁺.

Step (7): Refer to step (1) of Example 8, to obtain **22-9** tetramethyl ((5-(3-(2-(diethoxyphosphoryl)ethyl)-5-(((1,3-dihydroxy-2-(hydroxymethyl)propan-2-yl)amino)methyl)benzyl)-1,3-phenylene)bis(ethane-2,1-diyl))bis(phosphonate), 842 mg, 70% yield, as a colorless liquid: C₃₂H₅₄NO₁₂P₃, MS (ES+) m/z: 738.3 (M+H)⁺.

Step (8): Refer to step (2) of Example 8, to obtain Compound **22** sodium ((5-(3-(((1,3-dihydroxy-2-(hydroxymethyl)propan-2-yl)amino)methyl)-5-(2-phosphonatoethyl)benzyl)-1,3-phenylene)bis(ethane-2,1-diyl))bis(phosphonate), 221 mg, 25% yield, as a while solid: C₂₄H₃₂NNa₆O₁₂P₃•xH₂O, MS (ES+) m/z: 626.2 (M+H)⁺.

### Example 23 Pharmacodynamic Test

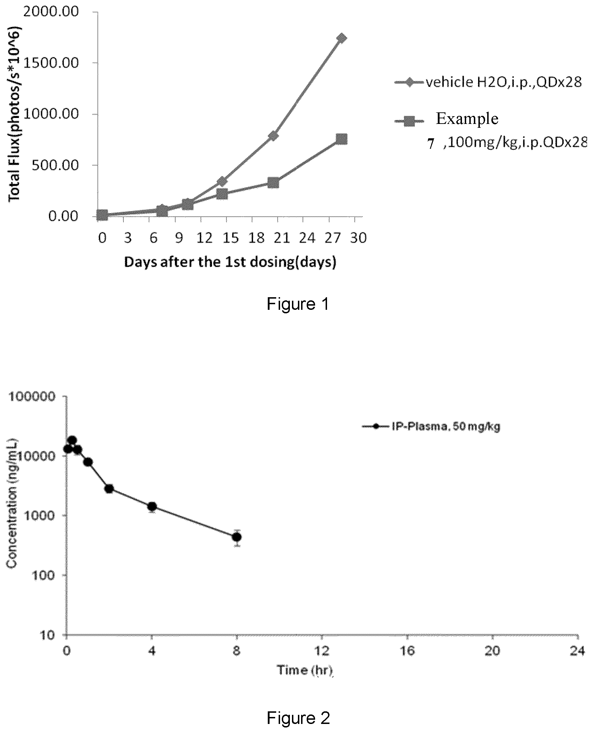

The compounds in present invention are used to test the antitumor activity in Hep3B2.1-7-Luc orthotopic xenograft tumor model
1. Cell culture: Hep3B2.1-7-Luc cells were cultured in vitro under EMEM medium with 1.5 g/L sodium bicarbonate, 1.0 mM sodium pyruvate, 2 ug/ml puromycin, 10% heat-inactivated fetal bovine serum, 100 U/ mL penicillin and 100 µ g/ mL streptomycin at 37 °C and 5% CO₂. Pass it through twice a week. When cells are growing exponentially, they are collected, counted, and inoculated.
2. Animal: BALB/c nude mice, female, 6-8 weeks old, body weight 18-22 g. Tumor inoculation: A 25 uL cell suspension of 1.25×10^6 Hep3B2.1-7-luc cells was orthotopically transplanted to left hepatic lobule of each mouse. The wound was sutured with stylolite. After 7 days of transportation, the mice were imaged with small animal imager IVIS Lumina XR, the mice with suitable signal intensity were selected to the pharmacological experiment, and each group included 10 animals.
3. Operation procedure of animal imaging
   1) Weigh a suitable amount of luciferin, prepare a concentration of 15 mg/ mL with DPBS, use 0.2 um filter membrane to filtration sterilization, store in dark place at -20 °C.
   2) Use a syringe of 25 × 5/8", 10 ul/g by mouse weight, each mouse to inject 150 mg luciferin/kg, i.e., 0.2 mL was injected to a 20 g mouse.
   3) After 10-12 minutes, anesthesia with isoflurane.
   4) Place the mouse to the imaging system box, abdomen upwards, to detect the tumor cells.
   5) The time horizon of time of exposure is one second to one minute. The imaging results is processed by image software, show as number of photons/second, imaging once a week, the last imaging to be done right before the experiment is finished.
4. Experiment Index: experiment index is to investigate if the tumor growth is inhibited, delayed or cured. Once a week via IVIS Lumina XR to image the mouse, to monitor the status of tumor growth. The tumor signal is counted on the number of exposure protons/second.
5. Data analysis: take the compound 7 of Example 7 for example, the result show as Figure 1. Compound 7 of Example 7 possessed obvious inhibition of tumor proliferation.

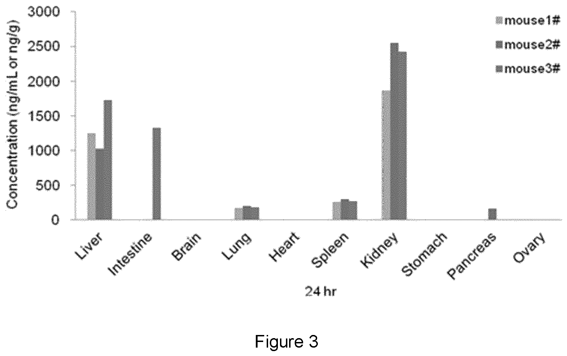

The pharmacokinetics of Compound 7 of Example 7 show as Figure 2 and 3, and below Table 1.

**Table 1 BALB/c nude mice ip -Blood Concentration and parameter**

| PK parameters | Unit | Mouse 1# | Mouse 2# | Mouse 3# | Mean (ng/ mL) | SD | CV(%) |
|---|---|---|---|---|---|---|---|
| Tmax | hr | 0.250 | 0.250 | 0.250 | 0.250 | 0.00 | 0.00 |
| Cmax | ng/ mL | 17600 | 19900 | 17900 | 18467 | 1250 | 6.77 |
| t1/2 | hr | 1.73 | 2.40 | 2.77 | 2.30 | 0.525 | 22.8 |
| AUCₗₐₛₜ | hr*ng/ mL | 26280 | 26280 | 25270 | 25943 | 583 | 2.25 |
| AUC_{INF} | hr*ng/ mL | 27030 | 27956 | 27469 | 27485 | 463 | 1.68 |

From Figure 3, BALB/c nude mice was treated with Compound 7 of Example 7 via i.p., after 24 hours, the drug compound is mainly located in liver, kidney, lung, intestine, spleen, prostate, and pancreatic. It hinted the compounds of present invention are particularly suitable to treating liver cancer, kidney cancer, prostate cancer, lung cancer, colon cancer, and pancreatic cancer.

## Claims

1. . The compound shown in a formula (II), or a tautomer, mesomer, racemate, enantiomer, and diastereoisomer thereof, or a mixture form thereof, or a pharmaceutically acceptable salt thereof, 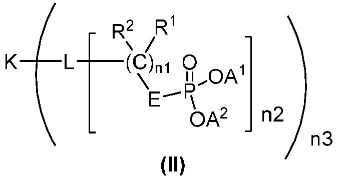
Wherein, L is selected from a group consisting of C1-C10 alkyl, cycloalkyl, cycloalkylalkyl, alkylcycloalkyl, aryl, arylalkyl, alkylaryl, heteroaryl, heteroarylalkyl, alkylheteroaryl, alkenyl, alkynyl, a C3-C15 linear or branched chain containing an O, or S atom, a linear or branched chain consisting of repeating units of C1-C15 linear or branched chains containing O, or S atoms, series of bisaryl, series of bisheteroaryl, series of aryl and heteroaryl, and bisaryl and bisheteroaryl linked by O, S or 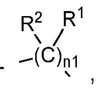 wherein the alkyl, the cycloalkyl, the heterocyclyl, the aryl or the heteroaryl may be each independently substituted by one or more substituents selected from a group consisting of hydroxyl, halogen, alkyl, alkoxyl, cycloalkyl, heterocyclyl, aryl, and heteroaryl;
R¹ and R² are each independently selected from a group consisting of hydrogen, halogen, alkyl, cycloalkyl, hydroxyalkane, alkoxylalkyl, alkoxylcycloalkyl, cycloalkylalkyl, alkylcycloalkyl, alkenyl, alkynyl, amino, hydroxyl, mercapto, carboxyl, alkoxyl, cycloalkoxyl, haloalkyl, cyano, thioalkyl, sulfo, sulfonyl, sulfinyl, phosphate, alkylphosphonate, arylphosphate, and arylphosphonate, wherein the alkyl, the cycloalkyl, the heterocyclyl, the aryl, or the heteroaryl may be each independently substituted by one or more substituents selected from a group consisting of hydroxyl, halogen, alkyl, alkoxyl, cycloalkyl, heterocyclyl, aryl, and heteroaryl;
K is selected from a group consisting of 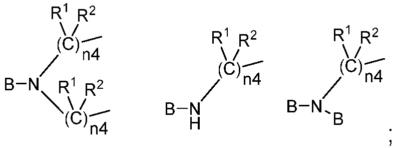
B is selected from a group consisting of 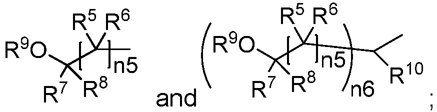
A¹ and A² are each independently selected from a group consisting of H, Li, Na, K, Cs, and a corresponding positive ion thereof, or A¹ and A² form Ca, Mg, Al, Sc, Ti, Cr, Co, Fe, Ni, Cu, Zn, Cd, Hg, and a corresponding positive ion thereof, collectively;
E is selected from a group consisting of C(R¹R²);
R⁵ and R⁶ are each independently selected from a group consisting of a hydrogen atom, halogen, alkyl, alkoxyalkyl, cycloalkyl, alkoxycycloalkyl, heterocyclyl, aryl and heteroaryl, wherein the alkyl, the cycloalkyl, the heterocyclyl, the aryl or the heteroaryl may be each independently substituted by one or more substituents selected from a group consisting of hydroxyl, halogen, alkyl, alkoxyl, cycloalkyl, heterocyclyl, aryl, and heteroaryl; or R⁵ and R⁶ may form a 3-membered to 8-membered ring, which may contain 1 to 2 heteroatoms of O, N and/or S;
R⁷ and R⁸ are each independently selected from a group consisting of a hydrogen atom, alkyl, hydroxyalkyl, cycloalkyl, alkoxyalkyl, alkoxycycloalkyl, heterocyclyl, aryl and heteroaryl, wherein the alkyl, the cycloalkyl, the heterocyclyl, the aryl or the heteroaryl may be each independently substituted by one or more substituents selected from a group consisting of hydroxyl, halogen, alkyl, alkoxy, cycloalkyl, heterocyclyl, aryl and heteroaryl;
R⁹ is a hydrogen atom;
R¹⁰ is selected from a group consisting of a hydrogen atom, halogen, alkyl, cycloalkyl, heterocyclyl, aryl, and heteroaryl, or is null, wherein the alkyl, the cycloalkyl, the heterocyclyl, the aryl, or the heteroaryl may be each independently substituted by one or more substituents selected from a group consisting of hydroxyl, halogen, alkyl, alkoxy, cycloalkyl, heterocyclyl, aryl, and heteroaryl;
n1 is selected from a group consisting of 0, 1, 2, 3, 4, 5, 6, 7, and 8;
n2 is selected from a group consisting of 1 and 2;
n3 is selected from a group consisting of 1 and 2;
n4 is selected from a group consisting of 0, 1, 2, 3, and 4;
n5 is selected from a group consisting of 0, 1, 2, and 3; and
n6 is selected from a group consisting of 1, 2, and 3.

2. . The compound according to claim 1, wherein the compound with the formula (II) comprises the following:

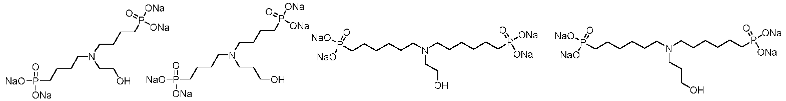

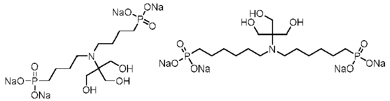

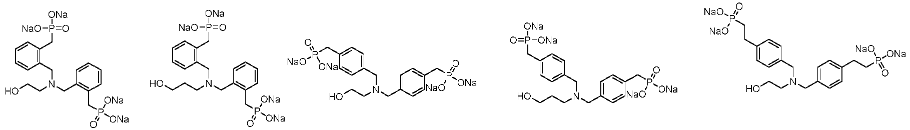

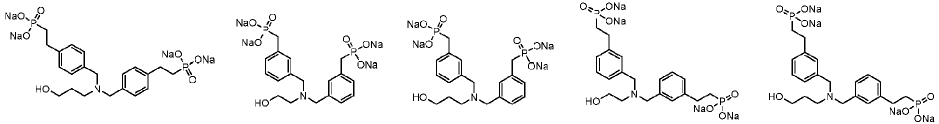

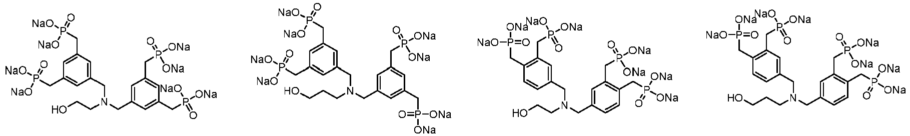

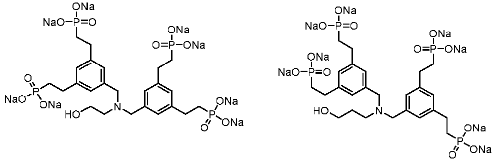

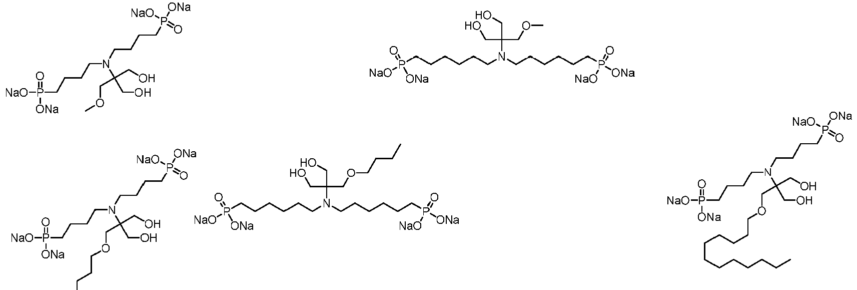

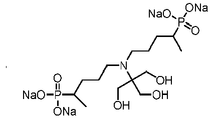

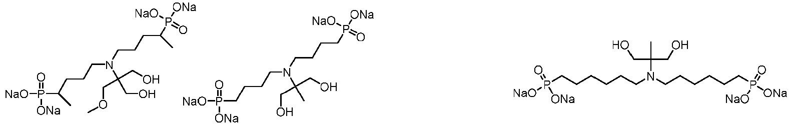

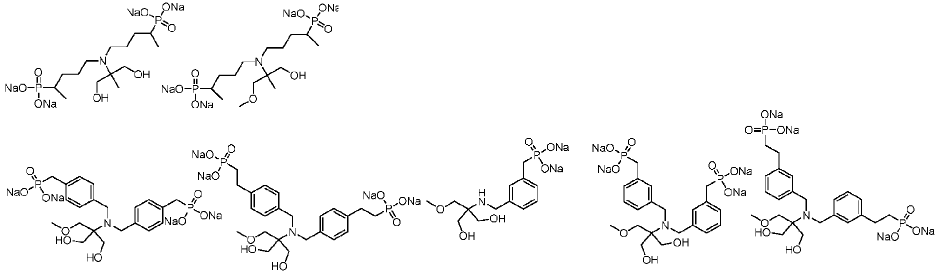

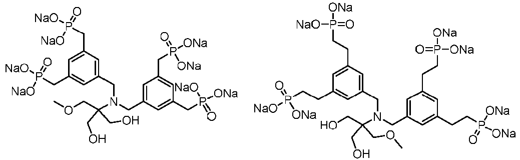

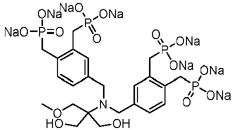

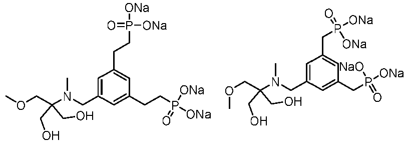

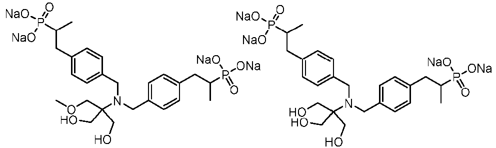

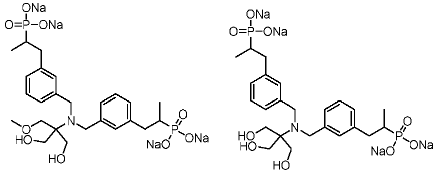

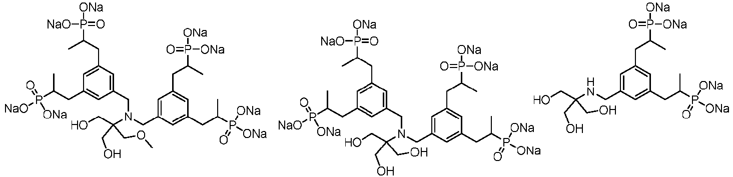

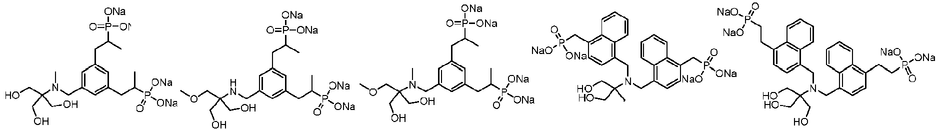

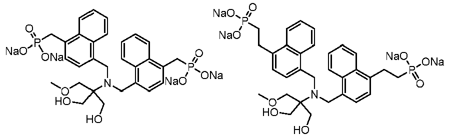

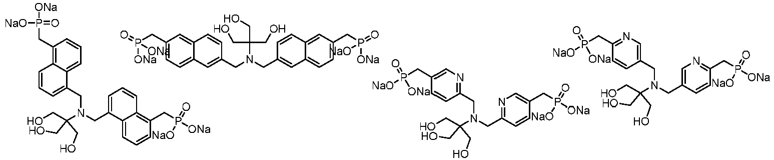

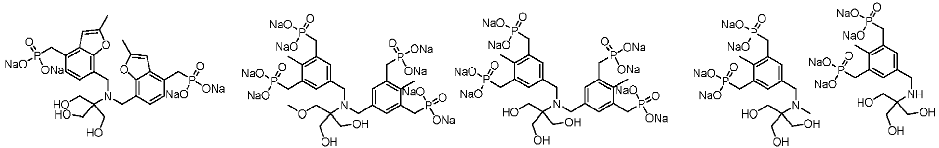

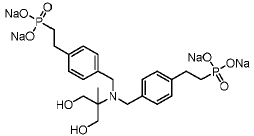

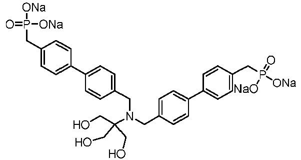

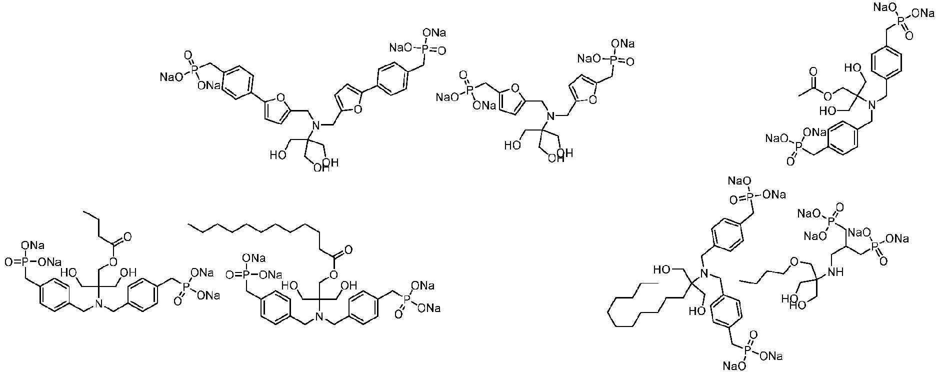

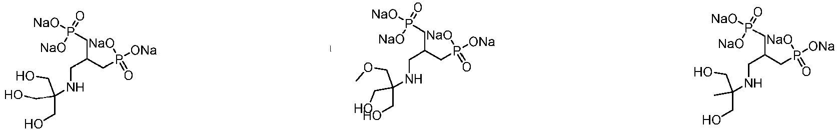

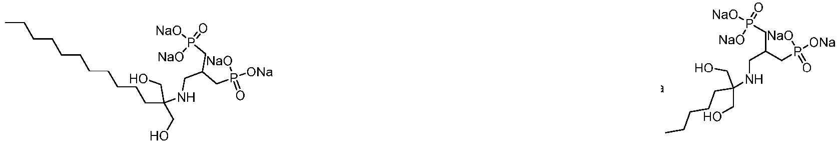

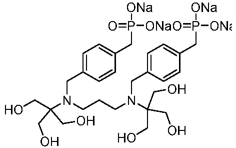

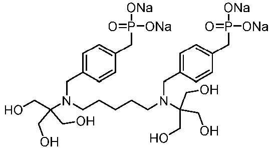

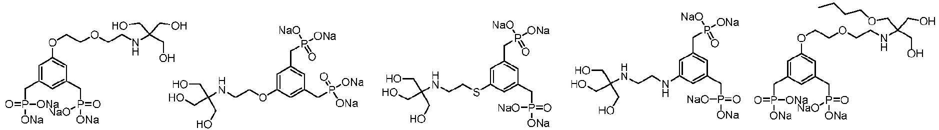

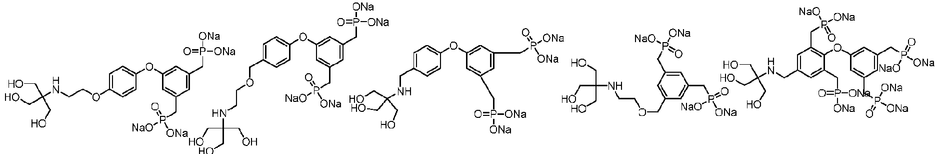

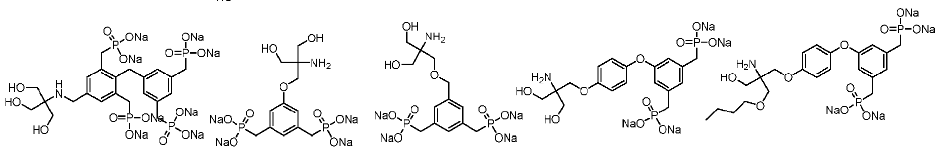

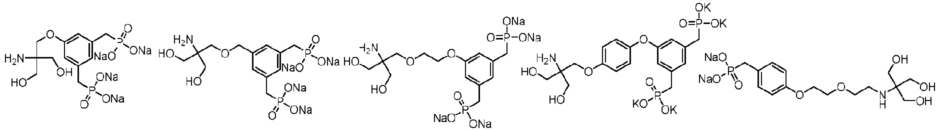

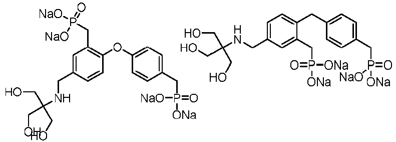

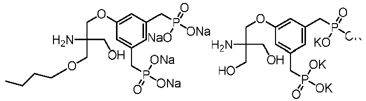

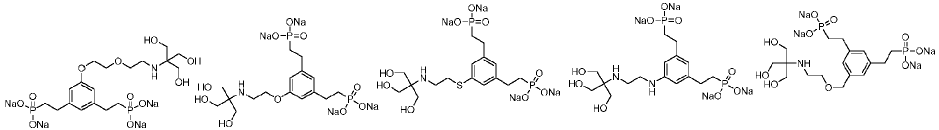

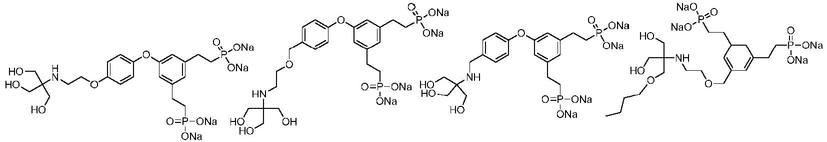

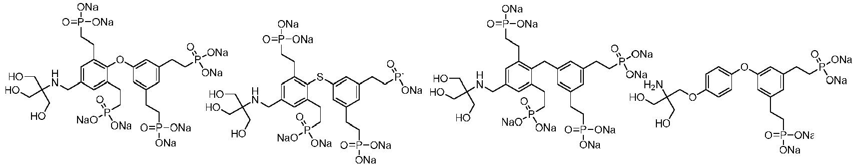

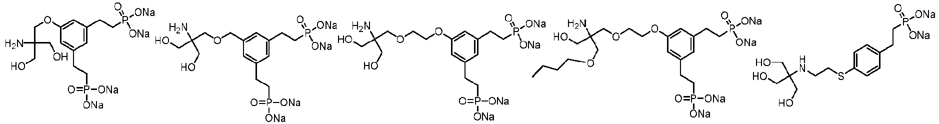

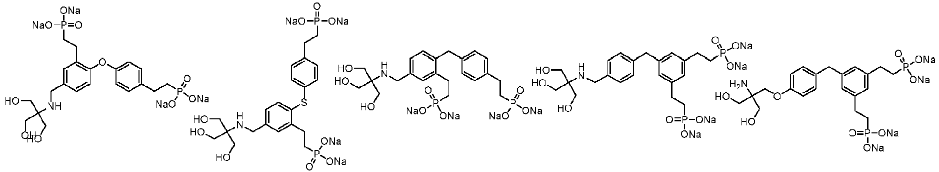

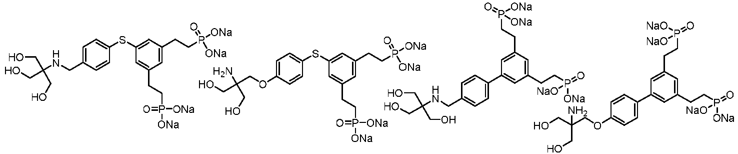

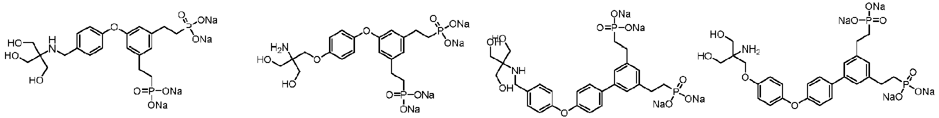

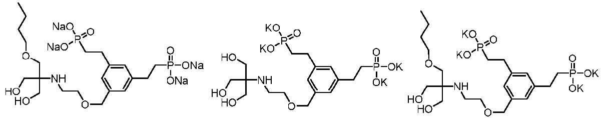

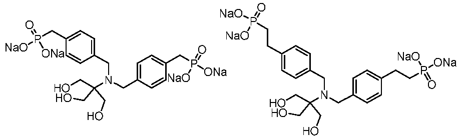

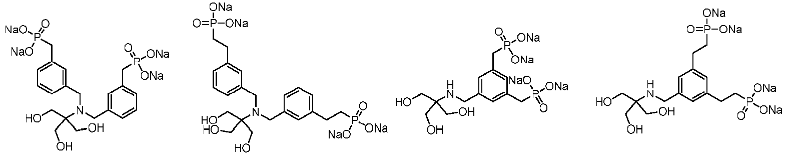

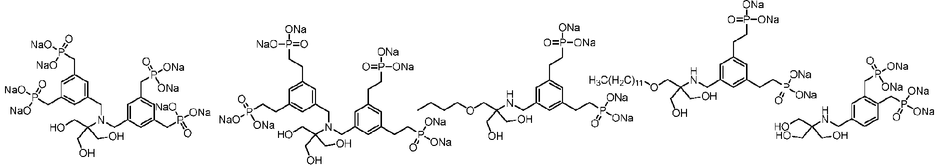

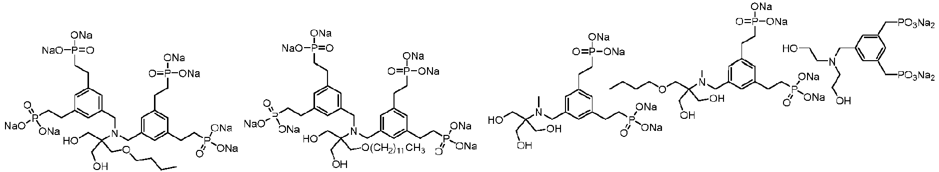

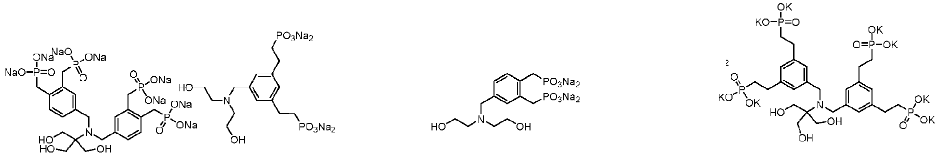

3. . A pharmaceutical composition, which comprises a therapeutically effective amount of the compound according to any one of claims 1 to 2, or the tautomer, mesomer, racemate, enantiomer, and diastereoisomer thereof, or the mixture form thereof, or the pharmaceutically acceptable salt thereof, and further comprises one or more pharmaceutically acceptable carriers, diluents, excipients.

4. . A compound according to any one of claims 1 to 2, or the tautomer, mesomer, racemate, enantiomer, and diastereoisomer thereof, or the mixture form thereof, or the pharmaceutically acceptable salt thereof, or the pharmaceutical composition according to claim 7, for use in a method for preventing and/or treating cancers.

5. . The compound for use according to claim 4, wherein the cancers comprise breast cancers, cervical cancers, colon cancers, lung cancers, stomach cancers, rectal cancers, pancreatic cancers, brain cancers, skin cancers, oral cancers, prostate cancers, bone cancers, kidney cancers, ovarian cancers, bladder cancers, liver cancers, tumors of the fallopian tube, ovarian tumors, peritoneal tumors, stage IV melanoma, glioma, glioblastoma, hepatocellular carcinoma, mastoid nephroma, head and neck tumors, leukemia, lymphoma, myeloma, non-small cell lung cancers, head and neck cancers, uterine cancers, testicular cancers, fallopian tube cancers, endometrial cancers, vaginal cancers, carcinoma of vulva, rectal cancers, colon cancers, anal cancers, breast cancers, esophageal cancers, small intestine cancers, endocrine system cancers, thyroid cancers, parathyroid cancers, adrenal cancers, urethra cancers, penile cancers, testicular cancers, lymph cancers, transitional cell carcinoma, ureteral cancers, renal cell carcinoma, renal pelvic cancers, Hodgkin's diseases, non-Hodgkin's lymphoma, soft tissue sarcoma, solid tumors in children, lymphocytic lymphoma, central nervous system (CNS) tumors, primary central nervous system lymphomas, tumor angiogenesis, spinal tumors, brainstem glioma, pituitary adenoma, melanoma, Kaposi's sarcoma, epidermoid carcinoma, squamous cell carcinoma, T cell lymphoma, chronic or acute leukemia, and/or combinations of the respective cancers.

6. . A compound according to any one of claims 1 to 2, or the tautomer, mesomer, racemate, enantiomer, and diastereoisomer thereof, or the mixture form thereof, or the pharmaceutically acceptable salt thereof, or the pharmaceutical composition according to claim 7, for use in a method for inhibiting cancer metastasis.

7. . A compound according to any one of claims 1 to 2, or the tautomer, mesomer, racemate, enantiomer, and diastereoisomer thereof, or the mixture form thereof, or the pharmaceutically acceptable salt thereof, or the pharmaceutical composition according to claim 7, for use in a method for preventing and/or treating acidosis-caused diseases.

8. . A compound according to any one of claims 1 to 6, or the tautomer, mesomer, racemate, enantiomer, and diastereoisomer thereof, or the mixture form thereof, or the pharmaceutically acceptable salt thereof, in combination with at least one additional anticancer drug for use in a method for treating cancers and/or inhibiting cancer metastasis.

## Patentansprüche

1. Verbindung, die in einer Formel (II) gezeigt wird, oder ein Tautomer, Mesomer, Racemat, Enantiomer und Diastereoisomer davon oder ein Gemisch davon oder ein pharmazeutisch verträgliches Salz davon, 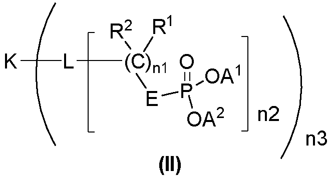
wobei L aus einer Gruppe ausgewählt ist, bestehend aus C1-C10 Alkyl, Cycloalkyl, Cacloalkylalkyl, Alkylcycloalkyl, Aryl, Arylalkyl, Alkylaryl, Heteroaryl, Heteroarylakyl, Alkylheteroaryl, Alkenyl, Alkinyl, einer C3-C15 linearen oder verzweigten Kette, die ein O- oder S-Atom enthält, einer linearen oder verzweigten Kette, die aus Wiederholeinheiten von C1-C15 linearen oder verzweigten Ketten besteht, die O- oder S-Atome enthalten, Reihen von Bisaryl, Reihen von Bisheteroaryl, Reihen von Aryl und
Heteroaryl und Bisaryl und Bisheteroaryl, die durch O, S oder 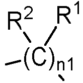
verbunden sind, wobei das Alkyl, das Cycloalkyl, das Heterocyclyl, das Aryl oder das Heteroaryl jeweils unabhängig durch einen oder mehrere Substituenten substituiert werden kann, ausgewählt aus einer Gruppe, bestehend aus Hydroxyl, Halogen, Alkyl, Alkoxyl, Cycloalkyl, Heterocyclyl, Aryl und Heteroaryl;
R¹ und R² jeweils unabhängig aus einer Gruppe ausgewählt sind, bestehend aus Wasserstoff, Halogen, Alkyl, Cycloalkyl, Hydroxyalkan, Alkoxylalkyl, Alkoxylcycloalkyl, Cycloalkylalkyl, Alkylcycloalkyl, Alkenyl, Alkinyl, Amino, Hydroxyl, Mercapto, Carboxyl, Alkoxyl, Cycloalkoxyl, Haloalkyl, Cyano, Thioalkyl, Sulfo, Sulfonyl, Sulfinyl, Phosphat, Alkylphosphonat, Arylphosphat und Arylphosphonat, wobei das Alkyl, das Cycloalkyl, das Heterocyclyl, das Aryl oder das Heteroaryl jeweils unabhängig durch einen oder mehrere Substituenten substituiert werden kann, ausgewählt aus einer Gruppe, bestehend aus Hydroxyl, Halogen, Alkyl, Alkoxyl, Cycloalkyl, Heterocyclyl, Aryl und Heteroaryl;
K aus einer Gruppe ausgewählt ist, bestehend aus 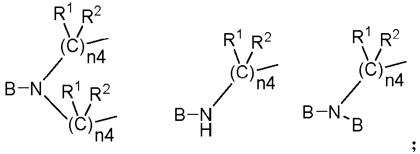
B aus einer Gruppe ausgewählt ist, bestehend aus 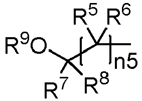
und 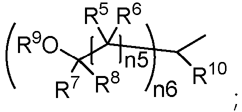
A¹ und A² jeweils unabhängig aus einer Gruppe ausgewählt sind, bestehend aus H, Li, Na, K, Cs und einem entsprechenden positiven Ion davon, oder A¹ und A² gemeinsam Ca, Mg, Al, Sc, Ti, Cr, Co, Fe, Ni, Cu, Zn, Cd, Hg und ein entsprechendes positives Ion davon bilden;
E aus einer Gruppe ausgewählt ist, bestehend aus C(R¹R²);
R⁵ und R⁶ jeweils unabhängig aus einer Gruppe ausgewählt sind, bestehend aus einem Wasserstoffatom, Halogen, Alkyl, Alkoxylalkyl, Cycloalkyl, Alkoxycycloalkyl, Heterocyclyl, Aryl und Heteroaryl, wobei das Alkyl, das Cycloalkyl, das Heterocyclyl, das Aryl oder das Heteroaryl jeweils unabhängig durch einen oder mehrere Substituenten substituiert werden kann, ausgewählt aus einer Gruppe, bestehend aus Hydroxyl, Halogen, Alkyl, Alkoxyl, Cycloalkyl, Heterocyclyl, Aryl und Heteroaryl; oder R⁵ und R⁶ einen 3-teiligen bis 8-teiligen Ring bilden können, der 1 bis 2 Heteroatome von O, N und/oder S enthalten kann;
R⁷ und R⁸ jeweils unabhängig aus einer Gruppe ausgewählt sind, bestehend aus einem Wasserstoffatom, Alkyl, Hydroxyalkyl, Cycloalkyl, Alkoxyalkyl, Alkoxycycloalkyl, Heterocyclyl, Aryl und Heteroaryl, wobei das Alkyl, das Cycloalkyl, das Heterocyclyl, das Aryl oder das Heteroaryl jeweils unabhängig durch einen oder mehrere Substituenten substituiert werden kann, ausgewählt aus einer Gruppe, bestehend aus Hydroxyl, Halogen, Alkyl, Alkoxy, Cycloalkyl, Heterocyclyl, Aryl und Heteroaryl;
R⁹ ein Wasserstoffatom ist;
R¹⁰ aus einer Gruppe ausgewählt ist, bestehend aus einem Wasserstoffatom, Halogen, Alkyl, Cycloalkyl, Heterocyclyl, Aryl und Heteroaryl, oder Null ist, wobei das Alkyl, das Cycloalkyl, das Heterocyclyl, das Aryl oder das Heteroaryl jeweils unabhängig durch einen oder mehrere Substituenten substituiert werden kann, ausgewählt aus einer Gruppe, bestehend aus Hydroxyl, Halogen, Alkyl, Alkoxy, Cycloalkyl, Heterocyclyl, Aryl und Heteroaryl;
n1 aus einer Gruppe ausgewählt ist, bestehend aus 0, 1, 2, 3, 4, 5, 6, 7 und 8;
n2 aus einer Gruppe ausgewählt ist, bestehend aus 1 und 2;
n3 aus einer Gruppe ausgewählt ist, bestehend aus 1 und 2;
n4 aus einer Gruppe ausgewählt ist, bestehend aus 0, 1, 2, 3 und 4;
n5 aus einer Gruppe ausgewählt ist, bestehend aus 0, 1, 2 und 3; und
n6 aus einer Gruppe ausgewählt ist, bestehend aus 1, 2 und 3.

2. Verbindung nach Anspruch 1, wobei die Verbindung mit der Formel (II) Folgendes umfasst:

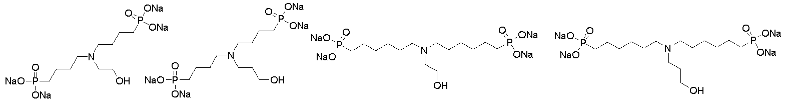

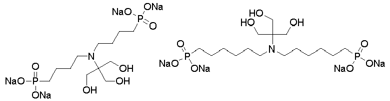

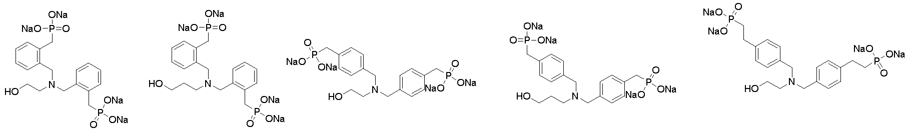

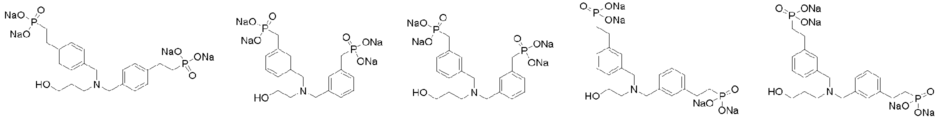

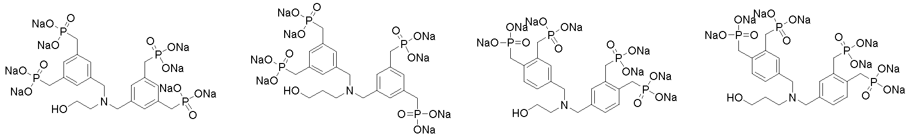

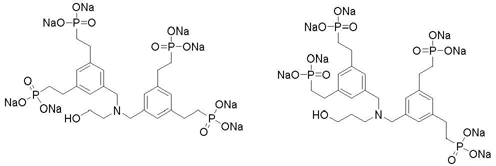

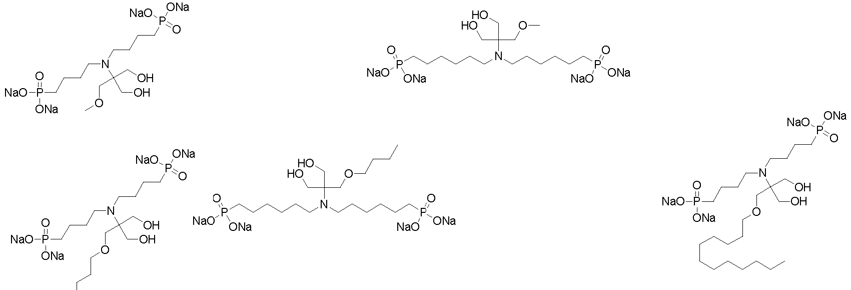

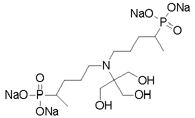

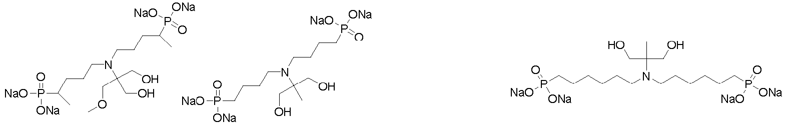

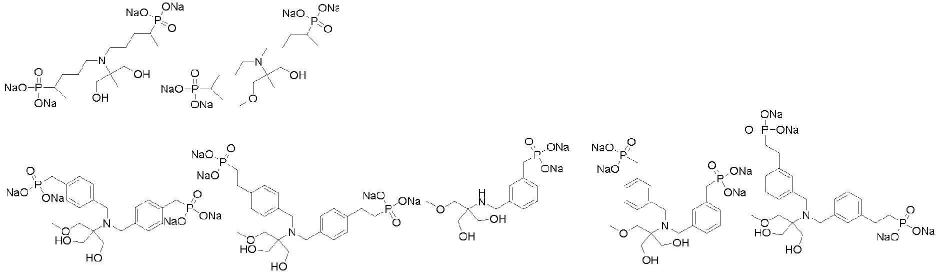

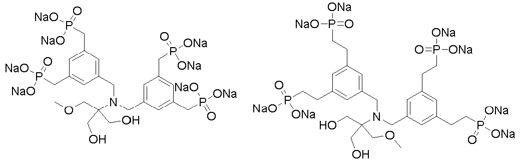

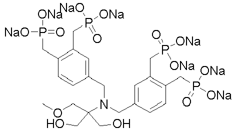

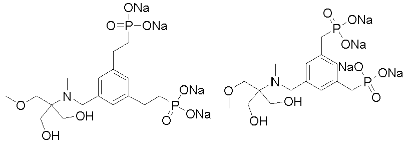

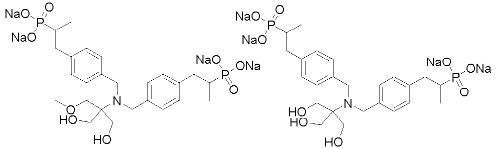

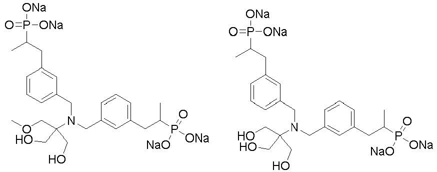

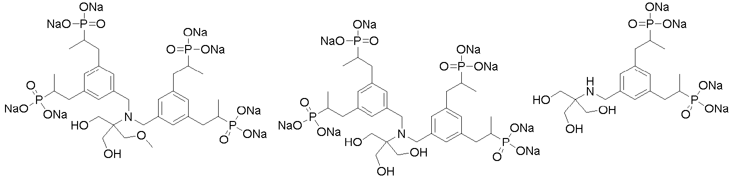

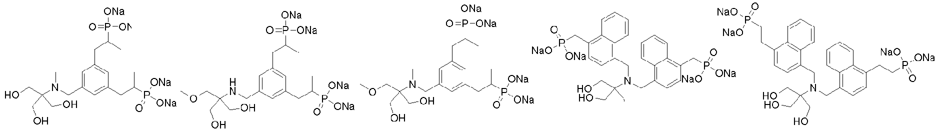

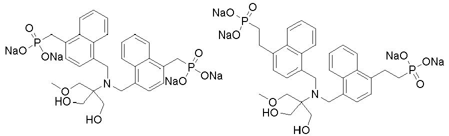

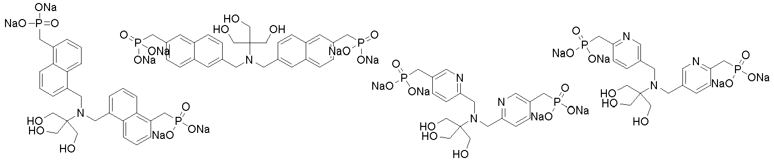

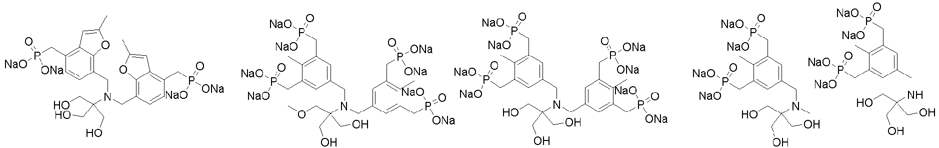

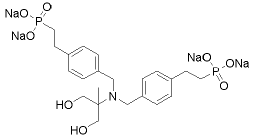

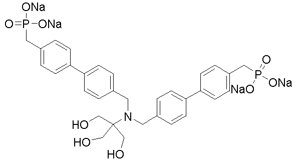

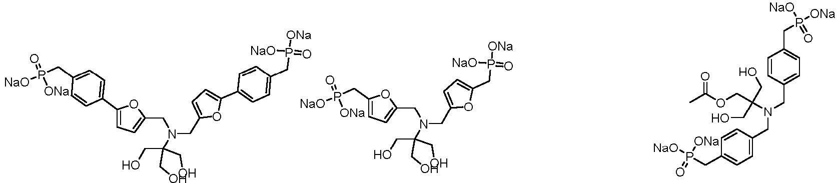

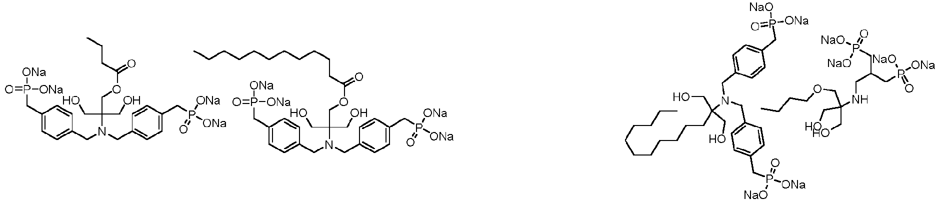

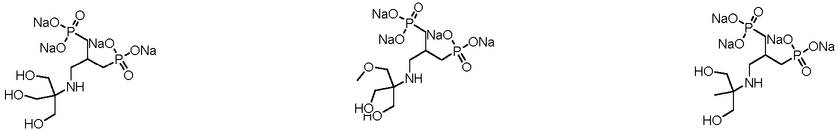

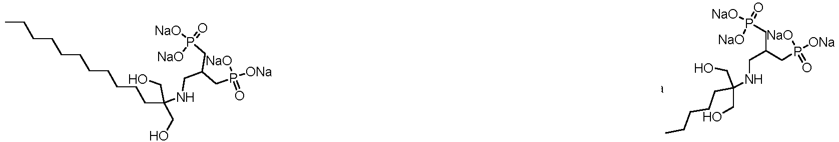

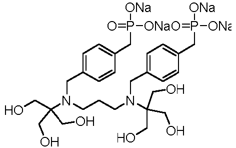

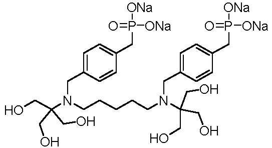

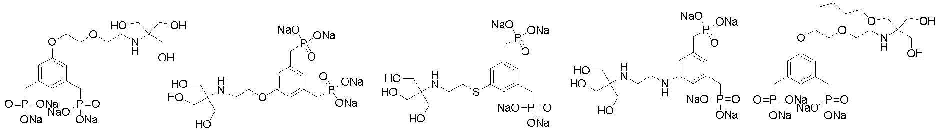

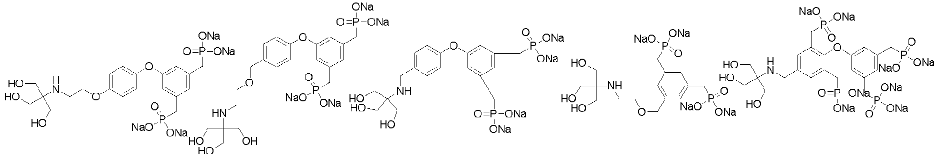

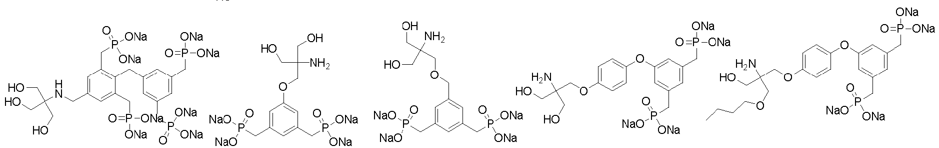

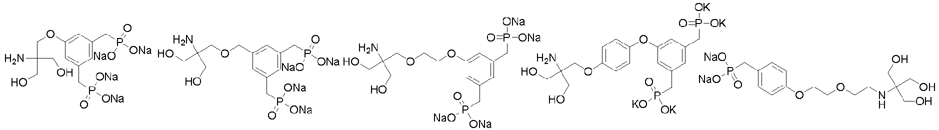

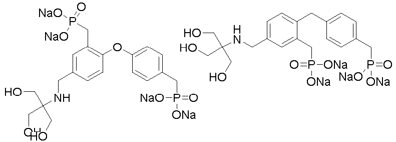

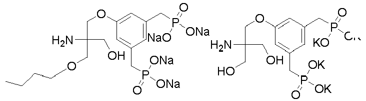

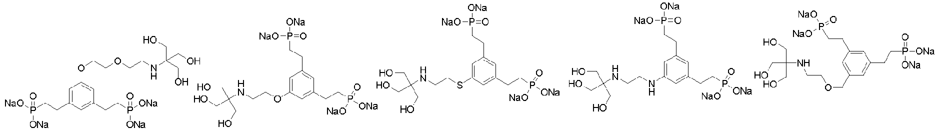

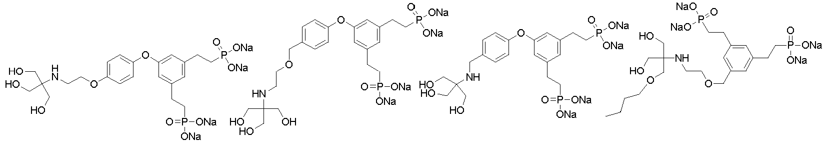

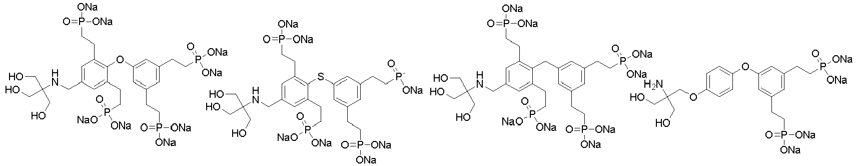

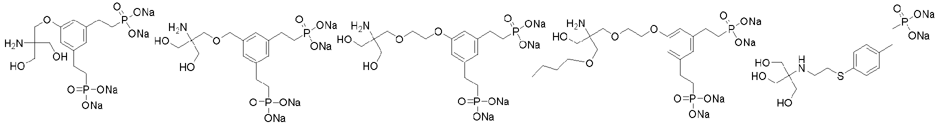

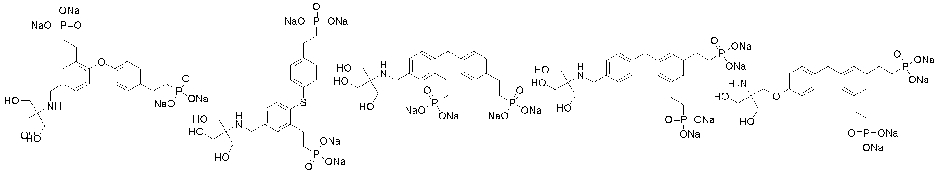

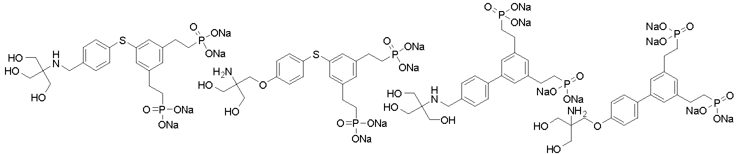

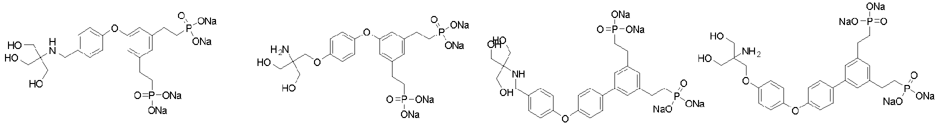

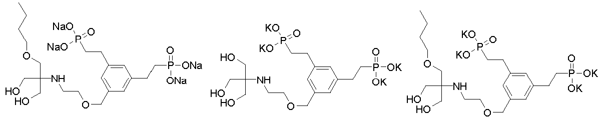

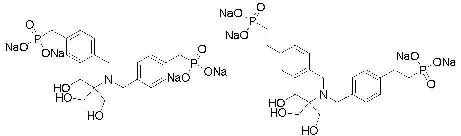

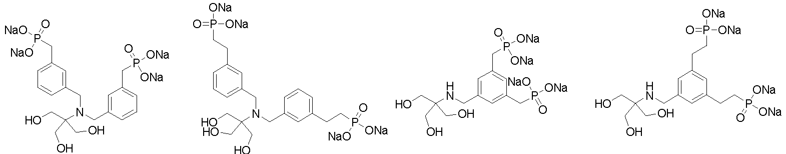

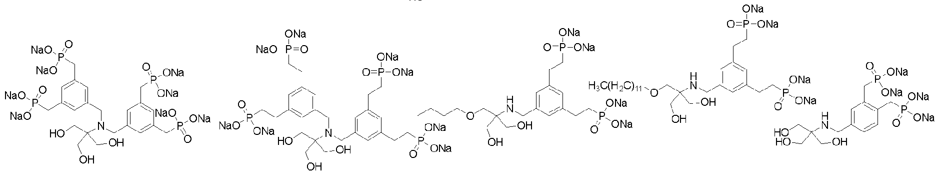

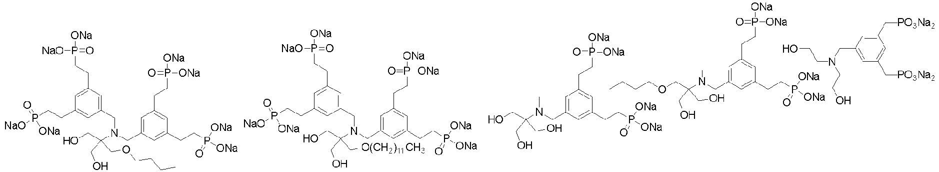

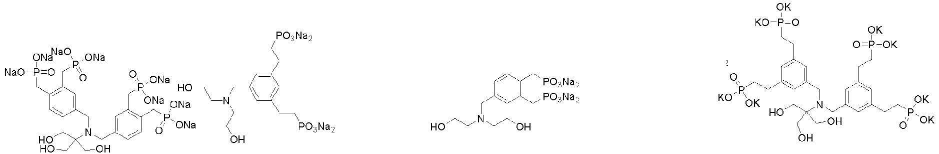

3. Pharmazeutische Zusammensetzung, die eine therapeutisch effektive Menge der Verbindung nach einem der Ansprüche 1 bis 2, oder des Tautomers, Mesomers, Racemats, Enantiomers und Diastereoisomers davon, oder der Form eines Gemisches davon oder des pharmazeutisch verträglichen Salzes davon umfasst und weiter einen oder mehrere pharmazeutisch verträgliche Trägerstoffe, Verdünnungsmittel, Exzipienten umfasst.

4. Verbindung nach einem der Ansprüche 1 bis 2, oder das Tautomer, Mesomer, Racemat, Enantiomer und Diastereoisomer davon, oder die Form eines Gemisches davon, oder das pharmazeutisch verträgliche Salz davon oder die pharmazeutische Zusammensetzung nach Anspruch 7 zur Verwendung in einem Verfahren zum Vorbeugen und/oder Behandeln von Krebserkrankungen.

5. Verbindung zur Verwendung nach Anspruch 4, wobei die Krebserkrankungen Brustkrebs, Gebärmutterhalskrebs, Dickdarmkrebs, Lungenkrebs, Magenkrebs, Rektumkarzinom, Bauchspeicheldrüsenkrebs, Hirnkrebs, Hautkrebs, Mundhöhlenkrebs, Prostatakrebs, Knochenkrebs, Nierenkrebs, Eierstockkrebs, Blasenkrebs, Leberkrebs, Eileitertumor, Eierstocktumor, Bauchfelltumor, Melanom im Stadium IV, Gliom, Glioblastom, Leberzellkarzinom, Mastoidnephrom, Kopf- und Halstumore, Leukämie, Lymphom, Myelom, nicht-kleinzellige Lungenkarzinome, Kopf- und Halskrebs, Gebärmutterkrebs, Hodenkrebs, Eileiterkrebs, Gebärmutterschleimhautkrebs, Vaginalkrebs, Vulvakarzinom, Rektumkarzinom, Dickdarmkrebs, Analkrebs, Brustkrebs, Speiseröhrenkrebs, Dünndarmkrebs, Krebs des endokrinen Systems, Schilddrüsenkrebs, Nebenschilddrüsenkrebs, Nebennierenkrebs, Harnröhrenkrebs, Peniskrebs, Hodenkrebs, Lymphdrüsenkrebs, Übergangszellkarzinom, Harnleiterkrebs, Nierenzellkarzinom, Nierenbeckenkrebs, Hodgkin-Krankheit, Non-Hodgkin-Lymphom, Weichteilsarkom, solide Tumore bei Kindern, lymphatisches Lymphom, Tumore des Zentralnervensystems (ZNS), primäre Lymphome des Zentralnervensystems, Tumorangiogenese, Wirbelsäulentumore, Hirnstamm-Gliom, Hypophysenadenom, Melanom, Kaposi-Sarkom, Epidermoidkarzinom, Plattenepithelkarzinom, T-Zell-Lymphom, chronische oder akute Leukämie und/oder Kombinationen der jeweiligen Krebserkrankungen umfassen.

6. Verbindung nach einem der Ansprüche 1 bis 2, oder das Tautomer, Mesomer, Racemat, Enantiomer und Diastereoisomer davon, oder die Form eines Gemisches davon, oder das pharmazeutisch verträgliche Salz davon oder die pharmazeutische Zusammensetzung nach Anspruch 7 zur Verwendung in einem Verfahren zum Hemmen von Metastasierung von Krebs.

7. Verbindung nach einem der Ansprüche 1 bis 2, oder das Tautomer, Mesomer, Racemat, Enantiomer und Diastereoisomer davon, oder die Form eines Gemisches davon, oder das pharmazeutisch verträgliche Salz davon oder die pharmazeutische Zusammensetzung nach Anspruch 7 zur Verwendung in einem Verfahren zum Vorbeugen und/oder Behandeln von durch Übersäuerung verursachten Erkrankungen.

8. Verbindung nach einem der Ansprüche 1 bis 6, oder das Tautomer, Mesomer, Racemat, Enantiomer und Diastereoisomer davon, oder die Form eines Gemisches davon, oder das pharmazeutisch verträgliche Salz davon in Kombination mit mindestens einem zusätzlichen Antikrebs-Arzneimittel zur Verwendung in einem Verfahren zum Behandeln von Krebsarten und/oder Hemmen von Krebsmetastasierung.

## Revendications

1. Composé représenté dans une formule (II), ou un tautomère, mésomère, racémate, énantiomère, et diastéréoisomère de celui-ci, ou une forme de mélange de celui-ci, ou un sel pharmaceutiquement acceptable de celui-ci, 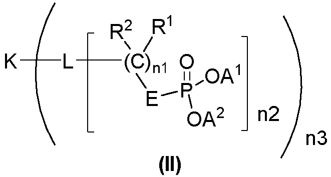
dans lequel, L est choisi dans un groupe se composant d'alkyle en C1-C10, cycloalkyle, cycloalkylalkyle, alkylcycloalkyle, aryle, arylalkyle, alkylaryle, hétéroaryle, hétéroarylalkyle, alkylhétéroaryle, alcényle, alcynyle, une chaîne linéaire ou ramifiée en C3-C15 contenant un atome d'O ou S, une chaîne linéaire ou ramifiée constituée d'unités répétées de chaînes linéaires ou ramifiées en C1-C15 contenant des atomes d'O ou S, une série de bisaryle, une série de bishétéroaryle, une série d'aryle et
d'hétéroaryle, et du bisaryle et du bishétéroaryle liés par O, S ou 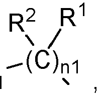
dans lequel l'alkyle, le cycloalkyle, l'hétérocyclyle, l'aryle ou l'hétéroaryle peut être chacun indépendamment substitué par un ou plusieurs substituants choisis dans un groupe se composant d'hydroxyle, halogène, alkyle, alkoxyle, cycloalkyle, hétérocyclyle, aryle, et hétéroaryle ;
R¹ et R² sont chacun indépendamment choisis dans un groupe se composant d'hydrogène, halogène, alkyle, cycloalkyle, hydroxyalcane, alkoxylalkyle, alkoxylcycloalkyle, cycloalkylalkyle, alkylcycloalkyle, alcényle, alcynyle, amino, hydroxyle, mercapto, carboxyle, alkoxyle, cycloalkoxyle, haloalkyle, cyano, thioalkyle, sulfo, sulfonyle, sulfinyle, phosphate, alkylphosphonate, arylphosphate, et arylphosphonate, dans lequel l'alkyle, le cycloalkyle, l'hétérocyclyle, l'aryle, ou l'hétéroaryle peut être chacun indépendamment substitué par un ou plusieurs substituants choisis dans un groupe se composant d'hydroxyle, halogène, alkyle, alkoxyle, cycloalkyle, hétérocyclyle, aryle, et hétéroaryle ;
K est choisi dans un groupe se composant de 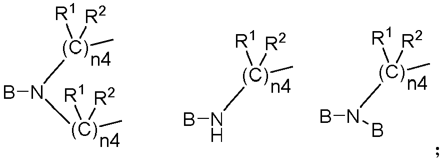
B est choisi dans un groupe se composant de 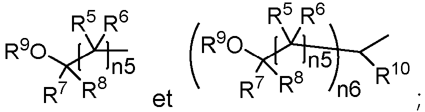
A¹ et A² sont chacun indépendamment choisis dans un groupe se composant de H, Li, Na, K, Cs, et un ion positif correspondant de ceux-ci, ou A¹ et A² forment Ca, Mg, Al, Sc, Ti, Cr, Co, Fe, Ni, Cu, Zn, Cd, Hg, et un ion positif correspondant de ceux-ci, collectivement ;
E est choisi dans un groupe se composant de C(R¹R²) ;
R⁵ et R⁶ sont chacun indépendamment choisis dans un groupe se composant d'un atome d'hydrogène, halogène, alkyle, alkoxyalkyle, cycloalkyle, alkoxycycloalkyle, hétérocyclyle, aryle et hétéroaryle, dans lequel l'alkyle, le cycloalkyle, l'hétérocyclyle, l'aryle ou l'hétéroaryle peut être chacun indépendamment substitué par un ou plusieurs substituants choisis dans un groupe se composant d'hydroxyle, halogène, alkyle, alkoxyle, cycloalkyle, hétérocyclyle, aryle, et hétéroaryle ; ou R⁵ et R⁶ peuvent former un cycle de 3 membres à 8 membres, pouvant contenir de 1 à 2 hétéroatomes d'O, N et/ou S ;
R⁷ et R⁸ sont chacun indépendamment choisis dans un groupe se composant d'un atome d'hydrogène, alkyle, hydroxyalkyle, cycloalkyle, alkoxyalkyle, alkoxycycloalkyle, hétérocyclyle, aryle et hétéroaryle, dans lequel l'alkyle, le cycloalkyle, l'hétérocyclyle, l'aryle ou l'hétéroaryle peut être chacun indépendamment substitué par un ou plusieurs substituants choisis dans un groupe se composant d'hydroxyle, halogène, alkyle, alkoxy, cycloalkyle, hétérocyclyle, aryle et hétéroaryle ;
R⁹ est un atome d'hydrogène ;
R¹⁰ est choisi dans un groupe se composant d'un atome d'hydrogène, halogène, alkyle, cycloalkyle, hétérocyclyle, aryle, et hétéroaryle, ou est nul, dans lequel l'alkyle, le cycloalkyle, l'hétérocyclyle, l'aryle, ou l'hétéroaryle peut être chacun indépendamment substitué par un ou plusieurs substituants choisis dans un groupe se composant d'hydroxyle, halogène, alkyle, alkoxy, cycloalkyle, hétérocyclyle, aryle, et hétéroaryle ;
n1 est choisi dans un groupe se composant de 0, 1, 2, 3, 4, 5, 6, 7, et 8 ;
n2 est choisi dans un groupe se composant de 1 et 2 ;
n3 est choisi dans un groupe se composant de 1 et 2 ;
n4 est choisi dans un groupe se composant de 0, 1, 2, 3, et 4 ;
n5 est choisi dans un groupe se composant de 0, 1, 2, et 3 ; et
n6 est choisi dans un groupe se composant de 1, 2, et 3.

2. Composé selon la revendication 1, dans lequel le composé avec la formule (II) comprend ce qui suit :

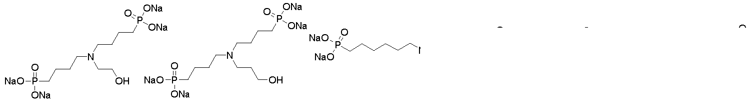

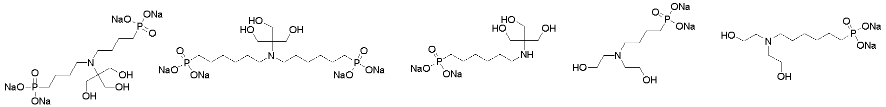

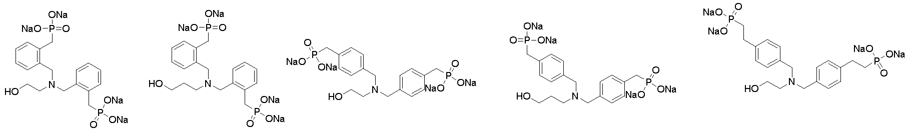

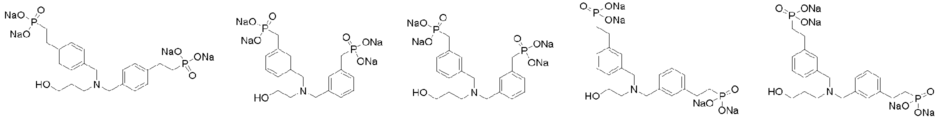

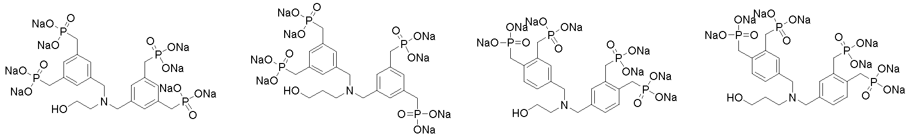

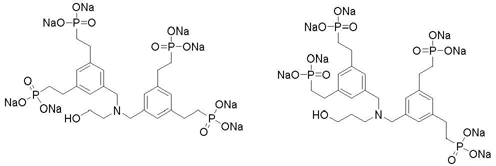

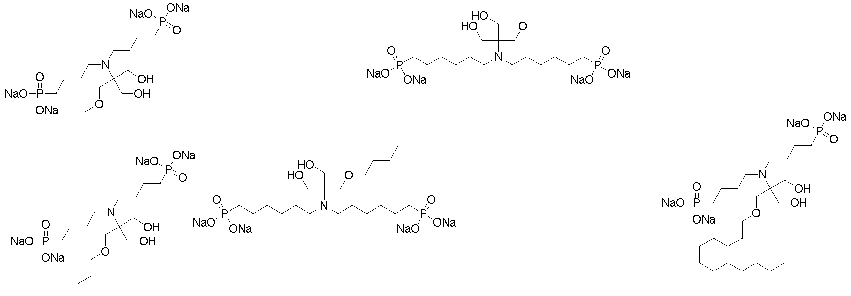

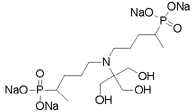

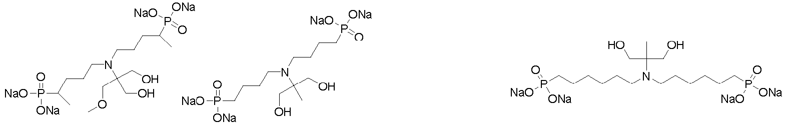

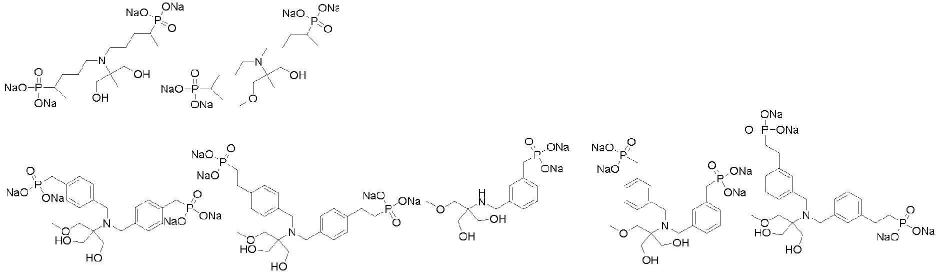

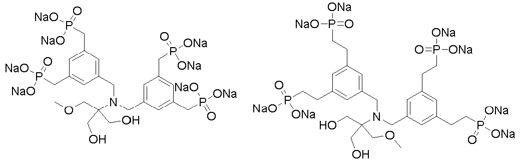

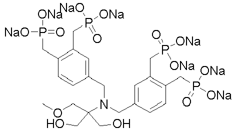

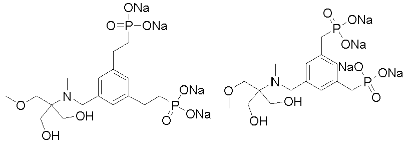

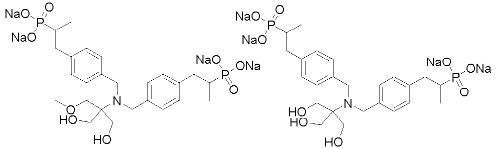

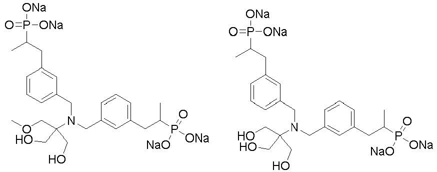

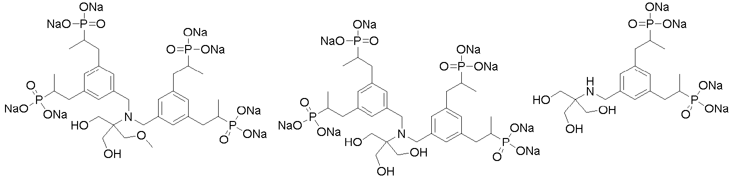

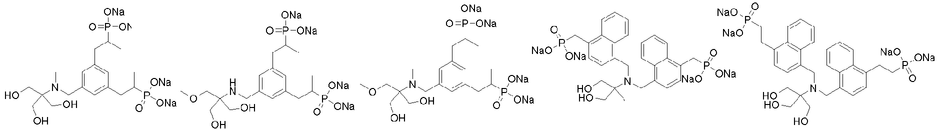

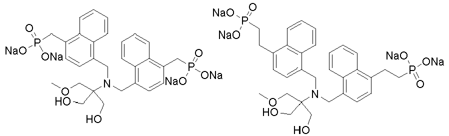

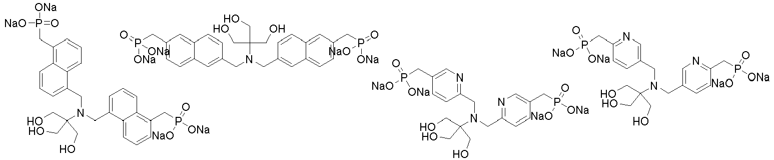

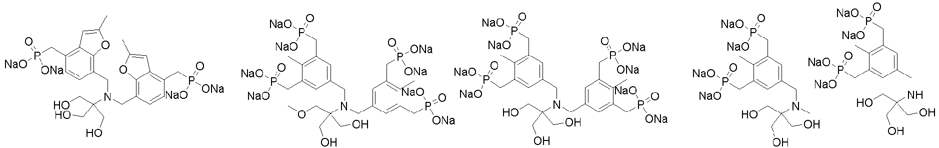

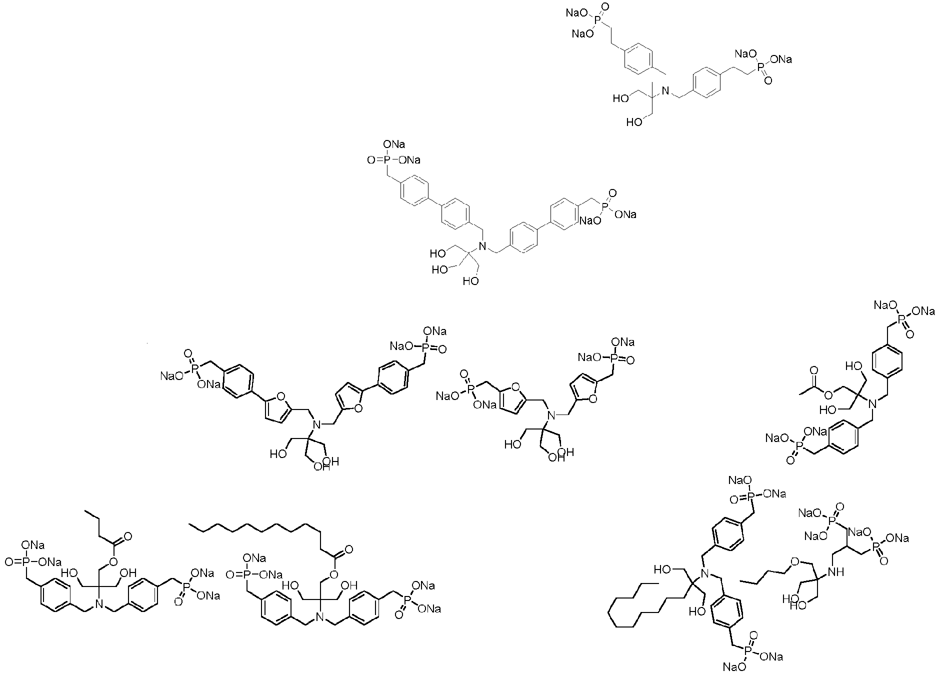

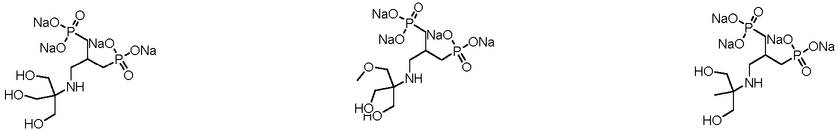

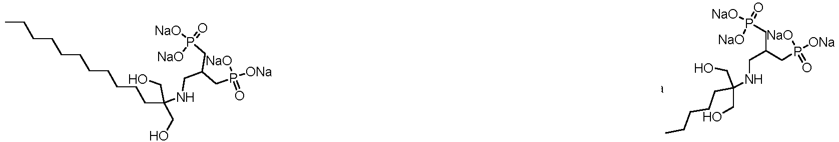

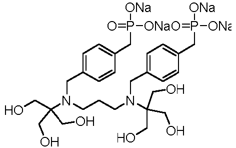

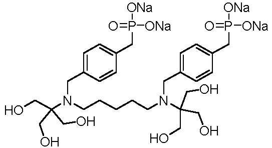

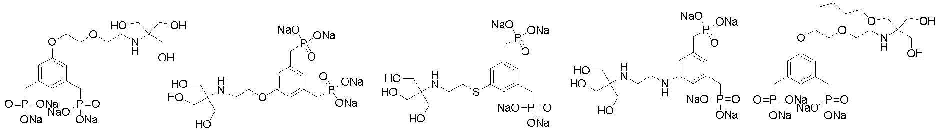

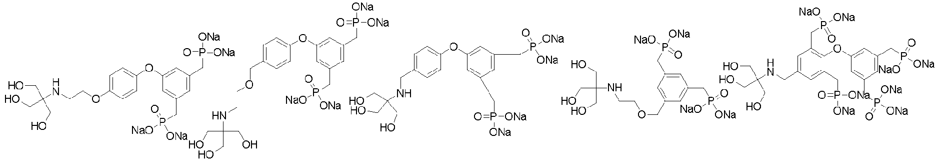

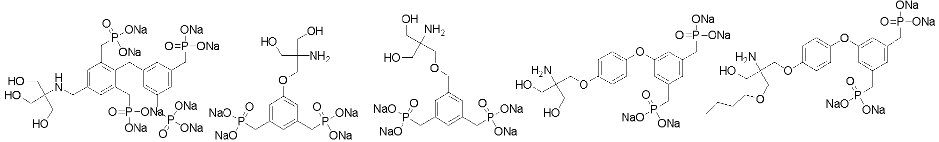

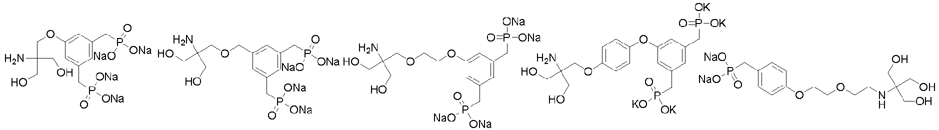

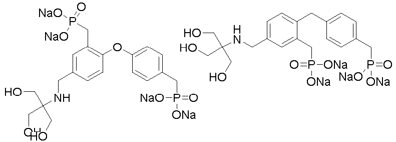

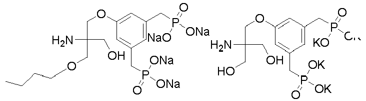

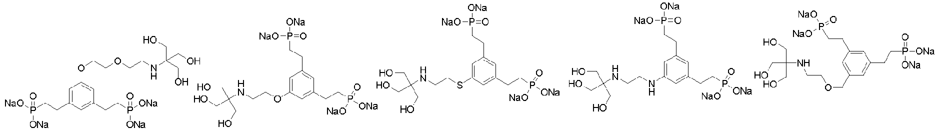

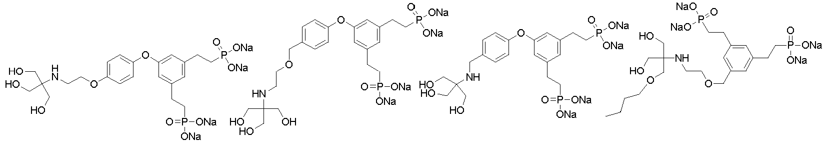

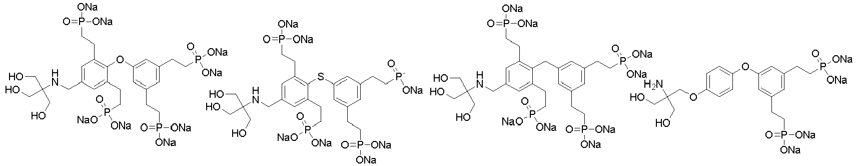

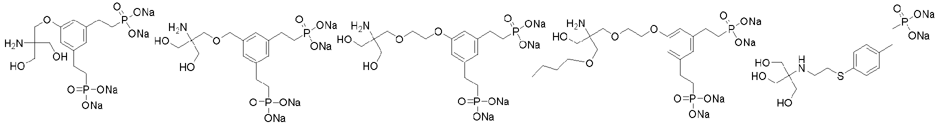

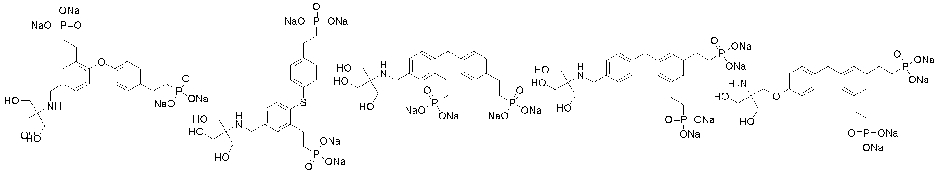

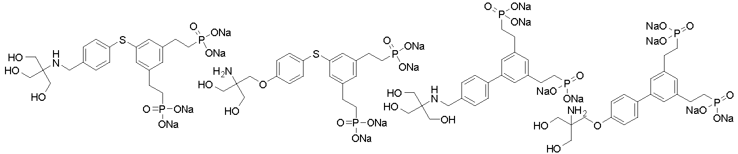

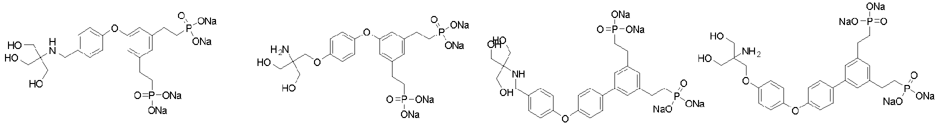

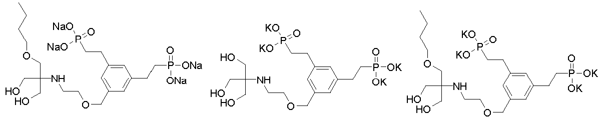

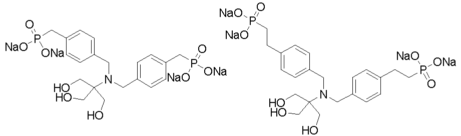

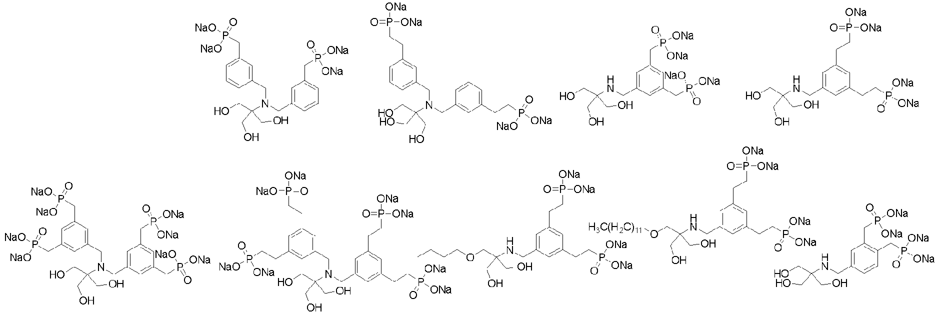

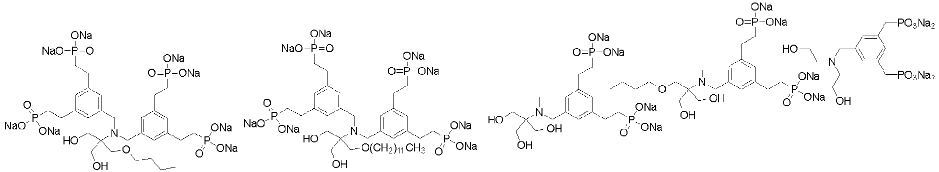

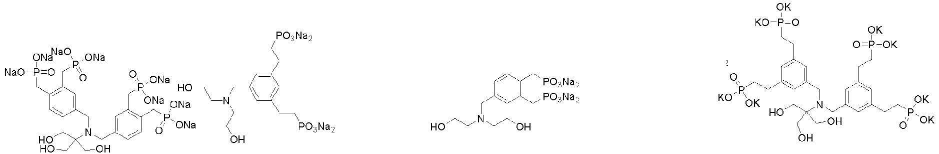

3. Composition pharmaceutique, qui comprend une quantité thérapeutiquement efficace du composé selon l'une quelconque des revendications 1 à 2, ou le tautomère, mésomère, racémate, énantiomère, et diastéréoisomère de celui-ci, ou la forme de mélange de celui-ci, ou le sel pharmaceutiquement acceptable de celui-ci, et comprend en outre un ou plusieurs supports, diluants, excipients pharmaceutiquement acceptables.

4. Composé selon l'une quelconque des revendications 1 à 2, ou le tautomère, mésomère, racémate, énantiomère, et diastéréoisomère de celui-ci, ou la forme de mélange de celui-ci, ou le sel pharmaceutiquement acceptable de celui-ci, ou la composition pharmaceutique selon la revendication 7, pour son utilisation dans un procédé de prévention et/ou de traitement de cancers.

5. Composé pour son utilisation selon la revendication 4, dans lequel les cancers comprennent des cancers du sein, des cancers du col de l'utérus, des cancers du colon, des cancers du poumon, des cancers de l'estomac, des cancers rectaux, des cancers du pancréas, des cancers du cerveau, des cancers de la peau, des cancers buccaux, des cancers de la prostate, des cancers des os, des cancers des reins, des cancers des ovaires, des cancers de la vessie, des cancers du foie, des tumeurs de la trompe de Fallope, des tumeurs des ovaires, des tumeurs péritonéales, un mélanome de stade IV, un gliome, une glioblastome, un carcinome hépatocellulaire, un néphrome mastoïde, des tumeurs de la tête et du cou, une leucémie, un lymphome, un myélome, des cancers du poumon non à petites cellules, des cancers de la tête et du cou, des cancers utérins, des cancers testiculaires, des cancers de la trompe de Fallope, des cancers de l'endomètre, des cancers vaginaux, un carcinome de la vulve, des cancers rectaux, des cancers du colon, des cancers de l'anus, des cancers du sein, des cancers de l'oesophage, des cancers du petit intestin, des cancers du système endocrinien, des cancers de la thyroïde, des cancers de la parathyroïde, des cancers surrénaliens, des cancers de l'urètre, des cancers du pénis, des cancers testiculaires, des cancers de la lymphe, un carcinome à cellules transitionnelles, des cancers de l'uretère, un carcinome à cellules rénales, des cancers pelviens rénaux, des maladies hodgkiniennes, un lymphome non hodgkinien, un sarcome des tissus mous, des tumeurs solides chez des enfants, un lymphome lymphocytaire, des tumeurs du système nerveux central (SNC), des lymphomes primaires du système nerveux central, une angiogenèse tumorale, des tumeurs rachidiennes, un gliome du tronc cérébral, un adénome hypophysaire, un mélanome, un sarcome de Kaposi, un carcinome épidermoïde, un carcinome des cellules squameuses, un lymphome à cellules T, une leucémie chronique ou aigüe, et/ou des combinations des cancers respectifs.

6. Composé selon l'une quelconque des revendications 1 à 2, ou le tautomère, mésomère, racémate, énantiomère, et diastéréoisomère de celui-ci, ou la forme de mélange de celui-ci, ou le sel pharmaceutiquement acceptable de celui-ci, ou la composition pharmaceutique selon la revendication 7, pour son utilisation dans un procédé d'inhibition des métastases cancéreuses.

7. Composé selon l'une quelconque des revendications 1 à 2, ou le tautomère, mésomère, racémate, énantiomère, et diastéréoisomère de celui-ci, ou la forme de mélange de celui-ci, ou le sel pharmaceutiquement acceptable de celui-ci, ou la composition pharmaceutique selon la revendication 7, pour son utilisation dans un procédé de prévention et/ou de traitement des maladies provoquées par l'acidose.

8. Composé selon l'une quelconque des revendications 1 à 6, ou le tautomère, mésomère, racémate, énantiomère, et diastéréoisomère de celui-ci, ou la forme de mélange de celui-ci, ou le sel pharmaceutiquement acceptable de celui-ci, en combinaison avec au moins un médicament anticancéreux supplémentaire pour son utilisation dans un procédé de traitement des cancers et/ou d'inhibition des métastases cancéreuses.
